# EUROPEAN PATENT APPLICATION

(11) **EP 3 653 611 A1**
(43) Date of publication of application: **20.05.2020**
(21) Application number: 18306493.0
(22) Date of filing: 15.11.2018
(51) Int. Cl.: C07D 249/10, C07D 401/04, C07D 401/06, C07D 401/12, C07D 405/06, C07D 409/04, C07D 417/04, A61P 31/04, A61K 31/4196

(54) **INHIBITORS OF METALLO-BETA-LACTAMASES**

(71) Applicant: CENTRE NATIONAL DE LA RECHERCHE SCIENTIFIQUE, 75016 Paris (FR); Universite de Sienne, 53100 Siena (IT); Ecole Nationale Supérieure de Chimie de Montpellier, 34090 Montpellier (FR); UNIVERSITE DE MONTPELLIER, 34090 Montpellier (FR)
(72) Inventor: HERNANDEZ, Jean-François, 34150 Gignac (FR); GAVARA, Laurent, 34920 Le Cres (FR); DOCQUIER, Jean-Denis, 53018 Sovicille (IT); SEVAILLE, Laurent, 34090 Montpellier (FR)
(74) Representative: Cabinet Becker et Associés

(57) **Abstract**

The present invention relates to new compounds that function as inhibitors of metallo-beta-lactamases (MBL). The present invention also relates to pharmaceutical compositions comprising such MBL inhibitors, and uses thereof in the treatment of bacterial infections. More generally, the present invention finds applications in any field wherein bacteria producing MBL must be removed.

## Description

The present invention relates to compounds that function as inhibitors of metallo-beta-lactamases (MBL). The present invention also relates to pharmaceutical compositions comprising such MBL inhibitors, and uses thereof in the treatment of bacterial infections. More generally, the present invention finds applications in any field wherein microorganism-producing MBL must be removed.

### BACKGROUND OF THE INVENTION

Infections caused by pathogenic bacteria are common worldwide, and thus antibacterial treatments of such infections are highly sought. However, the development and spread of bacteria resistant to such antibiotics is a growing public health issue. It threatens the effective treatment of infections and could render inefficient some of the major achievements of modern medicine. Currently, β-lactam antibacterials are amongst the most widely used antibacterial treatments. Although resistance to antibiotics is a natural phenomenon, it has been made worse by the overutilization of antibiotics and also by increased global spreading of resistant strains due to travelling. In Europe and other advanced societies, the highest proportion of resistant bacteria is found in hospitals. Today, about 70% of bacteria that cause infections in hospitals are resistant to at least one of the drugs most commonly used to treat them.

The most worrying antibiotic resistance mechanism is presently the enzyme-catalyzed hydrolysis of the β-lactam ring of the β-lactam family, the most widely used group of antibacterial agents, which includes penicillins, cephalosporins, carbapenems, etc. One highly relevant approach to overcome β-lactamase-mediated resistance deals with combination therapy in which a β-lactam drug is given along with a β-lactamase inhibitor, which protects the former from inactivation.

β-Lactamases are grouped into four molecular classes, A, B, C, and D. Classes A, C, and D are serine-enzymes in which a serine residue bears the catalytic activity. Most of them are not active against carbapenems. Class B represents the MBLs, wherein one or two Zn atom(s) promote(s) β-lactam hydrolysis. Whereas several β-lactamase inhibitors are currently marketed, they only target a limited number of β-lactamases of the serine catalytic type. It is still not the case for the important metallo-β-lactamase (MBL) class. The most worrisome characteristic of MBLs is their capacity to inactivate all classes of β-lactams (except monobactams) including carbapenems, which are stable against the vast majority of serine-β-lactamases, are the antibiotics with the broadest spectrum of activity and are considered as antimicrobial drugs of last resort at the hospital. Moreover, MBLs are not inactivated by the marketed β-lactamase inhibitors and can even degrade some of them.

The MBLs are produced by clinical isolates belonging to some of the most important clinically relevant bacterial species, causing a significant number of nosocomial infections (such as *Klebsiella pneumoniae, Pseudomonas aeruginosa, Aeromonas* spp., *Acinetobacter baumannii, Enterobacter cloacae, Escherichia coli, Serratia marcescens, etc*.). Furthermore, whereas MBLs were mainly found in nosocomial strains, another currently serious concern is their occurrence in bacteria causing community-acquired infections. MBLs are now regarded as a major therapeutic challenge.

Thus, there remains a need for new treatments to combat MBL mediated antibacterial resistance.

### SUMMARY OF THE INVENTION

It is thus the purpose of the present invention to provide new compounds, based on heterocyclic molecules containing a 1,2,4-triazole-3-thione moiety diversely substituted at positions 4 and 5, particularly effective against clinically-relevant MBLs, including NDM-, VIM- and IMP-type enzymes. A compound according to formula (II) below and having a 1,2,4-triazole-3-thione moiety had already been developed. However, such compound shows few or no activity against several MBLs, including VIM-2 and NDM-1. By working on this compound, the inventors developed stable compounds, wherein the hydrazone-like bond was replaced by a saturated N-C or C-C bond. This modification led to compounds showing increased stability and broad-spectrum inhibition of VIM-type and NDM-1 enzymes and activity in clinical bacterial strains.

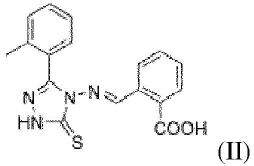

In one aspect, the present invention provides a compound of formula (I) as defined herein, or a pharmaceutically acceptable salt or solvate thereof.

In another aspect, the present invention provides a pharmaceutical composition, which comprises a compound of formula (I) as defined herein, or a pharmaceutically acceptable salt or solvate thereof, and one or more pharmaceutically acceptable excipients.

The present invention also provides a composition, which comprises a compound of formula (I) as defined herein and optionally at least one suitable antimicrobial agent (antibacterial, antifungal or antiviral). Said composition helps controlling microorganisms, including virus, bacteria and fungi, in pharmacological (human and veterinary treatment), phytosanitary, agriculture and cleaning (detergents) applications.

In another aspect, the present invention provides a compound of formula (I) as defined herein, or a pharmaceutically acceptable salt or solvate thereof, or a pharmaceutical composition as defined herein, for use in the treatment of bacterial infection.

In another aspect, the present invention provides a compound of formula (I) as defined herein, or a pharmaceutically acceptable salt or solvate thereof, or a pharmaceutical composition as defined herein, in combination with a suitable antibacterial agent, for use in the treatment of a bacterial infection.

In another aspect, the present invention provides a method of inhibiting a bacterial metallo-beta-lactamase in vitro or in vivo, said method comprising contacting a cell with an effective amount of a compound of formula (I) as defined herein, or a pharmaceutically acceptable salt or solvate thereof.

In another aspect, the present invention provides a method of treating a bacterial infection in a patient in need of such treatment, said method comprising administering to said patient, a therapeutically effective amount of a compound of formula (I) as defined herein, or a pharmaceutically acceptable salt or solvate thereof, or a pharmaceutical composition as defined herein, in combination with a suitable antibacterial agent.

In another aspect, the present invention provides a compound of formula (I) as defined herein, or a pharmaceutically acceptable salt or solvate thereof, for use in combination with a suitable antibacterial agent, for the inhibition of a MBL.

In another aspect, the present invention provides a method for removing bacteria producing MBL, wherein said bacteria are contacting with at least one compound of formula (I) and a suitable antibacterial agent.

The present invention also provides a kit comprising at least one compound of formula (I) and at least one suitable antibacterial agent. For instance, said kit may be used for *in vitro* test to evaluate the efficiency of an antibacterial agent on bacteria producing MBLs. Said kit may also be used for preparing pharmaceutical composition for treating a bacterial infection in patient in need thereof.

More particularly, the present invention relates to a compound of formula (I) or a pharmaceutically acceptable salt thereof, as shown below, 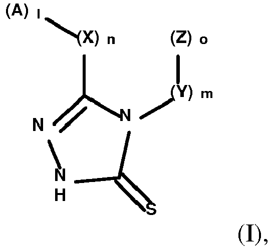 wherein,
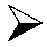 n and m are independently 0 or 1;
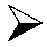 l is 1 or 2;
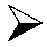 o is 0, 1 or 2;
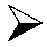 when l is 2, n is 1;
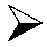 when o is 2, m is 1;
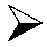 when m is 0, o is 1;
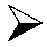 when n is 0,1 is 1;
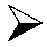 (X) represents a (C₁-C₃)alkyl;
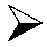 (A) represents a 5-14 membered ring selected in the group consisting of an aryl, a heteroaryl, a cycloalkyl, said 5-14 membered ring is optionally substituted by at least one radical selected in the group consisting of:
   ▪ a halogen, preferably a chlorine, a fluorine or a bromine,
   ▪ OH,
   ▪ a (C₁-C₆)alkoxy, optionally substituted by a heterocycloalkyl, preferably a methoxy or a morpholinylethoxy,
   ▪ a (C₁-C₆)alkyl, optionally substituted by at least one halogen, preferably a fluorine, and
   ▪ an aryl or aryl(C₁-C₆)alkyloxy;
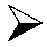 (Y) represents a (C₁-C₁₀)alkyl chain, comprising optionally a heteroatomic group;
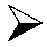 (Z) represents
   ∘ a 5-14 membered ring selected in the group consisting of an aryl, a cycloalkyl, a heterocycle, preferably a heteroaryl, a heterocycloalkyl, or a heterocyclic derivative, said 5-14 membered ring being optionally substituted by at least one radical selected in the group consisting of:
      ▪ a halogen, preferably a chlorine, a fluorine or a bromine,
      ▪ OH, COOH, NO₂, NH₂,
      ▪ a (C₁-C₆)alkyl, optionally substituted by at least one halogen, preferably a fluorine, or by COOH,
      ▪ An aryl optionally substituted by NO₂, or aryl(C₁-C₆)alkyloxy;
   ∘ A radical selected in the group consisting of:
      ▪ COOH,
      ▪ N₃,
      ▪ a NR₁R₂ group, wherein R₁ and R₂ represent independently H, Boc, (C₁-C₆)alkyl,
      ▪ SO₂-R₇, wherein R₇ represents a (C₁-C₆)alkyl, or a 5-14 membered ring, preferably an aryl, optionally substituted by an (C₁-C₆)alkyl,
      ▪ a benzoyl, and
      ▪ N-phenylurea; or
   ∘ a (C₁-C₆)alkyl, optionally substituted by at least one halogen, preferably a fluorine, or by COOH.

In a preferred embodiment, the compound of formula (I) is selected from the group consisting of:
- Compound 1. 2-[2-(3-phenyl-5-sulfanylidene-4,5-dihydro-1H-1,2,4-triazol-4-yl)ethyl]benzoic acid;
- Compound 2. 4-(3-phenyl-5-sulfanylidene-4,5-dihydro-1H-1,2,4-triazol-4-yl)butanoic acid;
- Compound 3. 2-{2-[3-(naphthalen-2-yl)-5-sulfanylidene-4,5-dihydro-1H-1,2,4-triazol-4-yl]ethyl}benzoic acid;
- Compound 4. 2-{2-[3-(2-hydroxy-5-methoxyphenyl)-5-sulfanylidene-4,5-dihydro-1H-1,2,4-triazol-4-yl]ethyl}benzoic acid;
- Compound 5. 2-{[(3-phenyl-5-sulfanylidene-4,5-dihydro-1H-1,2,4-triazol-4-yl)amino]methyl}benzoic acid;
- Compound 6. 3-(2-methylphenyl)-4-(2-phenylethyl)-4,5-dihydro-1H-1,2,4-triazole-5-thione;
- Compound 7. 4-benzyl-3-(2-hydroxy-5-methoxyphenyl)-4,5-dihydro-1H-1,2,4-triazole-5-thione;
- Compound 8. 3-phenyl-4-(3-phenylpropyl)-4,5-dihydro-1H-1,2,4-triazole-5-thione;
- Compound 9. 4-benzyl-3-phenyl-4,5-dihydro-1H-1,2,4-triazole-5-thione;
- Compound 10. 3-(2-hydroxy-5-methoxyphenyl)-4-(2-phenylethyl)-4,5-dihydro-1H-1,2,4-triazole-5-thione;
- Compound 11. 3-phenyl-4-(2-phenylethyl)-4,5-dihydro-1H-1,2,4-triazole-5-thione;
- Compound 12. 2-{2-[3-(furan-2-yl)-5-sulfanylidene-4,5-dihydro-1H-1,2,4-triazol-4-yl]ethyl}benzoic acid;
- Compound 13. 2-{2-[3-(thiophen-2-yl)-5-sulfanylidene-4,5-dihydro-1H-1,2,4-triazol-4-yl]ethyl}benzoic acid;
- Compound 14. 2-{2-[3-(2-methylphenyl)-5-sulfanylidene-4,5-dihydro-1H-1,2,4-triazol-4-yl]ethyl}benzoic acid;
- Compound 15. 5-(3-phenyl-5-sulfanylidene-4,5-dihydro-1H-1,2,4-triazol-4-yl)pentanoic acid;
- Compound 16. 2-({[3-(naphthalen-2-yl)-5-sulfanylidene-4,5-dihydro-1H-1,2,4-triazol-4-yl]amino}methyl)benzoic acid;
- Compound 17. 2-{1-[(3-phenyl-5-sulfanylidene-4,5-dihydro-1H-1,2,4-triazo1-4-yl)methyl]cyclohexyl}acetic acid;
- Compound 18. 4-[3-(2-hydroxy-5-methoxyphenyl)-5-sulfanylidene-4,5-dihydro-1H-1,2,4-triazol-4-yl]butanoic acid;
- Compound 19. 4-[3-(naphthalen-2-yl)-5-sulfanylidene-4,5-dihydro-1H-1,2,4-triazol-4-yl]butanoic acid;
- Compound 20. 3-phenyl-4-[(1R,2S)-2-phenylcyclopropyl]-4,5-dihydro-1H-1,2,4-triazole-5-thione;
- Compound 21. 3-(4-chlorophenyl)-4-(3-phenyl-5-sulfanylidene-4,5-dihydro-1H-1,2,4-triazol-4-yl)butanoic acid;
- Compound 22. 2-{2-[3-(3-methoxyphenyl)-5-sulfanylidene-4,5-dihydro-1H-1,2,4-triazol-4-yl]ethyl}benzoic acid;
- Compound 23. 2-{2-[3-(4-methyl-1,2,3-thiadiazol-5-yl)-5-sulfanylidene-4,5-dihydro-1H-1,2,4-triazol-4-yl]ethyl}benzoic acid;
- Compound 24. 4-[3-(3-methoxyphenyl)-5-sulfanylidene-4,5-dihydro-1H-1,2,4-triazol-4-yl]butanoic acid;
- Compound 25. 2-{[(tert-butoxy)carbonyl]amino}-5-(3-phenyl-5-sulfanylidene-4,5-dihydro-1H-1,2,4-triazol-4-yl)pentanoic acid;
- Compound 26. 4-hexyl-3-phenyl-4,5-dihydro-1H-1,2,4-triazole-5-thione;
- Compound 27. 2-{2-[3-(5-chlorothiophen-2-yl)-5-sulfanylidene-4,5-dihydro-1H-1,2,4-triazol-4-yl]ethyl}benzoic acid;
- Compound 28. 3-phenyl-4-(2-{[2-(trifluoromethyl)quinolin-4-yl]sulfanyl}ethyl)-4,5-dihydro-1H-1,2,4-triazole-5-thione;
- Compound 29. 5-[3-(2-hydroxy-5-methoxyphenyl)-5-sulfanylidene-4,5-dihydro-1H-1,2,4-triazol-4-yl]pentanoic acid;
- Compound 30. 5-[3-(naphthalen-2-yl)-5-sulfanylidene-4,5-dihydro-1H-1,2,4-triazol-4-yl]pentanoic acid;
- Compound 31. 4-[2-(4-hydroxyphenyl)ethyl]-3-phenyl-4,5-dihydro-1H-1,2,4-triazole-5-thione;
- Compound 32. 4-butyl-3-phenyl-4,5-dihydro-1H-1,2,4-triazole-5-thione;
- Compound 33. 4-[2-(benzylsulfanyl)ethyl]-3-phenyl-4,5-dihydro-1H-1,2,4-triazole-5-thione;
- Compound 34. 4-[(3-phenyl-5-sulfanylidene-4,5-dihydro-1H-1,2,4-triazol-4-yl)methyl]cyclohexane-1-carboxylic acid;
- Compound 35. 2-{2-[3-(adamantan-1-yl)-5-sulfanylidene-4,5-dihydro-1H-1,2,4-triazol-4-yl]ethyl}benzoic acid;
- Compound 36. 4-[3-(quinolin-2-yl)-5-sulfanylidene-4,5-dihydro-1H-1,2,4-triazol-4-yl]butanoic acid;
- Compound 37. 5-Phenyl-4-[(3-phthalideyl)methyl]-2,4-dihydro-1,2,4-triazole-3-thione;
- Compound 38. 5-(3-Biphenyl)-4-[3-carboxypropanyl]-1,2,4-triazole-3-thione;
- Compound 39. 5-(3-Biphenyl)-4-[2-(2-carboxyphenyl)ethyl]-1,2,4-triazole-3-thione;
- Compound 40. 5-(4-Benzyloxyphenyl)-4-[2-(2-carboxyphenyl)ethyl]-1,2,4-triazole-3-thione;
- Compound 41. 4-[2-(2,4-Dihydroxyphenyl)ethyl]-5-phenyl-1,2,4-triazole-3-thione;
- Compound 42. 5-(3-Biphenyl)-4-[2-(2,4-dihydroxyphenyl)ethyl]-1,2,4-triazole-3-thione;
- Compound 43. 5-(2-Biphenyl)-4-[2-(2,4-dihydroxyphenyl)ethyl]-1,2,4-triazole-3-thione;
- Compound 44. *o*-{2-[3-Methyl-*1H*-pyrrol-2-yl)-5-thioxo-1,4-dihydro-1,2,4-triazol-4-yl]ethyl}benzoic acid;
- Compound 45. 6-(3-Phenyl-5-sulfanylidene-4,5-dihydro-1H-1,2,4-triazol-4-yl)hexanoic acid;
- Compound 46. 3-Phenyl-4-(3-phenyl-5-thioxo-1,4-dihydro-1,2,4-triazol-4-yl)butyric acid;
- Compound 47. 3-(3-Phenyl-5-sulfanylidene-4,5-dihydro-1H-1,2,4-triazol-4-yl)cyclohexane-1-carboxylic acid;
- Compound 48. 4-[3-(1-Methyl-1*H*-pyrrol-2-yl)-5-thioxo-1,4-dihydro-1,2,4-triazol-4-yl]butyric acid;
- Compound 49. 4-(2-Azidoethyl)-3-phenyl-4,5-dihydro-1H-1,2,4-triazole-5-thione;
- Compound 50. 4-[3-(Quinolin-2-yl)-5-sulfanylidene-4,5-dihydro-1H-1,2,4-triazol-4-yl]butanoic acid;
- Compound 51. *o-*{[3-(3-Biphenylyl)-5-thioxo-1,4-dihydro-1,2,4-triazol-4-ylamino]methyl} benzoic acid;
- Compound 52. *m*-[(3-Phenyl-5-thioxo-1,4-dihydro-1,2,4-triazol-4-yl)methyl]benzoic acid;
- Compound 53. 5-Hydroxy-2-[2-(3-phenyl-5-thioxo-1,4-dihydro-1,2,4-triazol-4-yl)ethyl]benzoic acid;
- Compound 54. 4-[4-(Benzyloxy)phenylethyl]-5-phenyl-1,2,4-triazole-3-thione;
- Compound 55. *p*-[(3-Phenyl-5-thioxo-1,4-dihydro-1,2,4-triazol-4-yl)methyl]benzoic acid;
- Compound 56. *o-*{2-[3-(6-Quinolyl)-5-thioxo-1,4-dihydro-1,2,4-triazol-4-yl]ethyl}benzoic acid;
- Compound 57. *o*-{2-[3-(1*H*-Pyrrol-2-yl)-5-thioxo-1,4-dihydro-1,2,4-triazol-4-yl]ethyl}benzoic acid;
- Compound 58. *o-*{2-[3-(3-Thienyl)-5-thioxo-1,4-dihydro-1,2,4-triazol-4-yl]ethyl}benzoic acid;
- Compound 59. 8-(3-Phenyl-5-thioxo-1,4-dihydro-1,2,4-triazol-4-yl)octanoic acid;
- Compound 60. Phenyl[2-(3-phenyl-5-thioxo-1,4-dihydro-1,2,4-triazol-4-yl)ethylthio]acetic acid;
- Compound 61. 5-Amino-2-[2-(3-phenyl-5-thioxo-1,4-dihydro-1,2,4-triazol-4-yl)ethyl]benzoic acid.

In a particular embodiment, the compound of formula (I) is selected from the group consisting of:
- Compound 1. 2-[2-(3-phenyl-5-sulfanylidene-4,5-dihydro-1H-1,2,4-triazol-4-yl)ethyl]benzoic acid;
- Compound 2. 4-(3-phenyl-5-sulfanylidene-4,5-dihydro-1H-1,2,4-triazol-4-yl)butanoic acid;
- Compound 4. 2-{2-[3-(2-hydroxy-5-methoxyphenyl)-5-sulfanylidene-4,5-dihydro-1H-1,2,4-triazol-4-yl]ethyl}benzoic acid;
- Compound 5. 2-{[(3-phenyl-5-sulfanylidene-4,5-dihydro-1H-1,2,4-triazol-4-yl)amino]methyl}benzoic acid;
- Compound 12. 2-{2-[3-(furan-2-yl)-5-sulfanylidene-4,5-dihydro-1H-1,2,4-triazol-4-yl]ethyl}benzoic acid;
- Compound 13. 2-{2-[3-(thiophen-2-yl)-5-sulfanylidene-4,5-dihydro-1H-1,2,4-triazol-4-yl]ethyl}benzoic acid;
- Compound 14. 2-{2-[3-(2-methylphenyl)-5-sulfanylidene-4,5-dihydro-1H-1,2,4-triazol-4-yl]ethyl}benzoic acid;
- Compound 15. 5-(3-phenyl-5-sulfanylidene-4,5-dihydro-1H-1,2,4-triazol-4-yl)pentanoic acid.

In a particular embodiment, the compound of formula (I) is selected from the group consisting of:
- Compound 33. 4-[2-(benzylsulfanyl)ethyl]-3-phenyl-4,5-dihydro-1H-1,2,4-triazole-5-thione;
- Compound 37. 5-Phenyl-4-[(3-phthalideyl)methyl]-2,4-dihydro-1,2,4-triazole-3-thione;
- Compound 39. 5-(3-Biphenyl)-4-[2-(2-carboxyphenyl)ethyl]-1,2,4-triazole-3-thione; and
- Compound 42. 5-(3-Biphenyl)-4-[2-(2,4-dihydroxyphenyl)ethyl]-1,2,4-triazole-3-thione.

In a particular embodiment, the compound of formula (I) is selected from the group consisting of:
- Compound 12. 2-{2-[3-(furan-2-yl)-5-sulfanylidene-4,5-dihydro-1H-1,2,4-triazol-4-yl]ethyl}benzoic acid;
- Compound 13. 2-{2-[3-(thiophen-2-yl)-5-sulfanylidene-4,5-dihydro-1H-1,2,4-triazol-4-yl]ethyl}benzoic acid;
- Compound 14. 2-{2-[3-(2-methylphenyl)-5-sulfanylidene-4,5-dihydro-1H-1,2,4-triazol-4-yl]ethyl}benzoic acid.

### DETAILED DESCRIPTION OF THE INVENTION

### Definitions

The terms mentioned herein with prefixes such as for example C₁-C₃, C₁-C₆ or C₂-C₆ can also be used with lower numbers of carbon atoms such as C₁-C₂, C₁-C₅, or C₂-C₅. If, for example, the term C₁-C₃ is used, it means that the corresponding hydrocarbon chain may comprise from 1 to 3 carbon atoms, especially 1, 2 or 3 carbon atoms. If, for example, the term C₁-C₆ is used, it means that the corresponding hydrocarbon chain may comprise from 1 to 6 carbon atoms, especially 1, 2, 3, 4, 5 or 6 carbon atoms. If, for example, the term C₂-C₆ is used, it means that the corresponding hydrocarbon chain may comprise from 2 to 6 carbon atoms, especially 2, 3, 4, 5 or 6 carbon atoms.

The term "alkyl" refers to a saturated, linear or branched aliphatic group. The term "(C₁-C₃)alkyl" more specifically means methyl, ethyl, propyl, or isopropyl. The term "(C₁-C₆)alkyl" more specifically means methyl, ethyl, propyl, isopropyl, butyl, isobutyl, *tert*-butyl, pentyl or hexyl.

The term "alkoxy" or "alkyloxy" corresponds to the alkyl group as above defined bonded to the molecule by an -O- (ether) bond. (C₁-C₃)alkoxy includes methoxy, ethoxy, propyloxy, and isopropyloxy. (C₁-C₆)alkoxy includes methoxy, ethoxy, propyloxy, isopropyloxy, butyloxy, isobutyloxy, *tert*-butyloxy, pentyloxy and hexyloxy. In a preferred embodiment, the alkoxy is a methoxy.

The term "cycloalkyl" corresponds to a saturated or unsaturated mono-, bi- or tri-cyclic alkyl group comprising between 3 and 20 atoms of carbons. It also includes fused, bridged, or spiro-connected cycloalkyl groups. The term "cycloalkyl" includes for instance cyclopropyl, cyclobutyl, cyclopentyl, and cyclohexyl. The term "cycloalkyl" may also refer to a 5-10 membered bridged carbocyclyl such as bicyclo[2,2,1]heptanyl, bicyclo[2,2,2]octanyl, or adamantanyl. In a preferred embodiment, the cycloalkyl is an adamantanyl or cyclohexyl.

The term "heterocycloalkyl" corresponds to a saturated or unsaturated cycloalkyl group as above defined further comprising at least one heteroatom or heteroatomic group such as nitrogen, oxygen, or sulphur atom. It also includes fused, bridged, or spiro-connected heterocycloalkyl groups. Representative heterocycloalkyl groups include, but are not limited to 3-dioxolane, benzo [1,3] dioxolyl, pyrazolinyl, pyranyl, thiomorpholinyl, pyrazolidinyl, piperidyl, piperazinyl, 1,4-dioxanyl, imidazolinyl, pyrrolinyl, pyrrolidinyl, piperidinyl, imidazolidinyl, morpholinyl, 1,4-dithianyl, pyrrolidinyl, quinolizinyl, oxozolinyl, oxazolidinyl, isoxazolinyl, isoxazolidinyl, thiazolinyl, thiazolidinyl, isothiazolinyl, isothiazolidinyl, dihydropyranyl, tetrahydro-2H-pyranyl, tetrahydrofuranyl, and tetrahydrothiophenyl. The term "heterocycloalkyl" may also refer to a 5-10 membered bridged heterocyclyl such as 7-oxabicyclo[2,2,1]heptanyl. In a preferred embodiment, the heterocycloalkyl is morpholinyl.

The term "aryl" corresponds to a mono- or bi-cyclic aromatic hydrocarbons having from 5 to 14 carbon atoms. Preferably, the term "aryl" refers to phenyl, biphenyl, or naphthalenyl. The term "biphenyl" can be used for "a phenyl substituted by a phenyl".

The term "heteroaryl" as used herein corresponds to an aromatic, mono- or poly-cyclic group comprising between 5 and 14 atoms and comprising one or more heteroatoms, such as nitrogen (N), oxygen (O) or sulphur (S) atom, or heteroatomic groups. Examples of such mono- and poly-cyclic heteroaryl group may be: pyridinyl, thiazolyl, thiophenyl, furanyl, pyrrolyl, imidazolyl, triazolyl, tetrazolyl, benzofuranyl, thianaphthalenyl, indolyl, indolinyl, quinolinyl, isoquinolinyl, benzimidazolyl, triazinyl, thianthrenyl, isobenzofuranyl, phenoxanthinyl, isothiazolyl, isoxazolyl, pyrazinyl, pyridazinyl, indolizinyl, isoindolyl, indazolyl, purinyl, phtalazinyl, naphthyridinyl, quinoxalinyl, quinazolinyl, cinnolinyl, pteridinyl, carbazolyl, β-carbolinyl, phenanthridinyl, acridinyl, pyrimidinyl, phenanthrolinyl, phenazinyl, phenothiazinyl, furazanyl, phenoxazinyl, benzotriazolyl, benzoxazolyl, benzisoxazolyl, oxindolyl, benzothienyl, benzothiazolyl, s-triazinyl, oxazolyl, or thiofuranyl. In a preferred embodiment, the heteroaryl is furanyl, pyrrolyl, thiophenyl or quinolinyl.

The term "heterocycle" corresponds to an alicyclic or aromatic, mono- or poly-cyclic, hydrocarbon which contains one or more heteroatoms, such as oxygen (O), nitrogen (N) or sulphur atom (S), or heteroatomic groups, and optionally comprising one or more oxo groups. Heterocycles include, but are not limited to, heteroaryl, heterocycloalkyl, and heterocyclic derivatives. The "heterocyclic derivatives" are selected from the group consisting of isoindolinyl, indolinyl, chromanyl, isochromanyl, phthalidyl, pyrrolidinonyl, imidazolidinonyl, chromenyl, tetrahydroquinolinyl, tetrahydroisoquinolinyl, xanthenyl, benzoxazolinyl, isatinyl, and dihydropyridyl, preferably phthalidyl.

The term "arylalkyloxy" or "arylalkoxy" as used herein corresponds to an alkyloxy as defined above, substituted with an aryl group as defined above. More specifically, the "term aryl(C₁-C6)alkyloxy" corresponds to an arylalkyloxy, the alkyloxy of which is a (C₁-C₆)alkyloxy. In a preferred embodiment, the "aryl(C₁-C₆)alkyloxy" is a benzyloxy.

The term "halogen" corresponds to a fluorine, chlorine, bromine, or iodine atom, preferably a fluorine, chlorine or bromine atom.

The term "heteroatomic group" refers to a heteroatom optionally substituted with one or more hydrogens. Examples of heteroatomic group may be: O, S, Se, N, NH or Si. In a preferred embodiment, the heteroatomic group is O, N, NH, or S.

The term "Boc" refers to a tert-butoxycarbonyl group.

The expression "substituted by at least" means that the radical is substituted by one or several groups of the list.

As used herein, the terms "treatment", "treat" or "treating" refer to any act intended to ameliorate the health status of patients such as therapy, prevention, prophylaxis and retardation of a disease, in particular a bacterial infection. In certain embodiments, such terms refer to the amelioration or eradication of the disease, or symptoms associated with it. In other embodiments, this term refers to minimizing the spread or worsening of the disease, resulting from the administration of one or more therapeutic agents to a subject with such a disease.

As used herein, the terms "subject", "individual" or "patient" are interchangeable and refer to an animal, preferably to a mammal, even more preferably to a human, including adult, child, newborn and human at the prenatal stage. However, the term "subject" can also refer to non-human animals, in particular mammals such as dogs, cats, horses, cows, pigs, sheep and non-human primates, among others.

The terms "quantity," "amount," and "dose" are used interchangeably herein and may refer to an absolute quantification of a molecule.

As used herein, the terms "active principle", "active ingredient" and "active pharmaceutical ingredient" are equivalent and refers to a component of a pharmaceutical composition having a therapeutic effect.

As used herein, the term "therapeutic effect" refers to an effect induced by an active ingredient, or a pharmaceutical composition according to the invention, capable to prevent or to delay the appearance of a disease, or to cure or to attenuate the effects of a disease.

As used herein, the term "effective amount" refers to a quantity of an active ingredient or of a pharmaceutical composition which prevents, removes or reduces the deleterious effects of the disease. It is obvious that the quantity to be administered can be adapted by the man skilled in the art according to the subject to be treated, to the nature of the disease, etc. In particular, doses and regimen of administration may be function of the nature, of the stage and of the severity of the disease to be treated, as well as of the weight, the age and the global health of the subject to be treated, as well as of the judgment of the doctor.

As used herein, the term "excipient or pharmaceutically acceptable carrier" refers to any ingredient except active ingredients that is present in a pharmaceutical composition. Its addition may be aimed to confer a particular consistency or other physical or gustative properties to the final product. An excipient or pharmaceutically acceptable carrier must be devoid of any interaction, in particular chemical, with the active ingredients.

### Compounds

It is the purpose of the present invention to provide compounds, or pharmaceutically acceptable salts thereof, especially for use as MBL inhibitors, said compounds having the formula (I) as shown below, 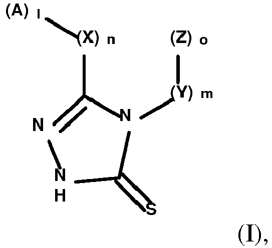 wherein,
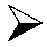 n and m are independently 0 or 1;
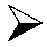 l is 1 or 2;
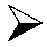 o is 0, 1 or 2;
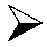 when m is 0, o is 1;
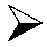 when n is 0,1 is 1;
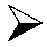 when l is 2, n is 1;
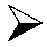 when o is 2, m is 1;
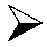 (X) represents a (C₁-C₃)alkyl;
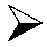 (A) represents a 5-14 membered ring selected in the group consisting of an aryl, a heteroaryl (preferably a furanyl, a thiophenyl, a thiadiazolyl, a quinolinyl, or a pyrrolyl), a cycloalkyl (preferably adamantanyl), said 5-14 membered ring is optionally substituted by at least one radical selected in the group consisting of:
   ▪ a halogen, preferably a chlorine, a fluorine or a bromine,
   ▪ OH,
   ▪ a (C₁-C₆)alkoxy, optionally substituted by a heterocycloalkyl, preferably a methoxy or a morpholinylethoxy,
   ▪ a (C₁-C₆)alkyl, optionally substituted by at least one halogen, preferably a fluorine, and
   ▪ an aryl (preferably a phenyl) or aryl(C₁-C₆)alkyloxy (preferably a benzyloxy);
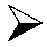 (Y) represents a (C₁-C₁₀)alkyl chain, comprising optionally a heteroatomic group;
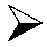 (Z) represents
   ∘ a 5-14 membered ring selected in the group consisting of an aryl (preferably a phenyl), a cycloalkyl (preferably a cyclohexyl or cyclopropyl), a heterocycle, preferably a heteroaryl (preferably a quinolinyl), a heterocycloalkyl (preferably a morpholinyl) or a heterocyclic derivative (preferably a phtalidyl), said 5-14 membered ring being optionally substituted by at least one radical selected in the group consisting of:
      ▪ a halogen, preferably a chlorine, a fluorine or a bromine,
      ▪ OH, COOH, NO₂, NH₂,
      ▪ a (C₁-C₆)alkyl, optionally substituted by at least one halogen, preferably a fluorine, or by COOH,
      ▪ an aryl (preferably a phenyl) optionally substituted by NO₂, or aryl(C₁-C₆)alkyloxy (preferably a benzyloxy);
   ∘ A radical selected in the group consisting of:
      ▪ COOH,
      ▪ N₃,
      ▪ a NR₁R₂ group, wherein R₁ and R₂ represent independently H, Boc, (C₁-C₆)alkyl,
      ▪ SO₂-R₇, wherein R₇ represents a (C₁-C₆)alkyl, or a 5-14 membered ring, preferably an aryl, optionally substituted by an (C₁-C₆)alkyl,
      ▪ a benzoyl, and
      ▪ N-phenylurea; or
   ∘ a (C₁-C₆)alkyl, optionally substituted by at least one halogen, preferably a fluorine, or by COOH.

In the present application, when n is 1 and l is 1, the moiety (X)ₙ-(A)ₗ is represented as follows: 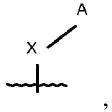 wherein X is a (C1-C3)alkyl chain and A, as defined above, replaces any one of the hydrogens of said (C1-C3)alkyl chain. Preferably, X is a linear (C1-C3)alkyl chain.

In the present application, when n is 1 and l is 2, the moiety (X)ₙ-(A)ₗ is represented as follows: 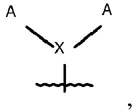 wherein X is a (C1-C3)alkyl chain and each of A's, as defined above, independently replaces any one of the hydrogens of said (C1-C3)alkyl chain.

Preferably, A is (are) in terminal position of said (C1-C3)alkyl chain, i.e. on the terminal carbon of said (C1-C3)alkyl chain.

For instance, when:
- X is a methyl, X-A is -CH₂-A and X-(A)₂ is -CH(A)₂;
- X is an ethyl, X-A is -CH₂-CH₂-A and X-(A)₂ is -CH₂-CH(A)₂;
- X is a propyl, X-A is -CH₂-CH₂-CH₂-A and X-(A)₂ is -CH₂-CH₂-CH(A)₂.

In the same way, in the present application, when m is 1 and o is 0, the moiety (Y)ₘ-(Z)ₒ is represented as follows: 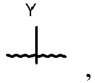 wherein Y is a (C1-C10)alkyl chain, comprising optionally a heteroatomic group. Preferably, Y is a linear (C1-C10)alkyl chain, comprising optionally a heteroatomic group.

When m is 1 and o is 1, the moiety (Y)ₘ-(Z)ₒ is represented as follows: 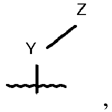 wherein Y is a (C1-C10)alkyl chain, comprising optionally a heteroatomic group, and wherein Z, as defined above, replaces any one of the hydrogens of said (C1-C3)alkyl chain.

When m is 1 and o is 2, the moiety (Y)ₘ-(Z)ₒ is represented as follows: 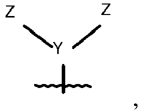 wherein Y is a (C1-C10)alkyl chain, comprising optionally a heteroatomic group, and wherein each of Z's, as defined above, independently replaces any one of the hydrogens of said (C1-C3)alkyl chain.

Preferably, Z is (are) in terminal position of said (C1-C10)alkyl chain, i.e. on the terminal carbon of said (C1-C10)alkyl chain or on the heteroatomic group when said heteroatomic group is on the terminal carbon of (C1-C10)alkyl chain.

Generally speaking:
- when m is 0, then o is 1 and thus Z is linked directly to the 1,2,4-triazole-5-thione cycle;
- when o is 2, then m is 1;
- when n is 0, then l is 1 and thus, A is linked directly to the 1,2,4-triazole-5-thione cycle;
- when l is 2, then n is 1.

In the present application, Y represents a (C₁-C₁₀)alkyl chain, comprising optionally a heteroatomic group. It is understood that the heteroatomic group, may be at any position of the (C₁-C₁₀)alkyl chain. In one embodiment, the heteroatomic group is at one of the two ends of the (C₁-C₁₀)alkyl chain, i.e. the heteroatomic group connects the 1,2,4-triazole-5-thione moiety of the compound of formula (I) with the (C₁-C₁₀)alkyl chain, or the heteroatomic group connects the (C₁-C₁₀)alkyl chain with one (if o =1) or two (if o = 2) Z groups. In another embodiment, the heteroatomic group is at any position of the (C₁-C₁₀)alkyl chain, except at one of its two ends, i.e. the heteroatomic group interrupts the (C₁-C₁₀)alkyl chain.

The compound of formula (I) may exist in the form of or in equilibrium with another tautomeric form, which is represented by the following formula (I'): 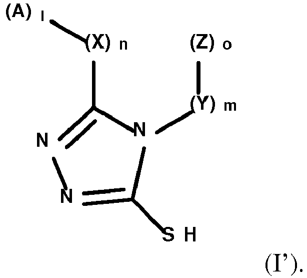 Thus, the present invention also encompasses such tautomeric form of compound of formula (I).

In a particular embodiment, the compound of formula (I) is selected from the group consisting of:
- Compound 1. 2-[2-(3-phenyl-5-sulfanylidene-4,5-dihydro-1H-1,2,4-triazol-4-yl)ethyl]benzoic acid;
- Compound 2. 4-(3-phenyl-5-sulfanylidene-4,5-dihydro-1H-1,2,4-triazol-4-yl)butanoic acid;
- Compound 3. 2-{2-[3-(naphthalen-2-yl)-5-sulfanylidene-4,5-dihydro-1H-1,2,4-triazol-4-yl]ethyl}benzoic acid;
- Compound 4. 2-{2-[3-(2-hydroxy-5-methoxyphenyl)-5-sulfanylidene-4,5-dihydro-1H-1,2,4-triazol-4-yl]ethyl}benzoic acid;
- Compound 5. 2-{[(3-phenyl-5-sulfanylidene-4,5-dihydro-1H-1,2,4-triazol-4-yl)amino]methyl}benzoic acid;
- Compound 6. 3-(2-methylphenyl)-4-(2-phenylethyl)-4,5-dihydro-1H-1,2,4-triazole-5-thione;
- Compound 7. 4-benzyl-3-(2-hydroxy-5-methoxyphenyl)-4,5-dihydro-1H-1,2,4-triazole-5-thione;
- Compound 8. 3-phenyl-4-(3-phenylpropyl)-4,5-dihydro-1H-1,2,4-triazole-5-thione;
- Compound 9. 4-benzyl-3-phenyl-4,5-dihydro-1H-1,2,4-triazole-5-thione;
- Compound 10. 3-(2-hydroxy-5-methoxyphenyl)-4-(2-phenylethyl)-4,5-dihydro-1H-1,2,4-triazole-5-thione;
- Compound 11. 3-phenyl-4-(2-phenylethyl)-4,5-dihydro-1H-1,2,4-triazole-5-thione;
- Compound 12. 2-{2-[3-(furan-2-yl)-5-sulfanylidene-4,5-dihydro-1H-1,2,4-triazol-4-yl]ethyl}benzoic acid;
- Compound 13. 2-{2-[3-(thiophen-2-yl)-5-sulfanylidene-4,5-dihydro-1H-1,2,4-triazol-4-yl]ethyl}benzoic acid;
- Compound 14. 2-{2-[3-(2-methylphenyl)-5-sulfanylidene-4,5-dihydro-1H-1,2,4-triazol-4-yl]ethyl}benzoic acid;
- Compound 15. 5-(3-phenyl-5-sulfanylidene-4,5-dihydro-1H-1,2,4-triazol-4-yl)pentanoic acid;
- Compound 16. 2-({[3-(naphthalen-2-yl)-5-sulfanylidene-4,5-dihydro-1H-1,2,4-triazol-4-yl] amino }methyl)benzoic acid;
- Compound 17. 2-{1-[(3-phenyl-5-sulfanylidene-4,5-dihydro-1H-1,2,4-triazol-4-yl)methyl]cyclohexyl}acetic acid;
- Compound 18. 4-[3-(2-hydroxy-5-methoxyphenyl)-5-sulfanylidene-4,5-dihydro-1H-1,2,4-triazol-4-yl]butanoic acid;
- Compound 19. 4-[3-(naphthalen-2-yl)-5-sulfanylidene-4,5-dihydro-1H-1,2,4-triazol-4-yl]butanoic acid;
- Compound 20. 3-phenyl-4-[(1R,2S)-2-phenylcyclopropyl]-4,5-dihydro-1H-1,2,4-triazole-5-thione;
- Compound 21. 3-(4-chlorophenyl)-4-(3-phenyl-5-sulfanylidene-4,5-dihydro-1H-1,2,4-triazol-4-yl)butanoic acid;
- Compound 22. 2-{2-[3-(3-methoxyphenyl)-5-sulfanylidene-4,5-dihydro-1H-1,2,4-triazol-4-yl]ethyl}benzoic acid;
- Compound 23. 2-{2-[3-(4-methyl-1,2,3-thiadiazol-5-yl)-5-sulfanylidene-4,5-dihydro-1H-1,2,4-triazol-4-yl]ethyl}benzoic acid;
- Compound 24. 4-[3-(3-methoxyphenyl)-5-sulfanylidene-4,5-dihydro-1H-1,2,4-triazol-4-yl]butanoic acid;
- Compound 25. 2-{[(tert-butoxy)carbonyl]amino}-5-(3-phenyl-5-sulfanylidene-4,5-dihydro-1H-1,2,4-triazol-4-yl)pentanoic acid;
- Compound 26. 4-hexyl-3-phenyl-4,5-dihydro-1H-1,2,4-triazole-5-thione;
- Compound 27. 2-{2-[3-(5-chlorothiophen-2-yl)-5-sulfanylidene-4,5-dihydro-1H-1,2,4-triazol-4-yl]ethyl}benzoic acid;
- Compound 28. 3-phenyl-4-(2-{[2-(trifluoromethyl)quinolin-4-yl]sulfanyl}ethyl)-4,5-dihydro-1H-1,2,4-triazole-5-thione;
- Compound 29. 5-[3-(2-hydroxy-5-methoxyphenyl)-5-sulfanylidene-4,5-dihydro-1H-1,2,4-triazol-4-yl]pentanoic acid;
- Compound 30. 5-[3-(naphthalen-2-yl)-5-sulfanylidene-4,5-dihydro-1H-1,2,4-triazol-4-yl]pentanoic acid;
- Compound 31. 4-[2-(4-hydroxyphenyl)ethyl]-3-phenyl-4,5-dihydro-1H-1,2,4-triazole-5-thione;
- Compound 32. 4-butyl-3-phenyl-4,5-dihydro-1H-1,2,4-triazole-5-thione;
- Compound 33. 4-[2-(benzylsulfanyl)ethyl]-3-phenyl-4,5-dihydro-1H-1,2,4-triazole-5-thione;
- Compound 34. 4-[(3-phenyl-5-sulfanylidene-4,5-dihydro-1H-1,2,4-triazol-4-yl)methyl]cyclohexane-1-carboxylic acid;
- Compound 35. 2-{2-[3-(adamantan-1-yl)-5-sulfanylidene-4,5-dihydro-1H-1,2,4-triazol-4-yl]ethyl}benzoic acid;
- Compound 36. 4-[3-(quinolin-2-yl)-5-sulfanylidene-4,5-dihydro-1H-1,2,4-triazol-4-yl]butanoic acid;
- Compound 37. 5-Phenyl-4-[(3-phthalideyl)methyl]-2,4-dihydro-1,2,4-triazole-3-thione;
- Compound 38. 5-(3-Biphenyl)-4-[3-carboxypropanyl]-1,2,4-triazole-3-thione;
- Compound 39. 5-(3-Biphenyl)-4-[2-(2-carboxyphenyl)ethyl]-1,2,4-triazole-3-thione;
- Compound 40. 5-(4-Benzyloxyphenyl)-4-[2-(2-carboxyphenyl)ethyl]-1,2,4-triazole-3-thione;
- Compound 41. 4-[2-(2,4-Dihydroxyphenyl)ethyl]-5-phenyl-1,2,4-triazole-3-thione;
- Compound 42. 5-(3-Biphenyl)-4-[2-(2,4-dihydroxyphenyl)ethyl]-1,2,4-triazole-3-thione;
- Compound 43. 5-(2-Biphenyl)-4-[2-(2,4-dihydroxyphenyl)ethyl]-1,2,4-triazole-3-thione;
- Compound 44. *o*-{2-[3-Methyl-*1H*-pyrrol-2-yl)-5-thioxo-1,4-dihydro-1,2,4-triazol-4-yl]ethyl}benzoic acid;
- Compound 45. 6-(3-Phenyl-5-sulfanylidene-4,5-dihydro-1H-1,2,4-triazol-4-yl)hexanoic acid;
- Compound 46. 3-Phenyl-4-(3-phenyl-5-thioxo-1,4-dihydro-1,2,4-triazol-4-yl)butyric acid;
- Compound 47. 3-(3-Phenyl-5-sulfanylidene-4,5-dihydro-1H-1,2,4-triazol-4-yl)cyclohexane-1-carboxylic acid;
- Compound 48. 4-[3-(1-Methyl-1*H*-pyrrol-2-yl)-5-thioxo-1,4-dihydro-1,2,4-triazol-4-yl]butyric acid;
- Compound 49. 4-(2-Azidoethyl)-3-phenyl-4,5-dihydro-1H-1,2,4-triazole-5-thione;
- Compound 50. 4-[3-(Quinolin-2-yl)-5-sulfanylidene-4,5-dihydro-1H-1,2,4-triazol-4-yl]butanoic acid;
- Compound 51. *o-*{[3-(3-Biphenylyl)-5-thioxo-1,4-dihydro-1,2,4-triazol-4-ylamino]methyl} benzoic acid;
- Compound 52. *m*-[(3-Phenyl-5-thioxo-1,4-dihydro-1,2,4-triazol-4-yl)methyl]benzoic acid;
- Compound 53. 5-Hydroxy-2-[2-(3-phenyl-5-thioxo-1,4-dihydro-1,2,4-triazol-4-yl)ethyl]benzoic acid;
- Compound 54. 4-[4-(Benzyloxy)phenylethyl]-5-phenyl-1,2,4-triazole-3-thione;
- Compound 55. *p*-[(3-Phenyl-5-thioxo-1,4-dihydro-1,2,4-triazol-4-yl)methyl]benzoic acid;
- Compound 56. *o-*{2-[3-(6-Quinolyl)-5-thioxo-1,4-dihydro-1,2,4-triazol-4-yl]ethyl}benzoic acid;
- Compound 57. *o*-{2-[3-(1*H*-Pyrrol-2-yl)-5-thioxo-1,4-dihydro-1,2,4-triazol-4-yl]ethyl}benzoic acid;
- Compound 58. *o-*{2-[3-(3-Thienyl)-5-thioxo-1,4-dihydro-1,2,4-triazol-4-yl]ethyl}benzoic acid;
- Compound 59. 8-(3-Phenyl-5-thioxo-1,4-dihydro-1,2,4-triazol-4-yl)octanoic acid;
- Compound 60. Phenyl[2-(3-phenyl-5-thioxo-1,4-dihydro-1,2,4-triazol-4-yl)ethylthio]acetic acid;
- Compound 61. 5-Amino-2-[2-(3-phenyl-5-thioxo-1,4-dihydro-1,2,4-triazol-4-yl)ethyl]benzoic acid.

In a particular embodiment, n is 1 and X is a methyl or an ethyl.

In another particular embodiment, n is 0 and A is an aryl selected in the group consisting of phenyl optionally substituted by a phenyl or a benzyloxy, and a naphthalenyl. Preferably, the compound of formula (I) is selected from the group consisting of: 2-[2-(3-phenyl-5-sulfanylidene-4,5-dihydro-1H-1,2,4-triazol-4-yl)ethyl]benzoic acid; 4-(3-phenyl-5-sulfanylidene-4,5-dihydro-1H-1,2,4-triazol-4-yl)butanoic acid; 2-{2-[3-(naphthalen-2-yl)-5-sulfanylidene-4,5-dihydro-1H-1,2,4-triazol-4-yl]ethyl}benzoic acid; 2-{ [(3-phenyl-5-sulfanylidene-4,5-dihydro-1H-1,2,4-triazol-4-yl)amino]methyl}benzoic acid; 3-phenyl-4-(3-phenylpropyl)-4,5-dihydro-1H-1,2,4-triazole-5-thione; 4-benzyl-3-phenyl-4,5-dihydro-1H-1,2,4-triazole-5-thione; 5-(3-phenyl-5-sulfanylidene-4,5-dihydro-1H-1,2,4-triazol-4-yl)pentanoic acid; 2-({[3-(naphthalen-2-yl)-5-sulfanylidene-4,5-dihydro-1H-1,2,4-triazol-4-yl]amino}methyl)benzoic acid; 4-[3-(naphthalen-2-yl)-5-sulfanylidene-4,5-dihydro-1H-1,2,4-triazol-4-yl]butanoic acid; 3-(4-chlorophenyl)-4-(3-phenyl-5-sulfanylidene-4,5-dihydro-1H-1,2,4-triazol-4-yl)butanoic acid; 2-{[(tert-butoxy)carbonyl]amino}-5-(3-phenyl-5-sulfanylidene-4,5-dihydro-1H-1,2,4-triazol-4-yl)pentanoic acid; 4-hexyl-3-phenyl-4,5-dihydro-1H-1,2,4-triazole-5-thione; 5-[3-(naphthalen-2-yl)-5-sulfanylidene-4,5-dihydro-1H-1,2,4-triazol-4-yl]pentanoic acid; 4-[2-(4-hydroxyphenyl)ethyl]-3-phenyl-4,5-dihydro-1H-1,2,4-triazole-5-thione; 4-butyl-3-phenyl-4,5-dihydro-1H-1,2,4-triazole-5-thione; 4-[2-(benzylsulfanyl)ethyl]-3-phenyl-4,5-dihydro-1H-1,2,4-triazole-5-thione; 5-Phenyl-4-[(3-phthalideyl)methyl]-2,4-dihydro-1,2,4-triazole-3-thione; 5-(3-Biphenyl)-4-[3-carboxypropanyl]-1,2,4-triazole-3-thione; 5-(3-Biphenyl)-4-[2-(2-carboxyphenyl)ethyl]-1,2,4-triazole-3-thione; 5-(4-Benzyloxyphenyl)-4-[2-(2-carboxyphenyl)ethyl]-1,2,4-triazole-3-thione; 5-(3-Biphenyl)-4-[2-(2,4-dihydroxyphenyl)ethyl]-1,2,4-triazole-3-thione; 6-(3-Phenyl-5-sulfanylidene-4,5-dihydro-1H-1,2,4-triazol-4-yl)hexanoic acid; *m*-[(3-Phenyl-5-thioxo-1,4-dihydro-1,2,4-triazol-4-yl)methyl]benzoic acid; 5-Hydroxy-2-[2-(3-phenyl-5-thioxo-1,4-dihydro-1,2,4-triazol-4-yl)ethyl]benzoic acid; *p*-[(3-Phenyl-5-thioxo-1,4-dihydro-1,2,4-triazol-4-yl)methyl]benzoic acid; 8-(3-Phenyl-5-thioxo-1,4-dihydro-1,2,4-triazol-4-yl)octanoic acid; and Phenyl[2-(3-phenyl-5-thioxo-1,4-dihydro-1,2,4-triazol-4-yl)ethylthio]acetic acid.

In a particular embodiment, the compound of formula (I) is selected from the group consisting of 4-[2-(benzylsulfanyl)ethyl]-3-phenyl-4,5-dihydro-1H-1,2,4-triazole-5-thione; 5-(3-Biphenyl)-4-[2-(2-carboxyphenyl)ethyl]-1,2,4-triazole-3-thione or 5-(3-Biphenyl)-4-[2-(2,4-dihydroxyphenyl)ethyl]-1,2,4-triazole-3-thione.

In a particular embodiment, n is 0 and A is a phenyl substituted by at least one radical selected in the group consisting of methyl, methoxy, OH, halogen (preferably CI). Preferably, the compound of formula (I) is selected from the group consisting of2-{2-[3-(2-hydroxy-5-methoxyphenyl)-5-sulfanylidene-4,5-dihydro-1H-1,2,4-triazol-4-yl]ethyl}benzoic acid; 3-(2-methylphenyl)-4-(2-phenylethyl)-4,5-dihydro-1H-1,2,4-triazole-5-thione; 4-benzyl-3-(2-hydroxy-5-methoxyphenyl)-4,5-dihydro-1H-1,2,4-triazole-5-thione; 3-(2-hydroxy-5-methoxyphenyl)-4-(2-phenylethyl)-4,5-dihydro-1H-1,2,4-triazole-5-thione; 2-{2-[3-(2-methylphenyl)-5-sulfanylidene-4,5-dihydro-1H-1,2,4-triazol-4-yl]ethyl}benzoic acid; 4-[3-(2-hydroxy-5-methoxyphenyl)-5-sulfanylidene-4,5-dihydro-1H-1,2,4-triazol-4-yl]butanoic acid; 2-{2-[3-(3-methoxyphenyl)-5-sulfanylidene-4,5-dihydro-1H-1,2,4-triazol-4-yl]ethyl}benzoic acid; 4-[3-(3-methoxyphenyl)-5-sulfanylidene-4,5-dihydro-1H-1,2,4-triazol-4-yl]butanoic acid; and 5-[3-(2-hydroxy-5-methoxyphenyl)-5-sulfanylidene-4,5-dihydro-1H-1,2,4-triazol-4-yl]pentanoic acid.

In a particular embodiment, n is 0 and A is a 5-membered ring selected in the group consisting of furanyl, thiophenyl, optionally substituted by a halogen, and pyrrolyl, optionally substituted by a methyl. Preferably, the compound of formula (I) is selected from the group consisting of: 2-{2-[3-(furan-2-yl)-5-sulfanylidene-4,5-dihydro-1H-1,2,4-triazol-4-yl]ethyl}benzoic acid; 2-{2-[3-(thiophen-2-yl)-5-sulfanylidene-4,5-dihydro-1H-1,2,4-triazol-4-yl]ethyl}benzoic acid; 2-{2-[3-(4-methyl-1,2,3-thiadiazol-5-yl)-5-sulfanylidene-4,5-dihydro-1H-1,2,4-triazol-4-yl]ethyl}benzoic acid; 2-{2-[3-(5-chlorothiophen-2-yl)-5-sulfanylidene-4,5-dihydro-1H-1,2,4-triazol-4-yl]ethyl}benzoic acid; *o*-{2-[3-Methyl-*1H*-pyrrol-2-yl)-5-thioxo-1,4-dihydro-1,2,4-triazol-4-yl]ethyl}benzoic acid; 4-[3-(1-Methyl-1*H*-pyrrol-2-yl)-5-thioxo-1,4-dihydro-1,2,4-triazol-4-yl]butyric acid; *o*-{2-[3-(1*H*-Pyrrol-2-yl)-5-thioxo-1,4-dihydro-1,2,4-triazol-4-yl]ethyl}benzoic acid; or *o-*{2-[3-(3-Thienyl)-5-thioxo-1,4-dihydro-1,2,4-triazol-4-yl]ethyl}benzoic acid.

In a particular embodiment, n is 0 and A is quinolinyl. Preferably, the compound of formula (I) is selected from the group consisting of: 4-[3-(quinolin-2-yl)-5-sulfanylidene-4,5-dihydro-1H-1,2,4-triazol-4-yl]butanoic acid; 4-[3-(Quinolin-2-yl)-5-sulfanylidene-4,5-dihydro-1H-1,2,4-triazol-4-yl]butanoic acid; or *o-*{2-[3-(6-Quinolyl)-5-thioxo-1,4-dihydro-1,2,4-triazol-4-yl]ethyl}benzoic acid.

In a particular embodiment, m is 1 and Y is selected in the group consisting of (C1-C7)alkyl chain, optionally comprising a heteroatomic group.

In a particular embodiment, o is 0, m is 1 and Y is (C1-C7)alkyl chain. Preferably, the compound of formula (I) is selected from the group consisting of: 4-hexyl-3-phenyl-4,5-dihydro-1H-1,2,4-triazole-5-thione or 4-butyl-3-phenyl-4,5-dihydro-1H-1,2,4-triazole-5-thione.

In a particular embodiment, m is 1, Y is (C1-C7)alkyl chain, o is 1, and Z is COOH. Preferably, the compound of formula (I) is selected from the group consisting of 4-(3-phenyl-5-sulfanylidene-4,5-dihydro-1H-1,2,4-triazol-4-yl)butanoic acid; 5-(3-phenyl-5-sulfanylidene-4,5-dihydro-1H-1,2,4-triazol-4-yl)pentanoic acid; 4-[3-(2-hydroxy-5-methoxyphenyl)-5-sulfanylidene-4,5-dihydro-1H-1,2,4-triazol-4-yl]butanoic acid; 4-[3-(naphthalen-2-yl)-5-sulfanylidene-4,5-dihydro-1H-1,2,4-triazol-4-yl]butanoic acid; 4-[3-(3-methoxyphenyl)-5-sulfanylidene-4,5-dihydro-1H-1,2,4-triazol-4-yl]butanoic acid; 5-[3-(2-hydroxy-5-methoxyphenyl)-5-sulfanylidene-4,5-dihydro-1H-1,2,4-triazol-4-yl]pentanoic acid; 5-[3-(naphthalen-2-yl)-5-sulfanylidene-4,5-dihydro-1H-1,2,4-triazol-4-yl]pentanoic acid; 5-(3-Biphenyl)-4-[3-carboxypropanyl]-1,2,4-triazole-3-thione; 6-(3-Phenyl-5-sulfanylidene-4,5-dihydro-1H-1,2,4-triazol-4-yl)hexanoic acid; 4-[3-(1-Methyl-1*H*-pyrrol-2-yl)-5-thioxo-1,4-dihydro-1,2,4-triazol-4-yl]butyric acid; 4-[3-(Quinolin-2-yl)-5-sulfanylidene-4,5-dihydro-1H-1,2,4-triazol-4-yl]butanoic acid or 8-(3-Phenyl-5-thioxo-1,4-dihydro-1,2,4-triazol-4-yl)octanoic acid.

In a particular embodiment, m is 1, Y is (C₁-C₇)alkyl chain, optionally comprising a heteroatomic group, o is 2 and Z is selected in the group consisting of:
- a (C₁-C₃)alkyl, preferably a methyl, optionally substituted by COOH,
- a phenyl optionally substituted by Cl,
- NH-Boc, COOH and N-phenylurea.

Preferably, the compound of formula (I) is selected from the group consisting of: 3-(4-chlorophenyl)-4-(3-phenyl-5-sulfanylidene-4,5-dihydro-1H-1,2,4-triazol-4-yl)butanoic acid; 2-{[(tert-butoxy)carbonyl]amino}-5-(3-phenyl-5-sulfanylidene-4,5-dihydro-1H-1,2,4-triazol-4-yl)pentanoic acid; 3-Phenyl-4-(3-phenyl-5-thioxo-1,4-dihydro-1,2,4-triazol-4-yl)butyric acid; or Phenyl[2-(3-phenyl-5-thioxo-1,4-dihydro-1,2,4-triazol-4-yl)ethylthio]acetic acid.

In a particular embodiment, o is 1 and Z is a cyclohexyl substituted by COOH or a (C1-C6)alkyl, preferably a methyl, substituted by COOH. Preferably, the compound of formula (I) is selected from the group consisting of: 2-{1-[(3-phenyl-5-sulfanylidene-4,5-dihydro-1H-1,2,4-triazol-4-yl)methyl]cyclohexyl}acetic acid; 4-[(3-phenyl-5-sulfanylidene-4,5-dihydro-1H-1,2,4-triazol-4-yl)methyl]cyclohexane-1-carboxylic acid; and 3-(3-Phenyl-5-sulfanylidene-4,5-dihydro-1H-1,2,4-triazol-4-yl)cyclohexane-1-carboxylic acid.

In a particular embodiment, m is 1 and Y is (C₁-C₇)alkyl chain, comprising optionally a heteroatomic group, and, o is 1 and Z is a phenyl substituted by a COOH, and optionally a OH or a NH₂. Preferably, the compound of formula (I) is selected from the group consisting of: 2-[2-(3-phenyl-5-sulfanylidene-4,5-dihydro-1H-1,2,4-triazol-4-yl)ethyl]benzoic acid; 2-{2-[3-(naphthalen-2-yl)-5-sulfanylidene-4,5-dihydro-1H-1,2,4-triazol-4-yl]ethyl}benzoic acid; 2-{2-[3-(2-hydroxy-5-methoxyphenyl)-5-sulfanylidene-4,5-dihydro-1H-1,2,4-triazol-4-yl]ethyl}benzoic acid; 2-{[(3-phenyl-5-sulfanylidene-4,5-dihydro-1H-1,2,4-triazol-4-yl)amino]methyl}benzoic acid; 2-{2-[3-(furan-2-yl)-5-sulfanylidene-4,5-dihydro-1H-1,2,4-triazol-4-yl]ethyl}benzoic acid; 2-{2-[3-(thiophen-2-yl)-5-sulfanylidene-4,5-dihydro-1H-1,2,4-triazol-4-yl]ethyl}benzoic acid; 2-{2-[3-(2-methylphenyl)-5-sulfanylidene-4,5-dihydro-1H-1,2,4-triazol-4-yl]ethyl}benzoic acid; 2-{2-[5-sulfanylidene-3-(thiophen-2-yl)-4,5-dihydro-1H-1,2,4-triazol-4-yl]ethyl}benzoic acid; 2-{2-[3-(2,2-diphenylethyl)-5-sulfanylidene-4,5-dihydro-1H-1,2,4-triazol-4-yl]ethyl}benzoic acid; 2-{2-[3-(3-methoxyphenyl)-5-sulfanylidene-4,5-dihydro-1H-1,2,4-triazol-4-yl]ethyl}benzoic acid; 2-{2-[3-(5-chlorothiophen-2-yl)-5-sulfanylidene-4,5-dihydro-1H-1,2,4-triazol-4-yl]ethyl}benzoic acid; 4-[3-(quinolin-2-yl)-5-sulfanylidene-4,5-dihydro-1H-1,2,4-triazol-4-yl]butanoic acid; 5-(3-Biphenyl)-4-[2-(2-carboxyphenyl)ethyl]-1,2,4-triazole-3-thione; 5-(4-Benzyloxyphenyl)-4-[2-(2-carboxyphenyl)ethyl]-1,2,4-triazole-3-thione; *o*-{2-[3-Methyl-*1H*-pyrrol-2-yl)-5-thioxo-1,4-dihydro-1,2,4-triazol-4-yl]ethyl}benzoic acid; *o*-[2-(3-Phenethyl-5-thioxo-1,4-dihydro-1,2,4-triazol-4-yl)ethyl]benzoic acid; *m*-[(3-Phenyl-5-thioxo-1,4-dihydro-1,2,4-triazol-4-yl)methyl]benzoic acid; 5-Hydroxy-2-[2-(3-phenyl-5-thioxo-1,4-dihydro-1,2,4-triazol-4-yl)ethyl]benzoic acid; *p*-[(3-Phenyl-5-thioxo-1,4-dihydro-1,2,4-triazol-4-yl)methyl]benzoic acid; *o*-{2-[3-(6-Quinolyl)-5-thioxo-1,4-dihydro-1,2,4-triazol-4-yl]ethyl}benzoic acid; *o*-{2-[3-(1*H*-Pyrrol-2-yl)-5-thioxo-1,4-dihydro-1,2,4-triazol-4-yl]ethyl}benzoic acid; *o*-{2-[3-(3-Thienyl)-5-thioxo-1,4-dihydro-1,2,4-triazol-4-yl]ethyl}benzoic acid; 5-(2-Biphenyl)-4-[2-(2-carboxyphenyl)ethyl]-1,2,4-triazole-3-thione; 2-({[3-(naphthalen-2-yl)-5-sulfanylidene-4,5-dihydro-1H-1,2,4-triazol-4-yl]amino}methyl) benzoic acid; *o*-{[3-(3-Biphenylyl)-5-thioxo-1,4-dihydro-1,2,4-triazol-4-ylamino]methyl} benzoic acid; 2-{2-[3-(adamantan-1-yl)-5-sulfanylidene-4,5-dihydro-1H-1,2,4-triazol-4-yl]ethyl}benzoic acid; 2-{[(3-phenyl-5-sulfanylidene-4,5-dihydro-1H-1,2,4-triazol-4-yl)amino]methyl}benzoic acid and 5-Amino-2-[2-(3-phenyl-5-thioxo-1,4-dihydro-1,2,4-triazol-4-yl)ethyl]benzoic acid.

More preferably, the compound of formula (I) is 5-(3-Biphenyl)-4-[2-(2-carboxyphenyl)ethyl]-1,2,4-triazole-3-thione.

In a particular embodiment, o is 1 and Z is a phenyl substituted by at least one OH. In a preferred embodiment, said phenyl is substituted by two OH. Preferably, the compound of formula (I) is selected from the group consisting of: 4-[2-(4-hydroxyphenyl)ethyl]-3-phenyl-4,5-dihydro-1H-1,2,4-triazole-5-thione; 4-[2-(2,4-Dihydroxyphenyl)ethyl]-5-phenyl-1,2,4-triazole-3-thione; 5-(3-Biphenyl)-4-[2-(2,4-dihydroxyphenyl)ethyl]-1,2,4-triazole-3-thione; 5-(2-Biphenyl)-4-[2-(2,4-dihydroxyphenyl)ethyl]-1,2,4-triazole-3-thione; or 5-Hydroxy-2-[2-(3-phenyl-5-thioxo-1,4-dihydro-1,2,4-triazol-4-yl)ethyl]benzoic acid. More preferably, the compound of formula (I) is 4-[2-(4-hydroxyphenyl)ethyl]-3-phenyl-4,5-dihydro-1H-1,2,4-triazole-5-thione or 5-(3-Biphenyl)-4- [2-(2,4-dihydroxyphenyl)ethyl]-1,2,4-triazole-3-thione.

In a particular embodiment, o is 1 and Z is a phenyl substituted by a benzyloxyphenyl. Preferably, the compound of formula (I) is 4-[4-(Benzyloxy)phenylethyl]-5-phenyl-1,2,4-triazole-3-thione.

In a particular embodiment, m is 1 and Y is a (C₁-C₅)alkyl chain, comprising a heteroatomic group. Preferably, said heteroatomic group is S. Preferably, the compound of formula (I) is selected from the group consisting of: 4-[2-(benzylsulfanyl)ethyl]-3-phenyl-4,5-dihydro-1H-1,2,4-triazole-5-thione or Phenyl[2-(3-phenyl-5-thioxo-1,4-dihydro-1,2,4-triazol-4-yl)ethylthio] acetic acid.

In a particular embodiment, l and o are 1, A is a phenyl optionally substituted by at least one radical selected from methyl, OMe, OH, halogen and CF₃, and Z is a phenyl optionally substituted by at least one radical selected from COOH and OH. Preferably, the compound of formula (I) is selected from the group consisting of: 4-benzyl-3-(2-hydroxy-5-methoxyphenyl)-4,5-dihydro-1H-1,2,4-triazole-5-thione; 4-benzyl-3-phenyl-4,5-dihydro-1H-1,2,4-triazole-5-thione; *m*-[(3-Phenyl-5-thioxo-1,4-dihydro-1,2,4-triazol-4-yl)methyl]benzoic acid; and *p*-[(3-Phenyl-5-thioxo-1,4-dihydro-1,2,4-triazol-4-yl)methyl]benzoic acid; 2-[2-(3-phenyl-5-sulfanylidene-4,5-dihydro-1H-1,2,4-triazol-4-yl)ethyl]benzoic acid; 2- {2-[3-(2-hydroxy-5-methoxyphenyl)-5-sulfanylidene-4,5-dihydro-1H-1,2,4-triazol-4-yl]ethyl}benzoic acid; 3-(2-hydroxy-5-methoxyphenyl)-4-(2-phenylethyl)-4,5-dihydro-1H-1,2,4-triazole-5-thione ; 3-phenyl-4-(2-phenylethyl)-4,5-dihydro-1H-1,2,4-triazole-5-thione; 2-{2-[3-(3-methoxyphenyl)-5-sulfanylidene-4,5-dihydro-1H-1,2,4-triazol-4-yl]ethyl}benzoic acid; 4-[2-(4-hydroxyphenyl)ethyl]-3-phenyl-4,5-dihydro-1H-1,2,4-triazole-5-thione; 4-[2-(2,4-Dihydroxyphenyl)ethyl]-5-phenyl-1,2,4-triazole-3-thione; 5-Hydroxy-2-[2-(3-phenyl-5-thioxo-1,4-dihydro-1,2,4-triazol-4-yl)ethyl]benzoic acid; 2-{ [(3-phenyl-5-sulfanylidene-4,5-dihydro-1H-1,2,4-triazol-4-yl)amino]methyl}benzoic acid; 3-phenyl-4-(3-phenylpropyl)-4,5-dihydro-1H-1,2,4-triazole-5-thione; 2-{2-[3-(2-methylphenyl)-5-sulfanylidene-4,5-dihydro-1H-1,2,4-triazol-4-yl]ethyl}benzoic acid; 3-phenyl-4-[(1R,2S)-2-phenylcyclopropyl]-4,5-dihydro-1H-1,2,4-triazole-5-thione; and 4-[2-(benzylsulfanyl)ethyl]-3-phenyl-4,5-dihydro-1H-1,2,4-triazole-5-thione.

In a more particular embodiment, l and o are 1, A is a phenyl optionally substituted by at least one radical selected from OMe and OH, Z is a phenyl optionally substituted by a COOH or at least one OH, and m is 1 and Y is an ethyl or a methyl. Preferably, the compound of formula (I) is selected from the group consisting of: 4-benzyl-3-(2-hydroxy-5-methoxyphenyl)-4,5-dihydro-1H-1,2,4-triazole-5-thione; 4-benzyl-3-phenyl-4,5-dihydro-1H-1,2,4-triazole-5-thione; *m*-[(3-Phenyl-5-thioxo-1,4-dihydro-1,2,4-triazol-4-yl)methyl]benzoic acid; and *p*-[(3-Phenyl-5-thioxo-1,4-dihydro-1,2,4-triazol-4-yl)methyl]benzoic acid; 2-[2-(3-phenyl-5-sulfanylidene-4,5-dihydro-1H-1,2,4-triazol-4-yl)ethyl]benzoic acid; 2- {2-[3-(2-hydroxy-5-methoxyphenyl)-5-sulfanylidene-4,5-dihydro-1H-1,2,4-triazol-4-yl]ethyl}benzoic acid; 3-(2-hydroxy-5-methoxyphenyl)-4-(2-phenylethyl)-4,5-dihydro-1H-1,2,4-triazole-5-thione ; 3-phenyl-4-(2-phenylethyl)-4,5-dihydro-1H-1,2,4-triazole-5-thione; 2-{2-[3-(3-methoxyphenyl)-5-sulfanylidene-4,5-dihydro-1H-1,2,4-triazol-4-yl]ethyl}benzoic acid; 4-[2-(4-hydroxyphenyl)ethyl]-3-phenyl-4,5-dihydro-1H-1,2,4-triazole-5-thione; 4-[2-(2,4-Dihydroxyphenyl)ethyl]-5-phenyl-1,2,4-triazole-3-thione; and 5-Hydroxy-2-[2-(3-phenyl-5-thioxo-1,4-dihydro-1,2,4-triazol-4-yl)ethyl]benzoic acid.

More preferably, the compound of formula (I) is 3-(2-hydroxy-5-methoxyphenyl)-4-(2-phenylethyl)-4,5-dihydro-1H-1,2,4-triazole-5-thione; 4-[2-(4-hydroxyphenyl)ethyl]-3-phenyl-4,5-dihydro-1H-1,2,4-triazole-5-thione or 4-benzyl-3-(2-hydroxy-5-methoxyphenyl)-4,5-dihydro-1H-1,2,4-triazole-5-thione.

In a particular embodiment, l and o are 1, A is a phenyl substituted by a phenyl, and Z is a COOH or a phenyl optionally substituted by at least one radical selected from COOH and OH. Preferably, the compound of formula (I) is selected from the group consisting of: 5-(3-Biphenyl)-4-[3-carboxypropanyl]-1,2,4-triazole-3-thione; 5-(3-Biphenyl)-4-[2-(2-carboxyphenyl)ethyl]-1,2,4-triazole-3-thione; 5-(3-Biphenyl)-4-[2-(2,4-dihydroxyphenyl)ethyl]-1,2,4-triazole-3-thione; 5-(2-Biphenyl)-4-[2-(2,4-dihydroxyphenyl)ethyl] -1,2,4-triazole-3-thione; and *o-*{ [3-(3-Biphenylyl)-5-thioxo-1,4-dihydro-1,2,4-triazol-4-ylamino]methyl} benzoic acid.

In a further particular embodiment, a compound of the invention is represented by formula (I), wherein:
- l and o are 1, A is a phenyl substituted by a phenyl, and Z is a COOH or a phenyl optionally substituted by at least one radical selected from COOH and OH; and
- m is 1 and Y is (C₁-C₇)alkyl chain with a heteroatomic group consisting of NH.

Preferably, the compound of formula (I) is *o*-{[3-(3-Biphenylyl)-5-thioxo-1,4-dihydro-1,2,4-triazol-4-ylamino]methyl} benzoic acid.

In a particular embodiment, the compound is represented by formula (I) wherein
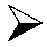 n and m are independently 0 or 1;
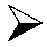 l is 1 or 2;
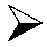 o is 0, 1 or 2;
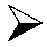 when l is 2, n is 1;
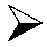 when o is 2, m is 1;
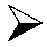 when m is 0, o is 1;
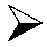 when n is 0,1 is 1;
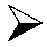 (X) represents a methyl or ethyl;
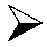 (A) represents a 5-14 membered ring selected in the group consisting of a phenyl, naphthalenyl, furanyl, thiophenyl, thiadiazolyl, quinolinyl, pyrrolyl, and adamantanyl, said 5-14 membered ring is optionally substituted by at least one radical selected in the group consisting of a chlorine, OH, a methoxy, a methyl, CF3, a morpholinylethoxy, a phenyl, a benzyloxy;
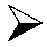 (Y) represents a methyl, ethyl, propyl, butyl, hexyl or heptyl chain, comprising optionally a heteroatomic group selected from NH, O, and S;
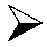 (Z) represents
   ∘ a 5-14 membered ring selected in the group consisting of a phenyl, a cyclohexyl, a cyclopropyl, a quinolinyl, a morpholinyl, a phtalidyl, said 5-14 membered ring being optionally substituted by at least one radical selected in the group consisting of: a chlorine, OH, COOH, NO₂, NH₂, a methyl, CF₃, CH₂-COOH, a benzyloxy, and a phenyl optionally substituted by NO₂;
   ∘ A radical selected in the group consisting of COOH, N₃, NHBoc, and N-phenylurea; or
   ∘ CF₃ or CH₂-COOH.

In a particular embodiment, the compound of the invention is selected from the group consisting of :
Compound 7. 4-benzyl-3-(2-hydroxy-5-methoxyphenyl)-4,5-dihydro-1H-1,2,4-triazole-5-thione;
Compound 10. 3-(2-hydroxy-5-methoxyphenyl)-4-(2-phenylethyl)-4,5-dihydro-1H-1,2,4-triazole-5-thione;
Compound 31. 4-[2-(4-hydroxyphenyl)ethyl]-3-phenyl-4,5-dihydro-1H-1,2,4-triazole-5-thione;
Compound 33. 4-[2-(benzylsulfanyl)ethyl]-3-phenyl-4,5-dihydro-1H-1,2,4-triazole-5-thione;
Compound 37. 5-Phenyl-4-[(3-phthalideyl)methyl]-2,4-dihydro-1,2,4-triazole-3-thione;
Compound 39. 5-(3-Biphenyl)-4-[2-(2-carboxyphenyl)ethyl]-1,2,4-triazole-3-thione; and
Compound 42. 5-(3-Biphenyl)-4-[2-(2,4-dihydroxyphenyl)ethyl]-1,2,4-triazole-3-thione.

A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulfuric, phosphoric, trifluoroacetic, formic, citric methane sulfonate or maleic acid. In addition, a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a pharmaceutically acceptable cation, for example a salt with methylamine, dimethylamine, trimethylamine, piperidine, morpholine or tris-(2-hydroxyethyl)amine.

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn-lngold-Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

The compounds of this invention may possess one or more asymmetric centers; such compounds can therefore be produced as individual (R)- or (S)-stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art (see discussion in Chapter 4 of "Advanced Organic Chemistry", 4th edition J. March, John Wiley and Sons, New York, 2001), for example by synthesis from optically active starting materials or by resolution of a racemic form. Some of the compounds of the invention may have geometric isomeric centres (E- and Z- isomers). It is to be understood that the present invention encompasses all optical, diastereoisomers and geometric isomers and mixtures thereof that possess antiproliferative activity.

It is also to be understood that certain compounds of the formula (I) may exist in solvated as well as unsolvated forms such as, for example, hydrated forms. It is to be understood that the invention encompasses all such solvated forms that possess antibacterial activity.

It is also to be understood that certain compounds of the formula (I) may exhibit polymorphism, and that the invention encompasses all such forms that possess antibacterial activity.

### Therapeutic Uses and Applications

The compounds of the present invention are inhibitors of metallo-beta-lactamases (MBLs). Many bacteria have developed resistance to β-lactam antibacterials (BLAs) and one of the main resistance mechanisms is the hydrolysis of BLAs by MBLs. Thus, the inhibition of bacterial MBLs by the compounds of the present invention can significantly enhance the activity of BLAs, when administered with a compound of the present invention.

The present invention provides compounds that function as inhibitors of metallo-beta-lactamases.

The present invention therefore provides a method of inhibiting bacterial metallo-beta-lactamase activity in vitro or in vivo, said method comprising contacting a cell with an effective amount of a compound, or a pharmaceutically acceptable salt, hydrate or solvate thereof, or a pharmaceutical composition as defined herein.

The present invention also provides a method for the prevention or treatment of bacterial infection in a patient in need of such treatment, said method comprising administering to said patient a therapeutically effective amount of a compound, or a pharmaceutically acceptable salt, hydrate or solvate thereof, or a pharmaceutical composition as defined herein, in combination with a suitable antibacterial agent.

In a preferred embodiment, the antibacterial agent is a β-lactam antibacterial agent, or analogue thereof. Non-limiting examples of suitable β-lactam antibacterial agents include carbapenems (e.g. meropenem, imipenem, ertapenem, doripenem, panipenem/betamipron and biapenem as well as razupenem, tebipenem, lenapenem and tomopenem), ureidopenicillins (e.g. piperacillin), penems (e.g., faropenem), carbacephems (e.g. loracarbef) and cephalosporins (e.g. cefpodoxime, ceftazidime, cefotaxime, ceftriaxone, ceftobiprole, ceftaroline,). Specific examples of suitable β-lactam antibacterial agents include, for example, temocillin, piperacillin, cefpodoxime, ceftazidime, cefotaxime, ceftriaxone, meropenem, faropenem, imipenem, loracarbef, etc.

The present invention also provides a method of inhibiting bacterial infection, in vitro or in vivo, said method comprising contacting a cell with an effective amount of a compound, or a pharmaceutically acceptable salt, hydrate or solvate thereof, as defined herein, in combination with a suitable antibacterial agent.

The present invention also provides a compound, or a pharmaceutically acceptable salt, hydrate or solvate thereof, or a pharmaceutical composition as defined herein for use in therapy.

The present invention also provides a compound, or a pharmaceutically acceptable salt, hydrate or solvate thereof, or a pharmaceutical composition as defined herein for use in the treatment of a bacterial infection. In one embodiment, the treatment may be prophylactic (i.e. intended to prevent disease).

The present invention provides a compound, or a pharmaceutically acceptable salt, hydrate or solvate thereof, as defined herein for use in the inhibition of metallo-beta-lactamase activity.

Furthermore, the present invention provides a compound, or a pharmaceutically acceptable salt, hydrate or solvate thereof, as defined herein for use in the treatment of a disease or disorder in which metallo-beta-lactamase activity is implicated.

The present invention also provides a kit of parts comprising a compound, or a pharmaceutically acceptable salt, hydrate or solvate thereof, or a pharmaceutical composition as defined herein, and a BLA and/or a BLA linked to a formula (I) compound.

The term "bacterial infection" will be understood to refer to the invasion of bodily tissue by any pathogenic bacteria that proliferate, resulting in tissue injury that can progress to disease. The bacterial infection may be caused by Gram-negative bacteria. For example, the bacterial infection may be caused by bacteria from one or more of the following genera; *Pseudomonas, Escherichia, Klebsiella, Enterobacter, Serratia, Acinetobacter, Stenotrophomonas, Burkholderia.*

It will be understood by a person skilled in the art that the patient in need thereof is suitably a human, but may also include, but is not limited to, primates (e.g. monkeys), commercially farmed animals (e.g. horses, cows, sheep or pigs) and domestic pets (e.g. dogs, cats, guinea pigs, rabbits, hamsters or gerbils). Thus the patient in need thereof may be any mammal that is capable of being infected by a bacterium.

### Regimen and administration

The compound according to the invention or the pharmaceutical composition according to the invention may be administered by any conventional route of administration. In particular, the compound or the pharmaceutical composition of the invention can be administered by a topical, enteral, oral, parenteral, intranasal, intravenous, intra-arterial, intramuscular, subcutaneous or intraocular administration and the like. In particular, the compound according to the invention or the pharmaceutical composition according to the invention can be formulated for a topical, enteral, oral, parenteral, intranasal, intravenous, intra-arterial, intramuscular, subcutaneous or intraocular administration and the like.

Preferably, the compound according to the invention or the pharmaceutical composition according to the invention is administered by enteral or parenteral route of administration. When administered parenterally, the compound according to the invention or the pharmaceutical composition according to the invention is preferably administered by intravenous route of administration. When administered enterally, the compound according to the invention or the pharmaceutical composition according to the invention is preferably administered by oral route of administration.

For oral administration, the composition can be formulated into conventional oral dosage forms such as tablets, capsules, powders, granules and liquid preparations such as syrups, elixirs, and concentrated drops. Nontoxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatin, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Pharmaceutical compositions according to the invention may be formulated to release the active drug substantially immediately upon administration or at any predetermined time or time period after administration.

The compound according to the invention or the pharmaceutical composition according to the invention may be administered as a single dose or in multiple doses.

Preferably, the treatment is administered regularly, preferably between every day and every month, more preferably between every day and every two weeks, more preferably between every day and every week, even more preferably the treatment is administered every day. In a particular embodiment, the treatment is administered several times a day, preferably 2 or 3 times a day, even more preferably 3 times a day.

The duration of treatment with the compound according to the invention or the pharmaceutical composition according to the invention is preferably comprised between 1 day and 20 weeks, more preferably between 5 days and 10 weeks, still more preferably between 5 days and 4 weeks, even more preferably between 5 days and 2 weeks. In a particular embodiment, the duration of the treatment is of at least 1 week. Alternatively, the treatment may last as long as the bacterial infection persists.

The amount of compound according to the invention or of pharmaceutical composition according to the invention to be administered has to be determined by standard procedure well known by those of ordinary skills in the art. Physiological data of the patient (e.g. age, size, and weight) and the routes of administration have to be taken into account to determine the appropriate dosage, so as a therapeutically effective amount will be administered to the patient.

The form of the pharmaceutical compositions, the route of administration and the dose of administration of the compound according to the invention, or the pharmaceutical composition according to the invention can be adjusted by the man skilled in the art according to the type and severity of the disease, and to the patient, in particular its age, weight, sex, and general physical condition.

### Biological Activity

The enzyme and *in vitro* cell-based assays described in accompanying Example section, or elsewhere in the literature, may be used to measure the pharmacological effects of the compounds of the present invention.

Although the pharmacological properties of the compounds of formula I vary with structural change, as expected, the compounds of the invention were found to be active in these enzyme assays.

### Diagnostic uses

The compounds of the present invention, or pharmaceutical compositions comprising these compounds in combination with a suitable antibacterial agent, may also be used in methods for the detection of metallo-beta-lactamases. It will be appreciated that the compounds of formula (I) may be modified to enable various types of assays known is the literature, such as those using spectroscopic such as fluorescence or luminescence-based methods. Thus, in one variation a sample containing bacteria, which is suspected of expressing MBLs, can be cultured (a) in the presence of a beta-lactam antibiotic agent; and (b) in the presence of the antibiotic combination of the invention. If the bacteria are seen to grow under conditions (a), this suggests that a beta-lactamase, able to hydrolyse the antibiotic agent, is causing resistance of the bacteria to the antibiotic agent. However, if the bacteria do not grow under condition (b), i.e. in the presence of compound of the present invention and a suitable antibacterial agent, then the beta-lactamases present have been inhibited. Such a result suggests that the beta-lactamases are metallo-beta-lactamases. The method can be used to determine whether bacteria express metallo-beta-lactamase enzymes.

Further aspects and advantages of the present invention will be described in the following examples, which should be regarded as illustrative and not limiting.

### EXAMPLES

### Example 1: General Synthesis

The synthesis of stable analogues (A pathway) where the hydrazone-like bond was replaced by a C-C bond is performed in two main steps according to Maingot et al. [Maingot L., Leroux F., Landry V., Dumont J., Nagase H., Villoutreix B., Sperandio O., Deprez-Poulain R. & Deprez B. (2010) Bioorg. Med. Chem. Letters 20, 6213-6]. The first one is the formation of a thiosemicarbazide precursor by reaction of an amine derivative with *O,O'*-di-2-pyridyl thionocarbonate (DPT) yielding a thiocarbamate, which then reacts with a hydrazide. The second step is the cyclization of the thiosemicarbazide in hot basic conditions (Scheme 1).

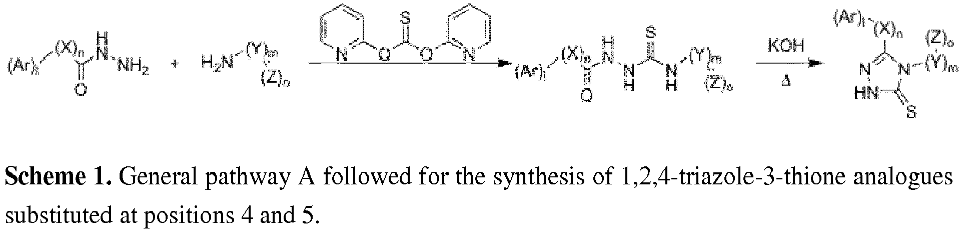

The hydrazide derivatives are formerly obtained by treatment of the corresponding ethyl or methyl carboxylates with hot hydrazine, hydrate.

The synthesis of stable analogues (B pathway) where the hydrazone-like bond was replaced by a reduced N-C bond is presented in Scheme 2. The 4-amino-1,2,4-triazole-3-thione precursors are first obtained following two well known routes depending on the substituent at position 5. These precursors are then condensed with various benzaldehydes to yield analogues with the hydrazone-like bond. Its reduction using NaBH₄ leads to stable compounds with a NH-CH₂ link (Scheme 2).

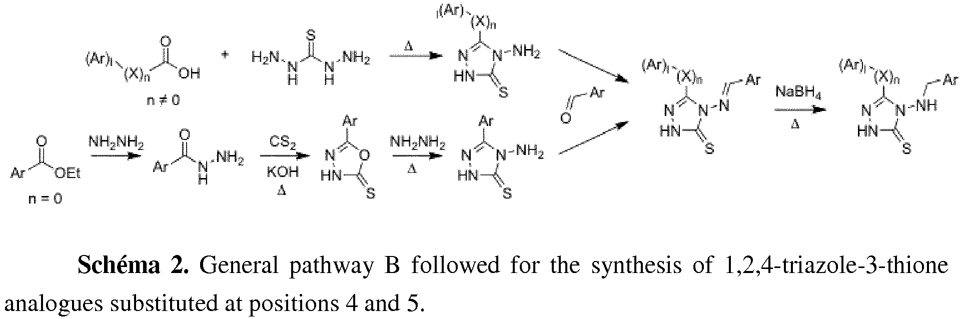

### Example 2: Experimental details of the chemical synthesis.

Synthetic pathway A (Scheme 1). The amine compound (1 mmol, 1 equiv.) (1.5 equiv. Na₂CO₃ is added if the amine is a hydrochloride salt) and DPT (1.05 equiv.) are solubilised in DMF (4 mL) and the mixture is stirred at 55 °C for 90 min. Then, the hydrazide compound (1.1 equiv.) is directly added and warming is continued for 90 min. After evaporation under vacuum, the obtained residue is directly engaged in the cyclization step without purification.

The residue is taken off in a mixture water/ethanol (40:60 - 10 mL) in the presence of KOH (3 equiv., 3 mmol) and the reaction is refluxed for 2h. The solution is neutralized using an aqueous 1M KHSO₄ solution and extracted twice with dichloromethane. After drying on MgSO₄, filtration and evaporation of the organic phase, the resulting residue is purified by chromatography on a silica gel column.

Synthetic pathway B (Scheme 2). Two routes for the synthesis of the 4-amino-1,2,4-triazole-3-thione derivatives.

Route 1 for alkanoic acids (n ≠ 0): one step (Beyer method). The carboxylic acid (1 equiv.) and thiocarbohydrazide (1 equiv.) are carefully ground together and the mixture is molten by warming at 160 °C for 90 min. After cooling to room temperature, an aqueous solution of Na₂CO₃ (20g/L, 10 mL/mmol) is added and the mixture is stirred under reflux for 1h. After cooling, the formed precipitate is filtered and washed with water. After drying the compound is recrystallized from EtOH.

Route 2 for aryloic acids (n = 0): three steps from the ethyl ester. The ester is solubilised in ethanol containing hydrazine, hydrate (5 equiv.) and the mixture is refluxed for 3h30. After cooling, the formed precipitate is filtered, washed with diethylether and dried to yield the hydrazide product. The latter is solubilised in ethanol and CS₂ (5 equiv.) and KOH (1.7 equiv.) are added. The mixture is refluxed for 5h. Water is added, the mixture is placed in an ice bath and neutralized with 4N HCl. The formed precipitate is filtered, washed with water and dried to give the 1,2,4-oxadiazole-3-thione intermediate. The latter is solubilised in ethanol. Hydrazine, hydrate (10 equiv.) is added and the mixture is refluxed overnight. After evaporation under vacuum, the residue is taken off in diethylether, filtered and recrystallized from ethanol to yield the 1,2,4-triazole-3-thione compound.

The 1,2,4-triazole-3-thione analogue is then solubilised in acetic acid (10 mL/mmol) in a sealed tube. The benzaldehyde derivative (2 to 5 equiv.) is added and the mixture is stirred at 110 °C (5h to overnight). After cooling, the formed precipitate is filtered, dried and recrystallized from ethanol to yield the analogue with a hydrazone-like bond.

Finally, the hydrazone-like compound (0.5 mmol, 1 equiv.) is solubilised in MeOH (5mL). NaBH₄ (10 equiv.) is added and the mixture is refluxed for 1h under inert atmosphere. After evaporation, the residue is purified on a silica gel column.

### Example 3: Compounds prepared according to the general chemical synthesis

### Compound 1: 2-[2-(3-phenyl-5-sulfanylidene-4,5-dihydro-1H-1,2,4-triazol-4-yl)ethyl]benzoic acid

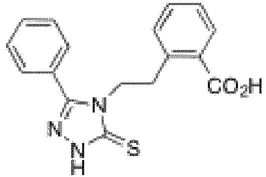

White powder, 82 mg, Yield: 52% LC-MS *t*_{R}: 1.27 min; *m*/*z* (ES+) 326.1 (M+H⁺); Mp = 220-221 °C; ¹H NMR (400 MHz, DMSO-*d*₆): 13.90 (bs, 1H), 12.72 (bs, 1H), 7.68 (dd, *J* = 7.6, 1.2 Hz, 1H), 7.48 (t, *J* = 7.4 Hz, 1H), 7.44-7.33 (m, 3H), 7.28 (t, *J* = 7.6 Hz, 1H), 7.21 (d, *J* = 7.6 Hz, 2H), 6.93 (d, *J* = 7.6 Hz, 2H), 4.36 (d, *J* = 6.6 Hz, 2H), 3.32 (d, *J* = 6.6 Hz, 2H); ¹³C NMR (100 MHz, DMSO-*d*₆): 167.7, 166.8, 151.5, 138.6, 131.8, 131.7, 130.7, 130.2 (2C), 128.5, 128.3, 126.8, 125.6, 44.4, 31.4; HRMS (ESI+) calcd for C₁₇H₁₆N₃O₂S (M+H)⁺ 326.0963, found 326.0965.

### Compound 2: 4-(3-phenyl-5-sulfanylidene-4,5-dihydro-1H-1,2,4-triazol-4-yl)butanoic acid

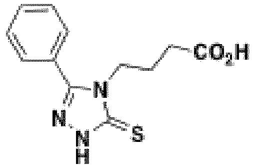

White powder, 186 mg, Yield: 61% LC-MS *t*_{R}: 0.95 min; *m*/*z* (ES+) 250.2 (M+H⁺); ¹H NMR (400 MHz, DMSO-*d*₆): 13.92 (bs, 1H), 12.11 (bs, 1H), 7.72-7.67 (m, 2H), 7.63-7.53 (m, 3H), 4.06 (t, *J* = 7.4 Hz, 2H), 2.14 (t, *J* = 7.4 Hz, 2H), 1.78 (quint, *J* = 7.4 Hz, 2H); ¹³C NMR (100 MHz, DMSO-*d*₆): 173.5, 167.1, 151.1, 130.6, 128.9, 128.5, 125.9, 43.1, 30.4, 23.0; HRMS (ESI+) calcd for C₁₂H₁₄N₃O₂S (M+H)⁺ 264.0807, found 264.0804.

### Compound 3: 2-{2-[3-(naphthalen-2-yl)-5-sulfanylidene-4,5-dihydro-1H-1,2,4-triazol-4-yl]ethyl}benzoic acid

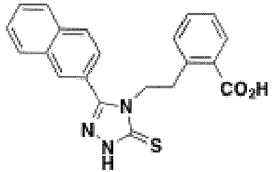

Light brown powder, 181 mg, Yield: 48% LC-MS *t*_{R}: 1.57 min; *m*/*z* (ES+) 376.1 (M+H⁺); Mp = 240-241 °C; ¹H NMR (400 MHz, DMSO-*d*₆): 13.98 (bs, 1H), 12.40 (bs, 1H), 7.99-7.90 (m, 3H), 7.75 (s, 1H), 7.64-7.56 (m, 3H), 7.42-7.35 (m, 2H), 7.25 (t, *J* = 7.6 Hz, 1H), 6.99 (d, *J* = 7.6 Hz, 1H), 4.46 (t, *J* = 6.6 Hz, 2H), 3.38 (t, *J* = 7.6 Hz, 1H); ¹³C NMR (100 MHz, DMSO-*d*₆): 167.7, 167.0, 151.3, 138.7, 133.1, 132.0, 131.8, 131.2, 130.6, 130.3, 128.4, 128.3, 128.2, 127.5, 127.4, 126.8, 126.7, 124.9, 122.9, 44.7, 31.3; HRMS (ESI+) calcd for C₂₁H₁₈N₃O₂S (M+H)⁺ 376.1120, found 376.1123.

### Compound 4: 2-{2-[3-(2-hydroxy-5-methoxyphenyl)-5-sulfanylidene-4,5-dihydro-1H-1,2,4-triazol-4-yl]ethyl}benzoic acid

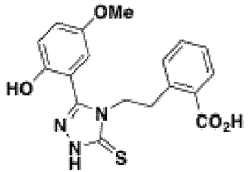

Yellow powder, 160 mg, Yield: 43%; LC-MS *t*_{R}: 1.28 min; *m*/*z* (ES+) 372.1 (M+H⁺); Mp = 88-89 °C; ¹H NMR (400 MHz, DMSO-*d*₆): 13.72 (bs, 1H), 12.33 (bs, 1H), 9.72 (bs, 1H), 7.67 (dd, *J* = 7.6, 1.6 Hz, 1H), 7.36 (td, *J* = 7.4, 1.6 Hz, 1H), 7.26 (td, *J* = 7.6, 1.6 Hz, 1H), 6.90-6.85 (m, 1H), 6.81-6.76 (m, 2H), 5.90 (d, *J* = 3.2 Hz, 1H), 4.25 (t, *J* = 6.4 Hz, 2H), 3.60 (s, 3H), 3.27 (t, *J* = 6.4 Hz, 2H); ¹³C NMR (100 MHz, DMSO-*d*₆): 167.5, 166.2, 151.5, 150.2, 148.7, 139.0, 131.6, 131.1, 130.7, 130.1, 126.6, 118.2, 116.4, 114.7, 55.2, 44.5, 31.3; HRMS (ESI+) calcd for C₁₈H₁₈N₃O₄S (M+H)⁺ 372.1018, found 372.1021.

### Compound 5: 2-{[(3-phenyl-5-sulfanylidene-4,5-dihydro-1H-1,2,4-triazol-4-yl)amino]methyl}benzoic acid

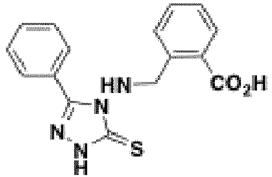

White powder, 68 mg, Yield: 69%; LC-MS *t*_{R}: 1.45 min; *m*/*z* (ES+) 327.1 (M+H⁺); Mp = 161-162 °C; ¹H NMR (400 MHz, DMSO-*d*₆): 13.92 (bs, 1H), 12.94 (bs, 1H), 7.88-7.84 (m, 2H), 7.73 (dd, *J* = 8, 1.2 Hz, 1H), 7.51-7.36 (m, 4H), 7.30 (td, *J* = 7.6, 1.2 Hz, 1H), 7.19 (d, *J* = 7.6 Hz, 1H), 6.77 (t, *J* = 4 Hz, 1H), 4.55 (d, J= 4 Hz, 2H); ¹³C NMR (100 MHz, DMSO-*d*₆): 168.1, 166.2, 148.8, 136.9, 131.5, 130.7, 130.4, 130.3 (2C), 128.3, 127.5, 127.5, 125.2, 50.8; HRMS (ESI+) calcd for C₁₆H₁₅N₄O₂S (M+H)⁺ 327.0916, found 327.0919.

### Compound 6: 3-(2-methylphenyl)-4-(2-phenylethyl)-4,5-dihydro-1H-1,2,4-triazole-5-thione

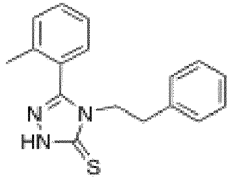

Light yellow powder, 247 mg, Yield: 47%; LC-MS *t*_{R}: 1.68 min; *m*/*z* (ES+) 296.2 (M+H⁺); Mp = 156-157 °C; ¹H NMR (400 MHz, DMSO-*d*₆): 13.95 (bs, 1H), 7.47 (t, *J* = 7.4 Hz, 1H), 7.36 (d, *J* = 7.6 Hz, 1H), 7.27 (t, *J* = 7.4 Hz, 1H), 7.21-7.17 (m, 3H), 7.05 (d, *J* = 7.6 Hz, 1H), 6.90-6.83 (m, 2H), 3.95 (t, *J* = 7.6 Hz, 1H), 2.90 (t, *J* = 7.6 Hz, 1H), 2.02 (s, 3H); ¹³C NMR (100 MHz, DMSO-*d*₆): 166.2, 150.5, 137.6, 137.2, 130.6, 130.3, 129.9, 128.4 (2C), 126.5, 125.8, 125.1, 44.8, 32.6, 18.9; HRMS (ESI+) calcd for C₁₇H₁₈N₃S (M+H)⁺ 296.1221, found 296.1220.

### Compound 7 : 4-benzyl-3-(2-hydroxy-5-methoxyphenyl)-4,5-dihydro-1H-1,2,4-triazole-5-thione

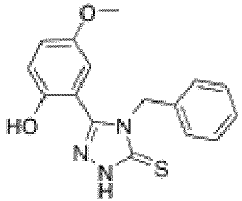

Off-white powder, 134 mg, Yield: 43%; LC-MS *t*_{R}: 1.47 min; *m*/*z* (ES+) 314.1 (M+H⁺); Mp = 211-212 °C; ¹H NMR (400 MHz, DMSO-*d*₆): 13.93 (bs, 1H), 12.96 (bs, 1H), 7.90 - 7.82 (m, 2H), 7.73 (dd, *J* = 7.8, 1.4 Hz, 1H), 7.53 - 7.34 (m, 4H), 7.31 (d, *J* = 7.6 Hz, 1H), 7.18 (d, *J* = 7.5 Hz, 1H), 6.77 (t, *J* = 5.8 Hz, 1H), 4.54 (d, *J* = 4.5 Hz, 2H); ¹³C NMR (100 MHz, DMSO-*d*₆): 168.17, 166.34, 148.93, 137.05, 131.60, 130.85, 130.50, 130.39, 128.44, 127.64, 127.58, 125.33, 50.92, 28.99; HRMS (ESI+) calcd for C₁₆H₁₆N₃O₂S (M+H)⁺ 314.0963, found 314.0967.

### Compound 8: 3-phenyl-4-(3-phenylpropyl)-4,5-dihydro-1H-1,2,4-triazole-5-thione

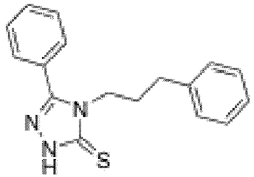

Off-white powder, 207 mg, Yield: 67%; LC-MS *t*_{R}: 1.67 min; *m*/*z* (ES+) 296.2 (M+H⁺); Mp = 160-161 °C; n¹H NMR (400 MHz, DMSO-*d*₆): 13.94 (bs, 1H), 7.62-7.45 (m, 5H), 7.25-7.03 (m, 5H), 4.02 (t, *J* = 7.8 Hz, 2H), 2.48 (t, *J* = 7.6 Hz, 2H), 1.88 (quint, J = 7.6 Hz, 2H); ¹³C NMR (100 MHz, DMSO-*d*₆): 167.0, 151.0, 140.3, 130.6, 128.9, 128.4, 128.2, 127.9, 126.0, 125.8, 43.3, 31.7, 28.9; HRMS (ESI+) calcd for C₁₇H₁₈N₃S (M+H)⁺ 296.1221, found 296.1222.

### Compound 9: 4-benzyl-3-phenyl-4,5-dihydro-1H-1,2,4-triazole-5-thione

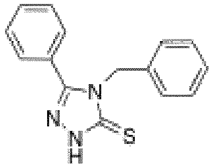

White powder, 159mg, Yield: 62%; LC-MS *t*_{R}: 1.51 min; *m*/*z* (ES+) 268.2 (M+H⁺); Mp = 186-187 °C; ¹H NMR (400 MHz, DMSO-*d*₆): 14.12 (bs, 1H), 7.52-7.46 (m, 5H), 7.28-7.19 (m, 3H), 7.04-6.99 (m, 2H), 5.35 (s, 2H); ¹³C NMR (100 MHz, DMSO-*d*₆): 167.9, 151.3, 135.6, 103.6, 128.8, 128.4, 128.2, 127.4, 126.4, 125.9, 46.6; HRMS (ESI+) calcd for C₁₅H₁₄N₃S (M+H)⁺ 268.0908, found 268.0910.

### Compound 10: 3-(2-hydroxy-5-methoxyphenyl)-4-(2-phenylethyl)-4,5-dihydro-1H-1,2,4-triazole-5-thione

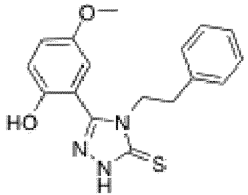

White powder, 282 mg, Yield: 43%; LC-MS *t*_{R}: 1.51 min; *m*/*z* (ES+) 328.1 (M+H⁺); Mp = 132-133 °C; ¹H NMR (400 MHz, DMSO-*d*₆): 13.85 (bs, 1H), 9.84 (s, 1H), 7.23 - 7.11 (m, 3H), 6.98 (dd, *J* = 8.9, 3.1 Hz, 1H), 6.94-6.83 (m, 3H), 6.39 (d, *J* = 3.0 Hz, 1H), 4.04 (t, *J* = 8.0 Hz, 2H), 3.65 (s, 3H), 2.91 (t, *J* = 8.0 Hz, 2H); ¹³C NMR (100 MHz, DMSO-*d*₆): 166.2, 151.8, 150.0, 149.1, 137.6, 128.5, 128.4, 126.5, 118.6, 116.7, 115.2, 113.2, 55.4, 45.4, 32.8; HRMS (ESI+) calcd for C₁₇H₁₈N₃O₂S (M+H)⁺ 328.1120, found 328.1122.

### Compound 11: 3-phenyl-4-(2-phenylethyl)-4,5-dihydro-1H-1,2,4-triazole-5-thione

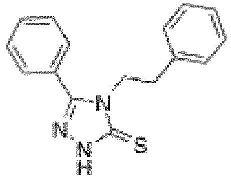

White powder, 323 mg, Yield: 61%; LC-MS *t*_{R}: 1.59 min; *m*/*z* (ES+) 282.3 (M+H⁺); Mp = 204-205 °C; ¹H NMR (400 MHz, DMSO-*d*₆): 13.95 (bs, 1H), 7.59-7.54 (m, 1H), 7.53-7.47 (m, 2H), 7.44-7.40 (m, 2H), 7.22-7.16 (m, 2H), 4.20 (t, *J* = 7.6 Hz, 2H), 2.93 (t, *J* = 7.6 Hz, 2H); ¹³C NMR (100 MHz, DMSO-*d*₆): 166.9, 151.5, 137.2, 130.5, 128.8, 128.6, 128.5 (2C), 126.6, 125.9, 45.1, 32.9; HRMS (ESI+) calcd for C₁₆H₁₆N₃S (M+H)⁺ 282.1065, found 282.1065.

### Compound 12: 2-{2-[3-(furan-2-yl)-5-sulfanylidene-4,5-dihydro-1H-1,2,4-triazol-4-yl]ethyl}benzoic acid

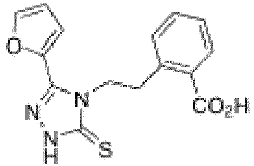

Light yellow powder, 54 mg, Yield: 17%; LC-MS *t*_{R}: 1.34 min; *m*/*z* (ES+) 316.1 (M+H⁺); Mp = 210-211 °C; ¹H NMR (400 MHz, DMSO-*d*₆): 13.99 (bs, 1H), 12.96 (bs, 1H), 7.80 (d, *J* = 1.6 Hz, 1H), 7.77 (dd, *J* = 8, 1.2 Hz, 1H), 7.41 (td, *J* = 7.4, 1.2 Hz, 1H), 7.28 (td, *J* = 7.4, 1.2 Hz, 1H), 7.09 (d, *J* = 7.6 Hz, 1H), 6.94 (d, *J* = 3.6 Hz, 1H), 6.63-6.60 (m, 1H), 4.49 (t, *J* = 7 Hz, 2H), 3.36 (d, *J* = 7 Hz, 2H); ¹³C NMR (100 MHz, DMSO-*d*₆): 168.2, 167.0, 145.1; 139.6, 138.5, 131.8, 131.1, 130.4, 130.3, 126.8, 112.3, 111.6, 44.9, 31.7; HRMS (ESI+) calcd for C₁₈H₁₈N₃O₂S (M+H)⁺ 340.1120, found 340.1119.

### Compound 13: 2-{2-[3-(thiophen-2-yl)-5-sulfanylidene-4,5-dihydro-1H-1,2,4-triazol-4-yl]ethyl}benzoic acid

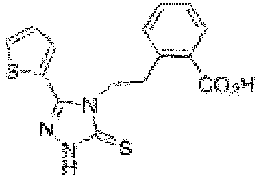

Off-white powder, 101 mg, Yield: 30% ; LC-MS *t*R: 1.37 min; *m*/*z* (ES+) 332.2 (M+H+); Mp = 195-196 °C; ¹H NMR (400 MHz, DMSO-*d*6): 13.97 (bs, 1H), 12.94 (bs, 1H), 7.77-7.73 (m, 2H), 7.53-7.50 (m, 1H), 7.46-7.40 (m, 1H), 7.15-7.09 (m, 2H), 4.48 (t, *J* = 7 Hz, 2H), 3.37 (t, *J* = 7 Hz, 2H); ¹³C NMR (100 MHz, DMSO-*d*6): 168.1, 167.3, 146.1, 138.6, 131.9, 131.1, 130.5, 130.4, 129.5, 128.8, 127.9, 126.8, 126.0, 44.8, 31.5; HRMS (ESI+) calcd for C15H14N3O2S (M+H)⁺ 332.0527, found 332.0526.

### Compound 14: 2-{2-[3-(2-methylphenyl)-5-sulfanylidene-4,5-dihydro-1H-1,2,4-triazol-4-yl]ethyl}benzoic acid

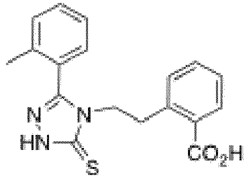

White powder, 193 mg, Yield: 57%; LC-MS *t*_{R}: 1.45 min; *m*/*z* (ES+) 340.1 (M+H⁺); Mp = 180-181 °C; ¹H NMR (400 MHz, DMSO-*d*₆): 13.90 (bs, 1H), 12.65 (bs, 1H), 7.75 (dd, *J* = 7.6, 1.2 Hz, 1H), 7.45-7.36 (m, 2H), 7.33 (td, *J* = 4.4, 1.2 Hz, 1H), 7.28 (d, *J* = 7.6 Hz, 1H), 7.13 (t, *J* = 7.2 Hz, 1H), 7.95 (d, *J* = 7.6 Hz, 1H), 6.64 (d, *J* = 7.6 Hz, 1H), 4.12 (t, *J* = 6.4 Hz, 2H), 3.32 (t, *J* = 6.4 Hz, 2H); ¹³C NMR (100 MHz, DMSO-*d*₆): 167.5, 166.3, 150.8, 138.8, 137.3, 131.8, 131.2, 130.8, 130.4, 130.3, 130.1, 129.7, 126.8, 125.6, 124.8, 44.2, 30.9, 19.9; HRMS (ESI+) calcd for C₁₅H₁₄N₃O₃S (M+H)⁺ 316.0756, found 316.0759.

### Compound 15: 5-(3-phenyl-5-sulfanylidene-4,5-dihydro-1H-1,2,4-triazol-4-yl)pentanoic acid

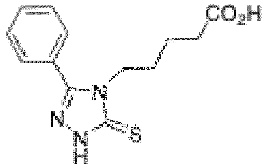

Off-white powder, 107 mg, Yield: 37%; LC-MS *t*_{R}: 1.20 min; *m*/*z* (ES+) 278.2 (M+H⁺); Mp = 176-177 °C; ¹H NMR (400 MHz, DMSO-*d*₆): 13.93 (bs, 1H), 11.98 (bs, 1H), 7.69-7.65 (m, 2H), 7.62-7.53 (m, 3H), 4.04 (t, *J* = 7.4 Hz, 2H), 2.10 (t, *J* = 7.4 Hz, 2H), 1.53 (quint, *J* = 7.4 Hz, 2H), 1.33 (quint, *J* = 7.4 Hz, 2H); ¹³C NMR (100 MHz, DMSO-*d*₆): 173.9, 167.0, 151.0, 130.7, 128.9, 128.4, 126.0, 43.2, 32.7, 26.9, 21.1; HRMS (ESI+) calcd for C₁₃H₁₆N₃O₂S (M+H)⁺ 278.0963, found 278.0965.

### Compound 16: 2-({[3-(naphthalen-2-yl)-5-sulfanylidene-4,5-dihydro-1H-1,2,4-triazol-4-yl] amino }methyl)benzoic acid

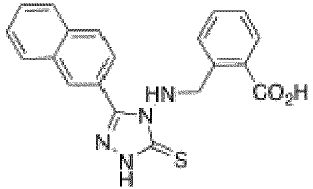

White powder, 34 mg, Yield: 45%; LC-MS *t*_{R}: 1.65 min; *m*/*z* (ES+) 377.2 (M+H⁺); Mp = 213-214 °C; ¹H NMR (400 MHz, DMSO-*d*₆): 14.01 (bs, 1H), 12.98 (bs, 1H), 8.60 (s, 1H), 7.94 (bs, 3H), 7.89 (d, *J* = 8.8 Hz, 1H), 7.70 (d, *J* = 8.8 Hz, 1H), 7.63-7.54 (m, 2H), 7.39-7.34 (m, 1H), 7.29-7.23 (m, 2H), 6.90-6.84 (m, 1H), 4.63 (d, *J* = 5.2 Hz, 2H); ¹³C NMR (100 MHz, DMSO-*d*₆): 168.1, 166.5, 148.6, 137.1, 133.2, 132.0, 131.5, 130.8, 130.6, 130.3, 128.6, 127.9, 127.6, 127.5, 127.4, 127.3, 126.6, 124.1, 122.6, 50.9; HRMS (ESI+) calcd for C₂₀H₁₇N₄O₂S (M+H)⁺ 377.1072, found 377.1070.

### Compound 17: 2-{1-[(3-phenyl-5-sulfanylidene-4,5-dihydro-1H-1,2,4-triazol-4-yl)methyl]cyclohexyl}acetic acid

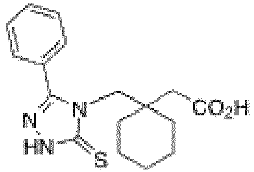

Light yellow powder, 113 mg, Yield: 34%; LC-MS *t*_{R}: 1.52 min; *m*/*z* (ES+) 332.3 (M+H⁺); Mp = 80-81 °C; ¹H NMR (400 MHz, DMSO-*d*₆): 13.91 (bs, 1H), 12.08 (bs, 1H), 7.69-7.47 (m, 5H), 4.38 (s, 2H), 2.23 (s, 2H), 1.39-1.10 (m, 8H), 1.01-0.85 (m, 2H); ¹³C NMR (100 MHz, DMSO-*d*₆): 172.4, 168.8, 151.8, 130.6, 129.1, 128.7, 50.6, 38.7, 38.5, 33.1, 24.9, 20.8; HRMS (ESI+) calcd for C₁₇H₂₂N₃O₂S (M+H)⁺ 332.1433, found 332.1433.

### Compound 18: 4-[3-(2-hydroxy-5-methoxyphenyl)-5-sulfanylidene-4,5-dihydro-1H-1,2,4-triazol-4-yl]butanoic acid

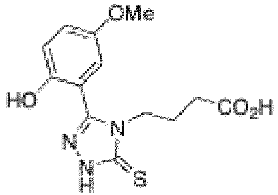

Light yellow powder, 99 mg, Yield: 32%; LC-MS *t*_{R}: 1.03 min; *m*/*z* (ES+) 310.1 (M+H⁺); Mp = 155-156 °C; ¹H NMR (400 MHz, DMSO-*d*₆): 13.81 (bs, 1H), 11.95 (bs, 1H), 9.80 (s, 1H), 7.04-6.98 (m, 1H), 6.94-6.89 (m, 2H), 3.89 (t, *J* = 7.2 Hz, 2H), 2.07 (t, *J* = 7.2 Hz, 2H), 1.73 (quint, *J* = 7.2 Hz, 2H); ¹³C NMR (100 MHz, DMSO-*d*₆): 173.4, 166.4, 151.9, 149.6, 149.4, 118.5, 116.8, 115.6, 113.2, 55.5, 43.1, 30.5, 23.0; HRMS (ESI+) calcd for C₁₃H₁₆N₃O₄S (M+H)⁺ 310.0862, found 310.0864.

### Compound 19: 4-[3-(naphthalen-2-yl)-5-sulfanylidene-4,5-dihydro-1H-1,2,4-triazol-4-yl]butanoic acid

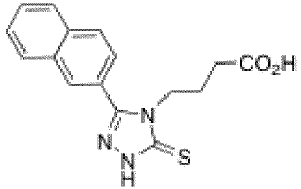

Yellow powder, 101 mg, Yield: 32%; LC-MS *t*_{R}: 1.39 min; *m*/*z* (ES+) 314.2 (M+H⁺); Mp = 220-221 °C; ¹H NMR (400 MHz, DMSO-*d*₆): 14.01 (bs, 1H), 12.12 (bs, 1H), 8.36 (d, *J* = 1.6 Hz, 1H), 8.12-7.99 (m, 3H), 7.80 (dd, *J* = 8.4, 1.6 Hz, 1H), 7.68-7.60 (m, 2H), 4.18 (t, *J* = 7.2 Hz, 2H), 2.19 (t, *J* = 7.2 Hz, 2H), 1.85 (quint, J = 7.2 Hz, 2H); ¹³C NMR (100 MHz, DMSO-*d*₆): 173.5, 167.3, 151.0, 133.4, 132.3, 128.7, 128.6, 128.3, 127.7, 127.6, 126.9, 125.1, 123.2, 43.4, 30.3, 23.0; HRMS (ESI+) calcd for C₁₆H₁₆N₃O₂S (M+H)⁺ 314.0963, found 314.0965.

### Compound 20: 3-phenyl-4-[(1R,2S)-2-phenylcyclopropyl]-4,5-dihydro-1H-1,2,4-triazole-5-thione

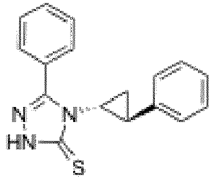

Yellow powder, 132 mg, Yield: 45%; LC-MS *t*_{R}: 1.72 min; *m*/*z* (ES+) 294.2 (M+H⁺); Mp = 185-186 °C; ¹H NMR (400 MHz, DMSO-*d*₆): 13.82 (bs, 1H), 7.75-7.71 (m, 2H), 7.57-7.51 (m, 1H), 7.47-7.42 (m, 2H), 7.29-7.13 (m, 3H), 7.04-7.00 (m, 2H), 3.53-3.48 (m, 1H), 2.26-2.19 (m, 1H), 1.50-1.43 (m, 1H), 1.21-1.14 (m, 1H); ¹³C NMR (100 MHz, DMSO-*d*₆): 168.6, 151.0, 139.3, 130.1, 128.6, 128.4, 128.0, 127.8, 127.8, 126.3, 125.9, 34.9, 26.0, 17.3; HRMS (ESI+) calcd for C₁₇H₁₆N₃S (M+H)⁺ 294.1065, found 294.1064.

### Compound 21: 3-(4-chlorophenyl)-4-(3-phenyl-5-sulfanylidene-4,5-dihydro-1H-1,2,4-triazol-4-yl)butanoic acid

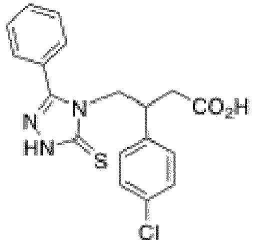

White powder, 230 mg, Yield: 77%; LC-MS *t*_{R}: 1.53 min; *m*/*z* (ES+) 374.0 (M+H⁺); Mp = 153-154 °C; ¹H NMR (400 MHz, DMSO-*d*₆): 13.89 (bs, 1H), 12.09 (bs, 1H), 7.55 (tt, *J* = 7.4, 1.2 Hz, 1H), 7.46 (t, *J* = 7.6 Hz, 2H), 7.32 (d, *J* = 7.2 Hz, 2H), 7.10 (d, *J* = 8.8 Hz, 2H), 6.81 (d, *J* = 8.8 Hz, 2H), 4.39 (dd, *J* = 14.4, 9.2 Hz, 1H), 4.22 39 (dd, *J* = 14.0, 6.4 Hz, 1H), 3.56-3.45 (m, 1H), 2.61-2.43 (m, 2H); ¹³C NMR (100 MHz, DMSO-*d*₆): 171.9,167.1,151.1,138.7,131.4, 130.3, 129.2, 128.5, 128.3, 128.3, 128.0, 125.8, 48.3, 38.8, 36.8; HRMS (ESI+) calcd for C₁₈H₁₇N₃O₂SCl (M+H)⁺ 374.0730, found 374.0732.

### Compound 22: 2-{2-[3-(3-methoxyphenyl)-5-sulfanylidene-4,5-dihydro-1H-1,2,4-triazol-4-yl]ethyl}benzoic acid

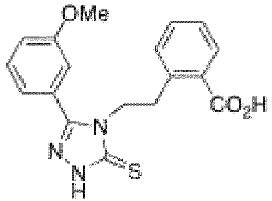

White powder, 91 mg, Yield: 26%; LC-MS *t*_{R}: 1.40 min; *m*/*z* (ES+) 356.1 (M+H⁺); Mp = 197-198 °C; ¹H NMR (400 MHz, DMSO-*d*₆): 13.88 (bs, 1H), 12.71 (bs, 1H), 7.66 (dd, *J* = 8.0, 1.6 Hz, 1H), 7.37 (td, *J* = 7.6, 1.6 Hz, 1H), 7.30-7.22 (m, 2H), 7.05-6.99 (m, 1H), 6.87 (d, *J* = 7.6 Hz, 1H), 6.74 (d, *J* = 7.6 Hz, 1H), 6.71-6.68 (m, 1H), 4.41 (t, *J* = 6.4 Hz, 2H), 3.74 (s, 3H), 3.29 (t, *J* = 6.4 Hz, 2H); ¹³C NMR (100 MHz, DMSO-*d*₆): 167.6, 166.8, 159.9, 151.4, 138.6, 131.7, 131.1, 130.7, 130.1, 126.7 (2C), 120.5, 116.3, 113.5, 55.1, 44.2, 31.4; HRMS (ESI+) calcd for C₁₈H₁₈N₃O₃S (M+H)⁺ 356.1069, found 356.1071.

### Compound 23: 2-{2-[3-(4-methyl-1,2,3-thiadiazol-5-yl)-5-sulfanylidene-4,5-dihydro-1H-1,2,4-triazol-4-yl]ethyl}benzoic acid

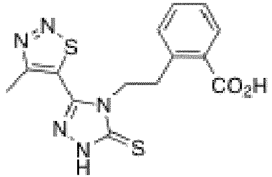

Yellow powder, 134 mg, Yield: 39%; LC-MS *t*_{R}: 1.28 min; *m*/*z* (ES+) 348.0 (M+H⁺); Mp = 195-196 °C; ¹H NMR (400 MHz, DMSO-*d*₆): 14.35 (bs, 1H), 12.88 (bs, 1H), 7.64 (dd, *J* = 8.0, 1.2 Hz, 1H), 7.37 (td, *J* = 7.6, 1.6 Hz, 1H), 7.25 7.37 (td, *J* = 7.6, 1.2 Hz, 1H), 6.94 (dd, *J* = 8.0, 1.6 Hz, 1H), 4.32 7.37 (t, *J* = 6.4 Hz, 2H), 3.28 (t, *J* = 6.4 Hz, 2H), 2.43 (s, 3H); ¹³C NMR (100 MHz, DMSO-*d*₆): 167.8, 167.5, 159.4, 142.2, 138.1, 132.9, 131.9, 131.4, 130.5, 130.1, 127.0, 445.1, 31.3, 13.1; HRMS (ESI+) calcd for C₁₄H₁₄N₅O₂S₂ (M+H)⁺ 348.0589, found 348.0592.

### Compound 24: 4-[3-(3-methoxyphenyl)-5-sulfanylidene-4,5-dihydro-1H-1,2,4-triazol-4-yl]butanoic acid

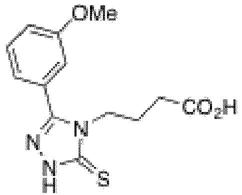

White powder, 249 mg, Yield: 85%; LC-MS *t*_{R}: 1.17 min; *m*/*z* (ES+) 294.1 (M+H⁺); Mp = 159-160 °C; ¹H NMR (400 MHz, DMSO-*d*₆): 13.91 (bs, 1H), 12.09 (bs, 1H), 7.47 (t, *J* = 8.0 Hz, 1H), 7.29-7.12 (m, 3H), 4.07 (t, *J* = 7.3 Hz, 2H), 3.81 (s, 3H); 2.15 (t, *J* = 7.3 Hz, 2H), 1.77 (quint, *J* = 7.3 Hz, 2H); ¹³C NMR (100 MHz, DMSO-*d*₆): 173.4, 167.1, 159.3, 150.9, 130.1, 127.2, 120.7, 116.6, 113.8, 55.3, 43.2, 30.4, 23.1; HRMS (ESI+) calcd for C₁₃H₁₆N₃O₃S (M+H)⁺ 294.0912, found 294.0912.

### Compound 25: 2-{[(tert-butoxy)carbonyl]amino}-5-(3-phenyl-5-sulfanylidene-4,5-dihydro-1H-1,2,4-triazol-4-yl)pentanoic acid

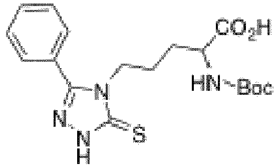

White powder, 312 mg, Yield: 53%; LC-MS *t*_{R}: 1.41 min; *m*/*z* (ES+) 393.2 (M+H⁺); Mp = 105-106 °C; ¹H NMR (400 MHz, DMSO-*d*₆): 13.92 (bs, 1H), 12.40 (bs, 1H), 7.70-7.51 (m, 5H), 6.99 (d, *J* = 8.0 Hz, 1H), 4.03 (t, *J* = 7.2 Hz, 2H), 3.77-3.68 (m, 1H), 1.64-1.35 (m, 4H), 1.34 (s, 9H); ¹³C NMR (100 MHz, DMSO-*d*₆): 173., 167.1, 155.4, 151.0, 130.6, 128.9, 128.5, 126.0, 77.9, 52.9, 43.3, 28.1, 27.7, 24.6; HRMS (ESI+) calcd for C₁₈H₂₅N₄O₄S (M+H)⁺ 393.1597, found 393.1595.

### Compound 26: 4-hexyl-3-phenyl-4,5-dihydro-1H-1,2,4-triazole-5-thione

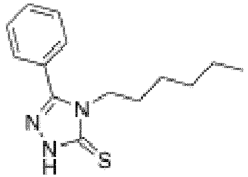

White powder, 138 mg, Yield: 53%; LC-MS *t*_{R}: 1.84 min; *m*/*z* (ES+) 262.0 (M+H⁺); Mp = 98-99 °C; ¹H NMR (400 MHz, DMSO-*d*₆): 13.92 (bs, 1H), 7.70-7.53 (m, 5H), 4.02 (t, *J* = 7.6 Hz, 2H), 1.50 (quint, *J* = 7.6 Hz, 2H), 1.32-1.05 (m, 6H), 0.76 (t, *J* = 7.0 Hz, 3H); ¹³C NMR (100 MHz, DMSO-*d*₆): 166.9, 151.1, 130.6, 128.9, 128.5, 126.2, 43.4, 30.2, 27.2, 25.2, 21.6, 13.6; HRMS (ESI+) calcd for C₁₄H₂₀N₃S (M+H)⁺ 262.1378, found 262.1379.

### Compound 27: 2-{2-[3-(5-chlorothiophen-2-yl)-5-sulfanylidene-4,5-dihydro-1H-1,2,4-triazol-4-yl]ethyl}benzoic acid

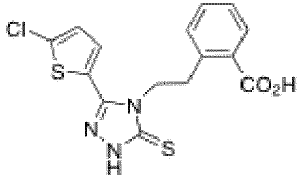

White powder, 177 mg, Yield: 48%; LC-MS *t*_{R}: 1.54 min; *m*/*z* (ES+) 366.0 (M+H⁺); Mp = 240-241 °C; ¹H NMR (400 MHz, DMSO-*d*₆): 14.03 (bs, 1H), 12.97 (bs, 1H), 7.77 (dd, *J* = 8.0, 1.2 Hz, 1H), 7.42 (td, *J* = 7.4, 1.2 Hz, 1H), 7.35 (d, *J* = 4.0 Hz, 1H), 7.30 (td, *J* = 7.6, 1.2 Hz, 1H), 7.13 (d, *J* = 4.0 Hz, 1H), 7.06 (d, *J* = 7.6 Hz, 1H), 4.46 (t, *J* = 6.8 Hz, 2H), 3.53 (t, *J* = 6.8 Hz, 2H); ¹³C NMR (100 MHz, DMSO-*d*₆): 168.2, 167.4, 145.0, 138.5, 131.9, 131.4, 131.3, 130.6, 130.4, 128.8, 127.6, 126.9, 125.0, 44.7, 31.5; HRMS (ESI+) calcd for C₁₅H₁₃N₃O₂S₂Cl (M+H)⁺ 366.0138, found 366.0139.

### Compound 28: 3-phenyl-4-(2-{[2-(trifluoromethyl)quinolin-4-yl]sulfanyl}ethyl)-4,5-dihydro-1H-1,2,4-triazole-5-thione

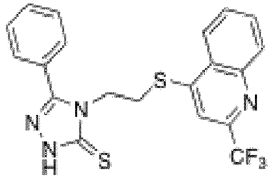

White powder, 166 mg, Yield: 38%; LC-MS *t*_{R}: 1.86 min; *m*/*z* (ES+) 433.1 (M+H⁺); Mp = 184-185 °C; ¹H NMR (400 MHz, DMSO-*d*₆): 14.05 (bs, 1H), 8.10 (d, *J* = 8.0 Hz, 1H), 7.97 (d, *J* = 8.0 Hz, 1H), 7.92 (t, *J* = 7.6 Hz, 1H), 7.78-7.74 (m, 2H), 7.36-7.32 (m, 2H), 7.16 (t, *J* = 7.4 Hz, 1H), 6.99 (t, *J* = 7.6 Hz, 2H); ¹³C NMR (100 MHz, DMSO-*d*₆): 166.6, 151.7, 148.5, 145.8, 145.3 (q, *J* = 33.5 Hz, 1C), 145.1, 131.4, 130.0, 129.9, 128.8, 128.5, 128.0, 126.0, 125.3, 123.2, 122.6, 119.9, 110.9, 41.4, 26.5; ¹⁹F NMR (376 MHz, DMSO-*d*₆): -65.9; HRMS (ESI+) calcd for C₂₀H₁₆F₃N₄S₂ (M+H)⁺ 433.0768, found 433.0770.

### Compound 29: 5-[3-(2-hydroxy-5-methoxyphenyl)-5-sulfanylidene-4,5-dihydro-1H-1,2,4-triazol-4-yl]pentanoic acid

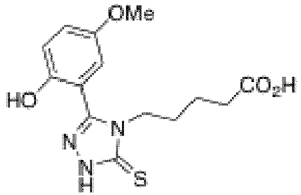

White powder, 26 mg, Yield: 8%; LC-MS *t*_{R}: 1.11 min; *m*/*z* (ES+) 324.1 (M+H⁺); Mp = 155-156 °C; ¹H NMR (400 MHz, DMSO-*d*₆): 13.81 (bs, 1H), 11.85 (bs, 3H), 9.89 (bs, 1H), 7.04-6.85 (m, 3H), 3.87 (t, *J* = 7.0 Hz, 2H), 3.70 (s, 3H), 2.03 (t, *J* = 7.4 Hz, 2H), 1.50 (q, *J* = 7.2 Hz, 2H), 1.27 (q, *J* = 7.2 Hz, 2H); ¹³C NMR (100 MHz, DMSO-*d*₆): 174.0, 166.4, 149.7, 149.4, 118.6, 116.9, 115.4, 113.3, 55.5, 43.2, 32.9, 26.9, 21.2; HRMS (ESI+) calcd for C₁₄H₁₈N₃O₄S (M+H)⁺ 324.1018, found 324.1018.

### Compound 30: 5-[3-(naphthalen-2-yl)-5-sulfanylidene-4,5-dihydro-1H-1,2,4-triazol-4-yl]pentanoic acid

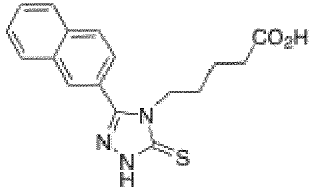

White powder, 83 mg, Yield: 25%; LC-MS *t*_{R}: 1.44 min; *m*/*z* (ES+) 328.0 (M+H⁺); Mp = 170-171 °C; ¹H NMR (400 MHz, DMSO-*d*₆): 14.01 (bs, 1H), 11.96 (bs, 1H), 8.29 (bs, 1H), 8.12-8.01 (m, 3H), 7.80-7.75 (m, 1H), 7.69-7.61 (m, 2H), 4.15 (t, *J* = 7.4 Hz, 2H), 2.12 (t, *J* = 7.2 Hz, 2H);, 1.60 (q, *J* = 7.6 Hz, 2H);, 1.236(q, *J* = 7.4 Hz, 2H); ¹³C NMR (100 MHz, DMSO-*d*₆): 173.9, 167.2, 151.0, 133.3, 132.3, 128.7, 128.5, 128.3, 127.7, 127.0, 125.0, 123.4, 43.4, 32.7, 27.1, 21.2; HRMS (ESI+) calcd for C₁₇H₁₈N₃O₂S (M+H)⁺ 328.1120, found 328.1122.

### Compound 31: 4-[2-(4-hydroxyphenyl)ethyl]-3-phenyl-4,5-dihydro-1H-1,2,4-triazole-5-thione

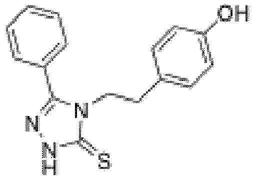

White powder, 109 mg, Yield: 37%; LC-MS *t*_{R}: 1.38 min; *m*/*z* (ES+) 298.1 (M+H⁺); Mp = 208-209 °C; ¹H NMR (400 MHz, DMSO-*d*₆): 13.92 (bs, 1H), 9.22 (bs, 1H), 7.59-7.43 (m, 5H), 6.73 (d, *J* = 8.4 Hz, 2H), 6.58 (d, *J* = 8.4 Hz, 2H), 4.14 (t, *J* = 7.4 Hz, 2H), 2.79 (t, *J* = 7.4 Hz, 2H); ¹³C NMR (100 MHz, DMSO-*d*₆): 166.7,155.9,151.4,130.4,129.3,128.7,128.5,127.1,125.9, 115.2, 45.4, 32.1; HRMS (ESI+) calcd for C₁₆H₁₆N₃OS (M+H)⁺ 298.1014, found .298.1016.

### Compound 32: 4-butyl-3-phenyl-4,5-dihydro-1H-1,2,4-triazole-5-thione

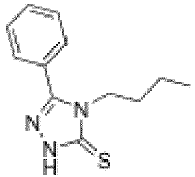

White foam, 132 mg, Yield: 57%; LC-MS *t*_{R}: 1.59 min; *m*/*z* (ES+) 234.1 (M+H⁺); Mp = 128-129 °C; ¹H NMR (400 MHz, DMSO-*d*₆): 13.91 (bs, 1H), 7.68-7.54 (m, 5H), 4.03 (t, *J* = 7.6 Hz, 2H), 1.51 (q, *J* = 7.5 Hz, 2H), 1.11 (st, *J* = 7.4 Hz, 2H), 0.73 (t, *J* = 7.8 Hz, 3H); ¹³C NMR (100 MHz, DMSO-*d*₆): 167.0, 151.1, 130.6, 129.0, 128.5, 126.2, 43.2, 29.4, 18.9, 13.1; HRMS (ESI+) calcd for C₁₂H₁₆N₃S (M+H)⁺ 234.1065, found 324.1065.

### Compound 33: 4-[2-(benzylsulfanyl)ethyl]-3-phenyl-4,5-dihydro-1H-1,2,4-triazole-5-thione

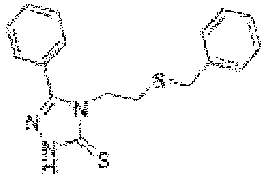

White powder, 212 mg, Yield: 65%; LC-MS *t*_{R}: 1.79 min; *m*/*z* (ES+) 328.0 (M+H⁺); Mp = 108-109 °C; ¹H NMR (400 MHz, DMSO-*d*₆): 13.99 (bs, 1H), 7.69-7.56 (m, 5H), 7.27-7.10 (m, 5H), 4.24 (t, *J* = 7.2 Hz, 2H), 3.51 (s, 2H), 2.64 (t, *J* = 7.2 Hz, 2H); ¹³C NMR (100 MHz, DMSO-*d*₆): 166.9, 151.3, 137.9, 130.7, 129.0, 128.8, 128.6, 128.3, 126.8, 126.0, 43.0, 34.4, 28.0; HRMS (ESI+) calcd for C₁₇H₁₈N₃O₂S₂ (M+H)⁺ 328.0942, found 328.0946.

### Compound 34: 4-[(3-phenyl-5-sulfanylidene-4,5-dihydro-1H-1,2,4-triazol-4-yl)methyl]cyclohexane-1-carboxylic acid

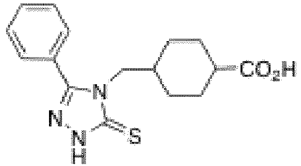

White powder, 71 mg, Yield: 22%; LC-MS *t*_{R}: 1.32 min; *m*/*z* (ES+) 318.2 (M+H⁺); Mp = 207-208 °C; ¹H NMR (400 MHz, DMSO-*d*₆): 13.94 (bs, 1H), 11.95 (bs, 1H), 7.70-7.53 (m, 5H), 3.98 (d, *J* = 7.6 Hz, 2H), 2.04-1.96 (m, 1H), 1.77-1.72 (m, 2H), 1.60-1.47 (m, 1H), 1.46-1.36 (m, 2H), 1.07-0.95 (m, 2H), 0.80-0.68 (m, 2H); ¹³C NMR (100 MHz, DMSO-*d*₆): 176.3, 167.4, 151.3, 130.6, 128.9, 128.7, 126.3, 48.8, 41.8, 35.4, 28.7, 27.8; HRMS (ESI+) calcd for C₁₆H₂₀N₃O₂S (M+H)⁺ 318.1276, found 318.1275.

### Compound 35: 2-{2-[3-(adamantan-1-yl)-5-sulfanylidene-4,5-dihydro-1H-1,2,4-triazol-4-yl]ethyl}benzoic acid

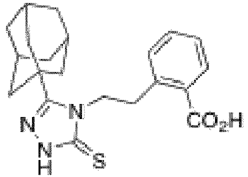

White powder, 72 mg, Yield: 19%; LC-MS *t*_{R}: 1.75 min; *m*/*z* (ES+) 384.4 (M+H⁺); Mp > 250 °C; ¹H NMR (400 MHz, DMSO-*d*₆): 13.63 (bs, 1H), 13.03 (bs, 1H), 7.83 (d, *J* = 7.6 Hz, 1H), 7.62 (d, *J* = 7.2 Hz, 1H), 7.54 (t, *J* = 7.6 Hz, 1H), 7.36 (t, *J* = 7.2 Hz, 1H), 4.36 (t, *J* = 7.6 Hz, 1H), 3.40 (t, *J* = 7.6Hz, 1H), 2.05-1.93 (m, 9H), 1.78-1.66 (m, 6H); ¹³C NMR (100 MHz, DMSO-*d*₆): 168.6, 167.5, 157.2, 138.8, 131.8, 131.3, 131.1, 130.0, 126.7, 36.1, 38.8, 35.6, 34.6, 31.6, 27.3; HRMS (ESI+) calcd for C₂₁H₂₆N₃O₂S (M+H)⁺ 384.1746, found 384.1748.

### Compound 36: 4-[3-(quinolin-2-yl)-5-sulfanylidene-4,5-dihydro-1H-1,2,4-triazol-4-yl]butanoic acid

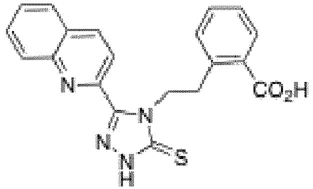

White powder, 111 mg, Yield: 35%; LC-MS *t*_{R}: 1.40 min; *m*/*z* (ES+) 315.1 (M+H⁺); Mp = 238-239 °C; ¹H NMR (400 MHz, DMSO-*d*₆): 14.20 (bs, 1H), 11.74 (bs, 1H), 8.55 (d, *J* = 8.4 Hz, 1H), 8.17-8.04 (m, 3H), 7.87 (t, *J* = 7.6 Hz, 1H), 7.72 (t, *J* = 7.6 Hz, 1H), 4.73 (t, *J* = 7.4 Hz, 1H), 2.37 (t, *J* = 7.4 Hz, 1H), 2.10 (quint, *J* = 7.4 Hz, 1H); ¹³C NMR (100 MHz, DMSO-*d*₆): 173.9, 168.5, 148.3, 147.0, 146.4, 145.8, 137.6, 130.5, 129.2, 127.9, 127.6, 119.6, 44.7, 31.1, 23.8; HRMS (ESI+) calcd for C₁₅H₁₅N₄O₂S (M+H)⁺ 315.0916, found 315.0919.

### Compound 37: 5-Phenyl-4-[(3-phthalideyl)methyl]-2,4-dihydro-1,2,4-triazole-3-thione

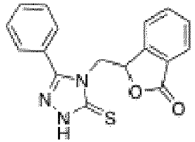

White powder, 385 mg, Yield: 40%; LC-MS *t*_{R}: 1.46 min; *m*/*z* (ES+) 324.1 (M+H⁺); ¹H NMR (500 MHz, DMSO-*d*₆): 14.16 (bs, 1H), 7.82-7.74 (m, 2H), 7.69-7.45 (m, 7H), 6.08 (dd, *J* = 11.0, 4.0 Hz, 1H), 4.77 (dd, *J* = 18.5, 4.0 Hz, 1H), 4.24 (dd, *J* = 18.5, 11.0 Hz, 1H); ¹³C NMR (125 MHz, DMSO-*d*₆): 168.5, 167.2, 151.6, 146.1, 134.5, 130.6, 129.8, 128.8, 128.7, 125.6, 125.1, 125.0, 122.7, 76.9,47.4; HRMS (ESI+) calcd for C₁₇H₁₄N₃O₂S (M+H)⁺ 324.0807, found 324.0809.

### Compound 38: 5-(3-Biphenyl)-4-[3-carboxypropanyl]-1,2,4-triazole-3-thione

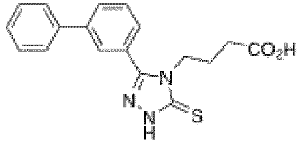

White powder, 61 mg, yield: 33%; LC-MS *t*_{R}: 1.51 min; *m*/*z* (ES+) 340.1 (M+H⁺); ¹H NMR (500 MHz, DMSO-*d*₆): δ (ppm) 13.97 (s, 1H), 12.07 (s, 1H), 7.95 (s, 1H), 7.93-7.87 (m, 1H), 7.78-7.72 (m, 2H), 7.71-7.63 (m, 2H), 7.49 (t, *J* = 7.6 Hz, 2H), 7.41 (t, *J* = 7.4 Hz), 4.19-4.07 (m, 2H), 2.16 (t, *J* = 7.2 Hz, 2H), 1.87-1.76 (m, 2H); ¹³C NMR (125 MHz, DMSO-*d*₆): δ (ppm) 173.5, 167.2, 151.2, 140.9, 139.1, 129.7, 129.1, 129.0, 128.0, 127.7, 126.9, 126.8, 43.3, 30.6, 23.2; HRMS (ESI+) calcd for C₁₈H₁₈N₃O₂S (M+H)⁺ 340.1120, found 340.1119.

### Compound 39: 5-(3-Biphenyl)-4-[2-(2-carboxyphenyl)ethyl]-1,2,4-triazole-3-thione

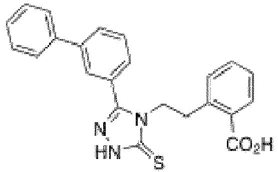

White powder, 84 mg, yield 40%; LC-MS *t*_{R}: 1.67 min; *m*/*z* (ES+) 402.1 (M+H⁺); ¹H NMR (500 MHz, DMSO-*d*₆): δ (ppm) 13.93 (s, 1H), 12.67 (s, 1H), 7.77-7.72 (m, 1H), 7.65-7.60 (m, 2H), 7.55-7.47 (m, 3H), 7.47-7.39 (m, 2H), 7.37-7.31 (m, 2H), 7.18-7.10 (m, 2H), 6.83 (d, *J* = 6.9 Hz, 1H), 4.46 (t, *J* = 6.3 Hz, 2H), 3.32 (t, *J* = 6.3 Hz, 2H); ¹³C NMR (125 MHz, DMSO-*d*₆): δ (ppm) 167.7, 167.0, 151.7, 140.5, 139.1, 138.7, 131.8, 131.3, 130.8, 130.2, 129.2, 129.0, 128.5, 127.8, 127.4, 126.9, 126.7, 126.3, 44.4, 31.5; HRMS (ESI+) calcd for C₂₃H₂₀N₃O₂S (M+H)⁺ 402.1276, found 402.1274.

### Compound 40: 5-(4-Benzyloxyphenyl)-4-[2-(2-carboxyphenyl)ethyl]-1,2,4-triazole-3-thione

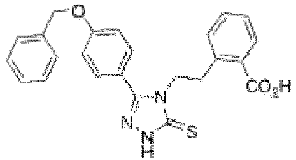

White powder, 27 mg, yield 40%; LC-MS *t*_{R}: 1.72 min; *m*/*z* (ES+) 432.2 (M+H⁺); ¹H NMR (500 MHz, DMSO-*d*₆): δ (ppm) 13.81 (s, 1H), 12.74 (s, 1H), 7.69 (dd, *J* = 7.6, 1.1 Hz, 1H), 7.50-7.33 (m, 6H), 7.28-7.23 (m, 1H), 7.18 (d, *J* = 8.8 Hz, 2H), 7.01 (d, *J* = 8.8 Hz, 2H), 6.96 (d, *J* = 7.6 Hz, 1H), 5.15 (s, 2H), 4.34 (t, *J* = 6.7 Hz, 2H), 3.32 (t, *J* = 6.3 Hz, 2H) ; ¹³C NMR (125 MHz, DMSO-*d*₆): δ (ppm) 167.9, 166.8, 159.8, 151.5, 138.8, 136.7, 131.9, 131.2, 130.9, 130.2, 130.0, 128.5, 128.0, 127.7, 126.9, 118.1, 114.9, 69.4, 44.5, 31.5 ; HRMS (ESI+) calcd for C₂₄H₂₂N₃O₃S (M+H)⁺ 432.1382, found 432.1375.

### Compound 41: 4-[2-(2,4-Dihydroxyphenyl)ethyl]-5-phenyl-1,2,4-triazole-3-thione

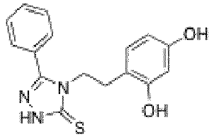

White powder, 27 mg, yield 18%; LC-MS *t*_{R}: 1.04 min; *m*/*z* (ES+) 314.2 (M+H⁺); ¹H NMR (500 MHz, DMSO-*d*₆): δ (ppm) 13.87 (s, 1H), 9.12 (s, 1H), 9.02 (s, 1H), 7.57-7.52 (m, 1H), 7.51-7.45 (m, 4H), 6.54 (d, *J* = 8.1 Hz, 1H), 6.14 (d, *J* = 2.4 Hz, 1H), 6.06 (dd, *J* = 8.1, 2.4 Hz, 1H), 4.11 (t, *J* = 7.4 Hz, 2H), 2.78 (t, *J* = 7.4 Hz, 2H); ¹³C NMR (125 MHz, DMSO-*d*₆): δ (ppm) 167.0, 157.2, 156.2, 151.6, 130.4, 130.4, 128.7, 128.6, 126.0, 113.8, 106.1, 102.3, 44.1, 27.6; HRMS (ESI+) calcd for C₁₆H₁₆N₃O₂S (M+H)⁺ 314.0963, found 314.0964.

### Compound 42: 5-(3-Biphenyl)-4-[2-(2,4-dihydroxyphenyl)ethyl]-1,2,4-triazole-3-thione

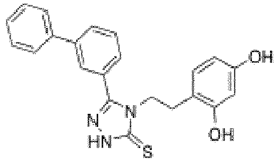

White powder, 16 mg, yield 1%; LC-MS *t*_{R}: 1.30 min; *m*/*z* (ES+) 390.0 (M+H⁺); ¹H NMR (400 MHz, DMSO-*d*₆): δ (ppm) 13.90 (s, 1H), 9.05 (s, 1H), 8.99 (s, 1H), 7.85-7.81 (m, 1H), 7.75-7.68 (m, 3H), 7.58-7.53 (m, 1H), 7.52-7.38 (m, 4H), 6.52 (d, *J* = 8.2 Hz, 1H), 6.11 (d, *J* = 2.3 Hz, 1H), 6.04 (dd, *J* = 8.2, 2.3 Hz, 1H), 4.19 (t, *J* = 7.2 Hz, 2H), 2.81 (t, *J* = 7.2 Hz, 2H); ¹³C NMR (101 MHz, DMSO-*d*₆): δ (ppm) 167.0, 157.1, 156.2, 151.5, 140.6, 139.1, 130.3, 129.3, 129.0, 128.6, 127.8, 127.5, 126.9, 126.7, 113.8, 106.0, 102.3, 44.1, 27.6; HRMS (ESI+) calcd for C₂₂H₂₀N₃O₂S (M+H)⁺ 390.1276, found 390.1281.

### Compound 43: 5-(2-Biphenyl)-4-[2-(2,4-dihydroxyphenyl)ethyl]-1,2,4-triazole-3-thione

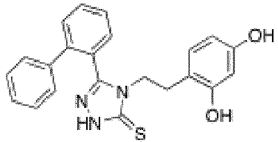

White powder, 186 mg, yield 68%; LC-MS *t*_{R}: 1.27 min; *m*/*z* (ES+) 390.1 (M+H⁺); ¹H NMR (500 MHz, DMSO-*d*₆): δ (ppm) 13.77 (s, 1H, NH), 9.01 (s, 1H, OH), 8.93 (s, 1H, OH), 7.66 (td, *J* = 7.6, 1.2 Hz, 1H), 7.53 (dd, *J* = 7.8, 0.9 Hz, 1H), 7.41 (td, *J* = 7.6, 1.2 Hz, 1H), 7.38-7.31 (m, 3H), 7.21-7.10 (m, 3H), 6.38 (d, *J* = 8.1 Hz, 1H), 6.10 (d, *J* = 2.4 Hz, 1H), 6.03 (dd, *J* = 8.1, 2.4 Hz, 1H), 3.44 (t, *J* = 7.1 Hz, 2H), 2.53-2.50 (m, 2H) ; ¹³C NMR (125 MHz, DMSO-*d*₆): δ (ppm) 166.1, 157.1, 156.2, 151.2, 141.1, 139.2, 131.2, 130.2, 129.9, 128.6, 128.4, 127.8, 127.6, 124.2, 113.8, 105.9, 102.4, 43.2, 27.2; HRMS (ESI+) calcd for C₂₂H₂₀N₃O₂S (M+H)+ 390.1276, found 390.1280.

### Compound 44: o-{2-[3-Methyl-1H-pyrrol-2-yl)-5-thioxo-1,4-dihydro-1,2,4-triazol-4-yl]ethyl}benzoic acid

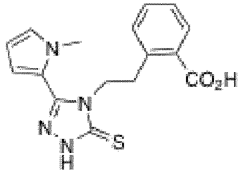

White powder, 37 mg, yield 24%; LC-MS *t*_{R}: 1.39 min; *m*/*z* (ES+) 329.2 (M+H⁺); ¹H NMR (500 MHz, DMSO-*d*₆): 13.83 (bs, 1H), 12.79 (bs, 1H), 7.77 (dd, *J* = 7.8, 1.3 Hz, 1H), 7.41 (dt, *J* = 7.5, 1.3 Hz, 1H), 7.29 (dt, *J* = 7.6, 1.1 Hz, 1H), 7.08 (dd, *J* = 7.6, 0.8 Hz, 1H), 6.89 (dd, *J* = 2.3, 1.8 Hz, 1H), 6.40 (dd, *J* = 3.9, 1.7 Hz, 1H), 6.06 (dd, *J* = 3.8, 2.6 Hz, 1H), 4.37 (t, *J* = 6.86 Hz, 1H), 3.45-3.32 (m, 5H); ¹³C NMR (125 MHz, DMSO-*d*₆): 168.0, 166.3, 144.8, 138.9, 131.6, 131.0, 130.5, 130.4, 126.7, 126.4, 116.5, 112.4, 107.7, 44.6, 34.9, 31.2; HRMS (ESI+) calcd for C₁₆H₁₇N₄O₂S (M+H)⁺ 329.1072, found 329.1076.

### Compound 45: 6-(3-Phenyl-5-sulfanylidene-4,5-dihydro-1H-1,2,4-triazol-4-yl)hexanoic acid

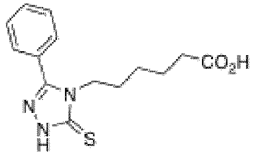

Off-white powder, 215 mg, Yield: 74%; LC-MS *t*_{R}: 1.28 min; *m*/*z* (ES+) 292.1 (M+H⁺); Mp = 136-137 °C; ¹H NMR (400 MHz, DMSO-*d*₆): 13.92 (bs, 1H), 11.98 (bs, 1H), 7.69-7.64 (m, 2H), 7.62-7.54 (m, 3H), 4.01 (t, *J* = 6.0 Hz, 2H), 2.08 (t, *J* = 5.8 Hz, 2H), 1.56-1.16 (m, 2H), 1.39-1.31 (m, 2H), 1.15-1.06 (m, 2H); ¹³C NMR (100 MHz, DMSO-*d*₆): 174.1, 167.0, 151.1, 130.6, 129.0, 128.5, 126.1, 43.4, 33.2, 27.1, 25.1, 23.6; HRMS (ESI+) calcd for C₁₄H₁₈N₃O₂S (M+H)⁺ 292.1121, found 292.1120.

### Compound 46: 3-Phenyl-4-(3-phenyl-5-thioxo-1,4-dihydro-1,2,4-triazol-4-yl)butyric acid

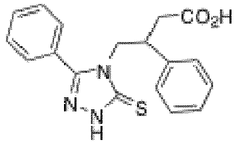

White solid, 27 mg, yield 38%; LC-MS *t*_{R}: 1.42 min; *m*/*z* (ES+) 340.1 (M+H⁺); ¹H NMR (400 MHz, DMSO-*d*₆): δ 13.87 (s, 1H), 12.02 (s, 1H), 7.56 (t, *J* = 7.4 Hz), 7.47 (t, *J* = 7.6 Hz, 2H), 7.15-7.08 (m, 3H), 6.82 (m, 2H), 4.39 (dd, *J* = 14, 7.2 Hz, 1H), 4.18 (dd, *J* = 14.0, 8.5 Hz, 1H), 3.60-3.52 (m, 1H), 2.56-2.52 (m, 1H), 2.47-2.41 (m, 1H); ¹³C NMR (101 MHz, DMSO-*d*₆): δ 172.6, 167.7, 151.8, 140.4, 130.9, 129.2, 129.1, 128.8, 127.9, 127.4, 126.5, 49.0, 40.6, 37.4; HRMS (ESI+) calcd for C₁₈H₁₈N₃O₂S (M+H)⁺ 340.1120, found 340.1119.

### Compound 47: 3-(3-Phenyl-5-sulfanylidene-4,5-dihydro-1H-1,2,4-triazol-4-yl)cyclohexane-1-carboxylic acid

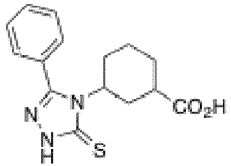

Off-white powder, 171 mg, Yield: 38%; LC-MS *t*_{R}: 1.32 min; *m*/*z* (ES+) 304.1 (M+H⁺); Mp = 234-235 °C; HRMS (ESI+) calcd for C₁₅H₁₈N₃O₂S (M+H)⁺ 304.1122, found 304.1120.

### Compound 48: 4-[3-(1-Methyl-1H-pyrrol-2-yl)-5-thioxo-1,4-dihydro-1,2,4-triazol-4-yl]butyric acid

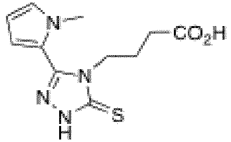

White powder, 121 mg, Yield: 45%; LC-MS *t*_{R}: 1.10 min; *m*/*z* (ES+) 267.2 (M+H⁺); ¹H NMR (500 MHz, DMSO-*d*₆): 13.89 (bs, 1H), 11.82 (bs, 1H), 7.05 (s, 1H), 6.67 (s, 1H), 6.29 (s, 1H), 4.05 (t, *J* = 7.4 Hz, 2H), 3.68 (s, 3H), 2.21(t, *J* = 7.4 Hz, 2H), 1.84 (q, *J* = 7.4 Hz, 2H); ¹³C NMR (125 MHz, DMSO-*d*₆): 173.5, 166.4, 140.8, 126.8, 112.4, 107.9, 104.7, 43.3, 35.3, 30.5, 22.9; HRMS (ESI+) calcd for C₁₁H₁₅N₄O₂S (M+H)⁺ 267.0916, found 267.0919.

### Compound 49: 4-(2-Azidoethyl)-3-phenyl-4,5-dihydro-1H-1,2,4-triazole-5-thione

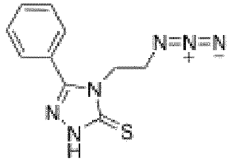

White powder, 94 mg, Yield: 38%; LC-MS *t*_{R}: 1.40 min; *m*/*z* (ES+) 247.1 (M+H⁺); Mp = 108-109 °C; ¹H NMR (400 MHz, DMSO-*d*₆): 14.04 (bs, 1H), 7.74-7.67 (m, 2H), 7.64-7.54 (m, 3H), 4.1 (t, *J* = 6 Hz, 2H), 3.64 (t, *J* = 6 Hz, 2H); ¹³C NMR (100 MHz, DMSO-*d*₆): 166.9, 151.6, 130.7, 128.9, 128.8, 128.7, 47.6, 43.2; HRMS (ESI+) calcd for C₁₀H₁₁N₆S (M+H)⁺ 247.0766, found 247.0769.

### Compound 50: 4-[3-(Quinolin-2-yl)-5-sulfanylidene-4,5-dihydro-1H-1,2,4-triazol-4-yl]butanoic acid

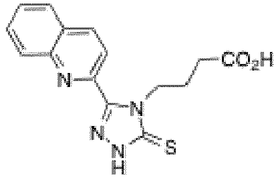

White powder, 111 mg, Yield: 35%; LC-MS *t*_{R}: 1.40 min; *m*/*z* (ES+) 315.1 (M+H⁺); Mp = 238-239 °C; ¹H NMR (400 MHz, DMSO-*d*₆): 14.20 (bs, 1H), 11.74 (bs, 1H), 8.55 (d, *J* = 8.4 Hz, 1H),8.17-8.04 (m, 3H), 7.87 (t, *J* = 7.6 Hz, 1H), 7.72 (t, *J* = 7.6 Hz, 1H), 4.73 (t, *J* = 7.4 Hz, 1H), 2.37 (t, *J* = 7.4 Hz, 1H), 2.10 (quint, *J* = 7.4 Hz, 1H); ¹³C NMR (100 MHz, DMSO-*d*₆): 173.9, 168.5, 148.3, 147.0, 146.4, 145.8, 137.6, 130.5, 129.2, 127.9, 127.6, 119.6, 44.7, 31.1, 23.8; HRMS (ESI+) calcd for C₁₅H₁₅N₄O₂S (M+H)⁺ 315.0916, found 315.0919.

### Compound 51: o-{[3-(3-Biphenylyl)-5-thioxo-1,4-dihydro-1,2,4-triazol-4-ylamino]methyl} benzoic acid

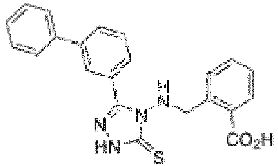

White powder, 163 mg, yield: 81%; LC-MS *t*_{R}: 1.76 min; *m*/*z* (ES+) 403.0 (M+H⁺); ¹H NMR (500 MHz, DMSO-*d*₆): 13.98 (bs, 1H), 12.94 (bs, 1H), 8.08 (t, *J* = 1.5 Hz, 1H), 7.83 (dt, *J* = 7.5, 1.5 Hz, 1H), 7.77-7.71 (m, 2H), 7.58-7.19 (m, 9H), 6.87 (t, *J* = 6.0 Hz, 1H), 4.60 (d, *J* = 6.0 Hz, 2H); ¹³C NMR (125 MHz, DMSO-*d*₆): 168.0, 166.4, 148.8, 140.2, 139.2, 137.2, 131.6, 130.4, 130.3, 130.2, 129.1, 128.9, 128.6, 127.7, 127.4, 126.7, 126.6, 125.9, 125.7, 50.9; HRMS (ESI+) calcd for C₂₂H₁₉N₄O₂S (M+H)⁺ 403.1229, found 403.1233.

### Compound 52: m-[(3-Phenyl-5-thioxo-1,4-dihydro-1,2,4-triazol-4-yl)methyl]benzoic acid

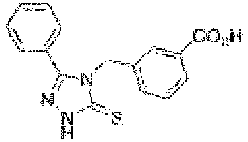

White powder, 27 mg, Yield: 9%; LC-MS *t*_{R}: 1.23 min; *m*/*z* (ES+) 312.0 (M+H⁺); ¹H NMR (500 MHz, DMSO-*d*₆): 14.15 (bs, 1H), 12.91 (bs, 1H), 7.78 (d, *J* = 8.0 Hz, 1H), 7.59 (s, 1H), 7.55-7.42 (m, 5H), 7.38 (t, *J* = 7.8 Hz, 1H), 7.24 (d, *J* = 8.0 Hz, 1H), 5.41 (s, 2H); ¹³C NMR (125 MHz, DMSO-*d*₆): 167.9, 166.8, 151.3, 136.1, 131.0, 130.9, 128.9, 128.8, 128.4, 128.3, 127.3, 125.8, 46.4; HRMS (ESI+) calcd for C₁₆H₁₄N₃O₂S (M+H)⁺ 312.0807, found 312.0809.

### Compound 53: 5-Hydroxy-2-[2-(3-phenyl-5-thioxo-1,4-dihydro-1,2,4-triazol-4-yl)ethyl]benzoic acid

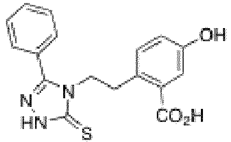

White solid, 58 mg, yield 75%; LC-MS *t*_{R}: 1.18 min; *m*/*z* 342.0 (M+H)⁺; ¹H NMR (500 MHz, DMSO-*d*₆): δ 13.88 (s, 1H), 12.53 (s, 1H), 9.57 (s, 1H), 7.51-7.48 (m, 1H), 7.40 (t, *J* = 7.7 Hz, 2H), 7.26 (dd, *J* = 8.2, 1.1 Hz, 2H), 7.09 (d, *J* = 2.7 Hz, 1H, Hₐᵣ), 6.78 (dd, *J* = 8.3, 2.7 Hz, 1H), 6.70 (d, *J* = 8.3 Hz, 1H), 4.30 (t, *J* = 6.6 Hz, 2H), 3.20 (t, *J* = 6.6 Hz, 2H); ¹³C NMR (125 MHz, DMSO-*d*₆): δ 168.2, 167.4, 156.4, 152.2, 132.8, 131.4, 130.8, 129.3, 129.0, 126.3, 119.4, 117.8, 45.3, 31.2; HRMS (ESI+) calcd for C₁₇H₁₆N₃O₃S (M+H)⁺ 342.0912, found 342.0915.

### Compound 54: 4-[4-(Benzyloxy)phenylethyl]-5-phenyl-1,2,4-triazole-3-thione

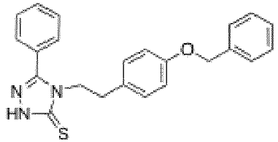

White powder, 87 mg, Yield 66%; LC-MS *t*_{R}: 1.98 min; *m*/*z* (ES+) 388.2 (M+H⁺); ¹H NMR (400 MHz, DMSO-*d*₆): δ (ppm) 13.92 (s, 1H), 7.57-7.52 (m, 1H), 7.50-7.35 (m, 8H), 7.35-7.29 (m, 1H), 6.82 (s, 4H), 5.05 (s, 2H), 4.23-4.11 (m, 2H), 2.93-2.79 (m, 2H); ¹³C NMR (101 MHz, DMSO-*d*₆): δ (ppm) 166.9,157.1,151.5,137.2,130.5,129.5,129.3,128.8,128.6,128.4,127.8, 127.6, 126.0, 114.8, 69.1, 45.3, 32.1; HRMS (ESI+) calcd for C₂₃H₂₂N₃OS (M+H)⁺ 388.1484, found 388.1490.

### Compound 55: p-[(3-Phenyl-5-thioxo-1,4-dihydro-1,2,4-triazol-4-yl)methyl]benzoic acid

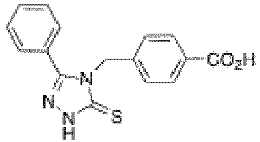

White powder, 47 mg, Yield: 30%; LC-MS *t*_{R}: 1.34 min; *m*/*z* (ES+) 312.2 (M+H⁺); Mp = 232-233 °C; ¹H NMR (500 MHz, DMSO-*d*₆): 14.16 (bs, 1H), 12.60 (bs, 1H), 7.82 (d, *J* = 8.0 Hz, 2H), 7.54-7.42 (m, 5H), 7.12 (d, *J* = 8.0 Hz, 2H), 5.40 (s, 2H); ¹³C NMR (125 MHz, DMSO-*d*₆): 167.9, 151.3, 140.3, 130.7, 129.5, 129.5, 128.8, 128.2, 126.4, 126.4, 125.8, 46.5; HRMS (ESI+) calcd for C₁₆H₁₄N₃O₂S (M+H)⁺ 312.0811, found 312.0807.

### Compound 56: o-{2-[3-(6-Quinolyl)-5-thioxo-1,4-dihydro-1,2,4-triazol-4-yl]ethyl}benzoic acid

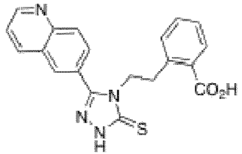

White powder, 201 mg, Yield: 53%; LC-MS *t*_{R}: 1.04 min; *m*/*z* (ES+) 377.0 (M+H⁺); ¹H NMR (500 MHz, DMSO-*d*₆): 14.05 (bs, 1H), 12.63 (bs, 1H), 8.98 (dd, *J* = 4.0, 1.5 Hz, 1H), 8.42 (d, *J* = 8.5 Hz, 1H), 7.99 (d, *J* = 8.5 Hz, 1H), 7.86 (d, *J* = 8.5 Hz, 1H), 7.65-7.58 (m, 2H), 7.51 (dd, *J* = 8.0, 1.5 Hz, 1H), 7.37 (td, *J* = 7.5, 1.0 Hz, 1H), 7.22 (td, *J* = 7.5, 1.0 Hz, 1H), 6.98 (d, *J* = 7.5 Hz, 1H), 4.47 (t, *J* = 6.5 Hz, 2H), 3.37 (t, *J* = 6.5 Hz, 2H); ¹³C NMR (125 MHz, DMSO-*d*₆): 167.8, 167.1, 151.9, 150.9, 147.8, 138.5, 136.7, 131.6, 131.2, 130.8, 130.5, 129.3, 128.7, 128.6, 127.2, 126.8, 123.5, 122.1, 44.7, 31.4; HRMS (ESI+) calcd for C₂₀H₁₇N₄O₂S (M+H)⁺ 337.1072, found 377.1072.

### Compound 57: o-{2-[3-(1H-Pyrrol-2-yl)-5-thioxo-1,4-dihydro-1,2,4-triazol-4-yl]ethyl}benzoic acid

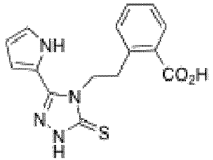

White powder, 99 mg, Yield: 63%; LC-MS *t*_{R}: 1.33 min; *m*/*z* (ES+) 315.1 (M+H⁺); ¹H NMR (500 MHz, DMSO-*d*₆): 13.78 (bs, 1H), 12.57 (bs, 1H), 11.58 (bs, 1H), 7.82 (dd, *J* = 7.8, 1.2 Hz, 1H), 7.47 (dt, *J* = 7.5, 1.3 Hz, 1H), 7.37-7.23 (m, 2H), 6.94 (s, 1H), 6.73 (s, 1H), 6.16 (dd, *J* = 6.0, 2.5 Hz, 1H), 4.43 (t, *J* = 7.2 Hz, 2H), 3.37 (t, *J* = 7.2 Hz, 2H); ¹³C NMR (125 MHz, DMSO-*d*₆): 168.4, 166.6, 145.5, 138.9, 131.9, 131.1, 130.4, 130.3, 126.8, 121.7, 116.2, 110.0, 109.1, 44.8, 31.6; HRMS (ESI+) calcd for C₁₅H₁₅N₄O₂S (M+H)⁺ 315.0916, found 315.0917.

### Compound 58: o-{2-[3-(3-Thienyl)-5-thioxo-1,4-dihydro-1,2,4-triazol-4-yl]ethyl}benzoic acid

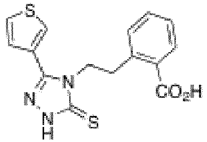

White powder, 118 mg, Yield: 36%; LC-MS *t*_{R}: 1.35 min; *m*/*z* (ES+) 332.1 (M+H⁺); ¹H NMR (500 MHz, DMSO-*d*₆): 13.88 (bs, 1H), 12.94 (bs, 1H), 7.88-7.86 (m, 1H), 7.75 (dd, *J* = 9.5, 1.0 Hz, 1H), 7.66-7.63 (m, 1H), 7.64 (td, *J* = 9.5, 1.5 Hz, 1H), 7.29 (td, *J* = 9.5, 1.0 Hz, 1H), 7.18 (dd, *J* = 6.0, 1.5 Hz, 1H), 7.07 (d, *J* = 9.5 Hz, 1H), 4.42 (t, *J* = 8.8 Hz, 2H), 3.34 (t, *J* = 8.8 Hz, 2H); ¹³C NMR (125 MHz, DMSO-*d*₆): 168.1, 166.9, 147.4, 138.7, 131.8, 131.2, 130.6, 130.2, 127.3, 127.1, 126.9, 126.8, 125.9, 44.7, 31.6; HRMS (ESI+) calcd for C₁₅H₁₄N₃O₂S₂ (M+H)⁺ 332.0527, found 332.0529.

### Compound 59: 8-(3-Phenyl-5-thioxo-1,4-dihydro-1,2,4-triazol-4-yl)octanoic acid

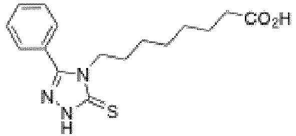

White powder, 132 mg, Yield: 60%; LC-MS *t*_{R}: 1.38 min; *m*/*z* (ES+) 320.1 (M+H⁺); ¹H NMR (500 MHz, DMSO-*d*₆): 13.92 (bs, 1H), 12.03 (bs, 1H), 7.70-7.53 (m, 5H), 4.02 (t, *J* = 9.5 Hz, 2H), 2.12 (t, *J* = 9.5 Hz, 2H), 1.52 (sext, *J* = 9.5 Hz, 2H), 1.39 (sext, *J* = 9.5 Hz, 2H), 1.19-1.05 (m, 6H); ¹³C NMR (125 MHz, DMSO-*d*₆): 174.3, 166.9, 151.1, 130.6, 128.9, 128.5, 126.2, 43.4, 33.5, 28.1, 27.7, 27.1, 25.4, 24.2; HRMS (ESI+) calcd for C₁₆H₂₂N₃O₂S (M+H)⁺ 320.1433, found 320.1437.

### Compound 60: Phenyl[2-(3-phenyl-5-thioxo-1,4-dihydro-1,2,4-triazol-4-yl)ethylthio]acetic acid

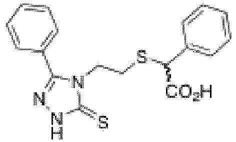

White powder, 417 mg, Yield: 56%; LC-MS *t*_{R}: 1.52 min; *m*/*z* (ES+) 372.0 (M+H⁺); ¹H NMR (600 MHz, DMSO-*d*₆): 13.32 (bs, 1H), 11.62 (bs, 1H), 7.64-7.53 (m, 5H), 7.32-7.24 (m, 5H), 4.59 (s,1H), 4.27-4.14 (m, 2H), 2.77-2.70 (m, 1H), 2.62-2.56 (m, 1H); ¹³C NMR (150 MHz, DMSO-*d*₆): 171.2, 166.9, 151.1, 136.7, 130.7, 129.0, 128.6, 128.4, 128.1, 127.8, 125.8, 51.2, ,43.0, 28.2; HRMS (ESI+) calcd for C₁₈H₁₈N₃O₂S₂ (M+H)⁺ 372.0840, found 372.0846.

### Compound 61: 5-Amino-2-[2-(3-phenyl-5-thioxo-1,4-dihydro-1,2,4-triazol-4-yl)ethyl]benzoic acid

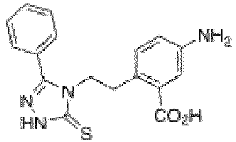

White foam, 38 mg, yield 34%; LC-MS *t*_{R}: 0.97 min; *m*/*z* 341.1 (M+H)⁺; ¹H NMR (600MHz, DMSO-*d*₆): δ 13.87 (s, 1H), 12.58 (s, 1H), 7.52-7.48 (m, 1H), 7.43-7.39 (m, 2H), 7.25 (dt, *J* = 8.3, 1.5 Hz, 2H), 7.13 (s, 2H), 6.78 (d, *J* = 7.1 Hz, 1H), 6.69 (d, *J* = 8.1 Hz, 2H), 4.28 (t, *J* = 6.6 Hz, 2H), 3.81-3.69 (m, 2H), 3.19 (t, *J* = 6.6 Hz, 2H); HRMS (ESI+) calcd for C₁₇H₁₇N₄O₂S (M+H)⁺ 341.1072, found 341.1077.

### Example 4: Experimental details of the biological evaluation

### 1- Enzymatic assays for the determination of inhibition constants.

The inhibition potency of the compounds has been assessed with a reporter substrate method and specifically by measuring the rate of hydrolysis of the reporter substrate (such as 150 µM imipenem, 150 µM meropenem, 120 µM cefotaxime or 100 µM nitrocefin) in the absence and presence of several concentrations of the inhibitor (final concentration, 0.5 - 1,000 µM).

The reaction rate was measured as the variation of absorbance observed upon substrate hydrolysis in a UV-Vis spectrophotometer or microplate reader at a wavelength of 300 nm (imipenem, meropenem), 260 nm (cefotaxime) or 482 nm (nitrocefin) in 50 mM HEPES buffer (pH 7.5) and in the presence of a purified MBL enzyme (such as VIM-1, VIM-2, VIM-4, NDM-1 and IMP-1, at a final concentration ranging 1 - 70 nM).

The inhibition constants (*K*ᵢ) were determined on the basis of a model of competitive inhibition by analyzing the dependence of the ratio *v*₀/*v*ᵢ (*v*₀, hydrolysis velocity in the absence of inhibitor; *v*ᵢ, hydrolysis velocity in the presence of inhibitor) as a function of [I] as already described [Docquier J.D., Lamotte-Brasseur J., Galleni M., Amicosante G., Frère J.M., Rossolini G.M. (2003) J. Antimicrob. Chemother. 51, 257-266]. The slope of the plot of *v*₀/*v*ᵢ vs [I], which corresponds to *K*ₘ^{S}/(*K*ₘ^{S} + [S])*Kᵢ* (where *K*ₘ^{S} is the *K*ₘ value of the reporter substrate and [S] its concentration in the reaction mixture) and allowed the calculation of the *Kᵢ* value. Alternatively, a Dixon plot analysis was carried out, by measuring the initial hydrolysis rates in the presence of variable concentrations of inhibitor and substrate. This allowed to determine *Kᵢ* values and to support that the various compounds behaved as competitive inhibitors of the various tested enzymes.

The results of inhibition tests are presented in Table 1. Numerous compounds showed *Kᵢ* in the micromolar or submicromolar range or > 85% inhibition at 100 µM toward at least one important MBLs such as VIM-type or NDM-type enzymes (e.g., NDM-1). Among them, several showed a large spectrum of inhibition.

**Table 1: MBLs inhibitors and their inhibitory potency toward isolated MBLs.**

| | **Ki^{a}** | | | | |
|---|---|---|---|---|---|
| **Cpd** | **VIM-1** | **VIM-2** | **VIM-4** | **NDM-1** | **IMP-1** |
| **1** | - | +++ | ++ | - | - |
| **2** | - | ++ | ++ | - | - |
| **3** | - | +++ | 75% at 100 µM | 60% at 200 µM | + |
| **4** | - | +++ | 80% at 100 µM | - | - |
| **5** | - | ++ | - | - | - |
| **6** | 60% at 200 µM | 60% at 200 µM | 50% at 100 µM | - | 80% at 200 µM |
| **7** | +++ | ++ | ++ | - | - |
| **8** | ND | - | +++ | - | 70% at 200 µM |
| **9** | ND | - | ++ | - | ++ |
| **10** | ++ | ++ | ++ | ++ | + |
| **11** | ND | - | 75% at 100 µM | - | ++ |
| **12** | ND | +++ | 70% at 100 µM | - | - |
| **13** | - | ++ | ND | - | - |
| **14** | ++ | +++ | 80% at 100 µM | - | - |
| **15** | - | ++ | 85% at 100 µM | - | 60% at 200 µM |
| **16** | - | ++ | ND | - | - |
| **17** | ND | ++ | 60% at 100 µM | - | - |
| **18** | - | ++ | 90% at 100 µM | - | - |
| **19** | - | ++ | 75% at 100 µM | - | - |
| **20** | ND | 70% at 200 µM | - | + | - |
| **21** | - | ++ | 95% at 100 µM | - | - |
| **22** | ND | +++ | 80% at 100 µM | - | - |
| **23** | ND | - | - | - | 95% at 200 µM |
| **24** | ND | 70% at 200 µM | 85% at 100 µM | - | - |
| **25** | ND | ++ | 85% at 100 µM | - | - |
| **26** | ND | ++ | 85% at 100 µM | - | - |
| **27** | ND | ++ | 85% at 100 µM | - | - |
| **28** | ND | - | - | + | - |
| **29** | ND | 60% at 200 µM | 90% at 100 µM | - | - |
| **30** | ND | ++ | 80% at 100 µM | - | 60% at 200 µM |
| **31** | ++ | ++ | 70% at 100 µM | + | + |
| **32** | ND | ++ | 75% at 100 µM | - | + |
| **33** | +++ | ++ | +++ | ++ | - |
| **34** | ND | 75% at 200 µM | 90% at 100 µM | - | - |
| **35** | ND | ++ | 55% at 100 µM | - | - |
| **36** | ND | - | 85% at 100 µM | - | - |
| **37** | +++ | - | - | 50% at 100 µM | - |
| **38** | - | +++ | +++ | - | - |
| **39** | ++ | +++ | ++ | - | - |
| **40** | - | +++ | ++ | - | - |
| **41** | - | - | - | + | + |
| **42** | +++ | ++ | +++ | + | - |
| **43** | - | - | + | + | - |
| **44** | ND | +++ | 80% at 100 µM | - | - |
| **45** | ND | +++ | 85% at 100 µM | 55% at 100 µM | - |
| **46** | - | - | 80% at 100 µM | - | - |
| **47** | ND | 60% at 100 µM | 70% at 100 µM | - | - |
| **48** | ND | - | 75% at 100 µM | - | - |
| **49** | ND | - | - | - | 90% at 200 µM |
| **50** | ND | 80% at 200 µM | 85% at 100 µM | 50% at 200 µM | - |
| **51** | ND | 75% at 100 µM | ND | - | - |
| **52** | ND | 84% at 100 µM | ND | - | - |
| **53** | ND | 90% at 100 µM | ND | - | - |
| **54** | ND | + | - | - | 60% at 200 µM |
| **55** | ND | 80% at 200 µM | 80% at 100 µM | - | - |
| **56** | ND | 85% at 100 µM | ND | - | - |
| **57** | ND | 80% at 200 µM | 65% at 100 µM | 60% at 200 µM | - |
| **58** | ND | 85% at 100 µM | ND | - | - |
| **59** | ND | 90% at 100 µM | ND | - | - |
| **60** | ND | 96% at 100 µM | ND | - | - |
| **61** | ND | 75% at 100 µM | ND | - | - |

| | | | | | |
|---|---|---|---|---|---|
| ^{a} ND = not determined; - = no significant inhibition or Ki > 100µM; + = 10µM < Ki ≤ 100µM; ++ = 1µM < Ki ≤ 10µM; +++ = Ki ≤ 1µM When Ki was not evaluated, the % inhibition at the maximal tested concentration is indicated. | | | | | |

### 2- Microbiological assays to determine the synergistic activity of MBL inhibitors with β-lactams.

The microbiological assays were carried out using different methodologies:
A] A disk-diffusion test in which the inhibitor was added (40 - 320 µg) to a disk containing a defined amount of a β-lactam antibiotic (such as 30 µg cefoxitin) was used to evaluate the antimicrobial activity (corresponding to the diameter of the growth inhibition zone, expressed in mm) of the combination when compared of that of the antibiotic alone.

These tests were performed on isogenic laboratory strains (such as *Escherichia coli* DH5α or XL-1) producing a defined MBL after its transformation with a pLB-II plasmid derivative (such as pLBII-VIM-2) [Borgianni L., Vandenameele J., Matagne A., Bini L., Bonomo R.A., Frère J.M., Rossolini G.M., Docquier J.D. (2010) Antimicrob. Agents Chemother. 54, 3197-3204] harbouring a cloned gene encoding the functional metallo-β-lactamase. Alternatively, hyperpermeable strains (such as *Escherichia coli* AS19) were used instead of *Escherichia coli* DH5α or XL-1.

Results are presented in Table 2. These first microbiological tests performed on recombinant VIM-2 producing bacteria were very promising as several compounds showed capacity to restore the sensitivity to cefoxitin.

**Table 2: Microbiological assays - in vitro antibacterial synergistic activity of MBL inhibitors with cefoxitin in a combo test setup**

| **Activity on *E. coli* AS19 and LZ2310[pLBII-VIM2] (*E. coli* K12 *ΔnorE ΔmdfA N43 acrA1*)** | | | |
|---|---|---|---|
| **Compound added to cefoxitin disk (30 µg)*^{a}*** | ***K*ᵢ*^{d}* on VIM-2** | ***Inhibition zone di ameter (mm)^{b,c}*** | |
| | | **AS 19** | **LZ 2310** |
| **None** | N.A. | 16 | 14 |
| **EDTA (220 µg)** | N.A. | 26 | 30 |
| II | ++ | | +3 |
| **14** | +++ | +3 | +9 |
| **1** | +++ | | +9 |
| **5** | ++ | | +6 |
| **4** | +++ | +4 | +13 |
| **12** | +++ | +3 | +7 |
| **13** | ++ | +4 | +11 |
| **2** | ++ | | +6 |
| **15** | ++ | +3 | +8 |

| | | | |
|---|---|---|---|
| *^{a}*A variable volume of the compound was added to the disc in order to obtain a final inhibitor quantity of 40 µg. *^{b}*EUCAST resistance breakpoint = 18 mm (i.e. +4) for LZ2310 ^{c}Difference between measured diameter and diameter without compound. ^{d}N.A. = not applicable; ++ = 1µM < Ki ≤ 10µM; +++ = Ki ≤ 1µM | | | |

B] A broth microdilution method in which the Minimum Inhibitory Concentration (MIC) of a β-lactam antibiotic (such as imipenem, meropenem or ceftazidime) was measured in both the absence and presence of a fixed concentration (such as 16 µg/ml, 32 µg/ml or 64 µg/ml) of the various inhibitors.

This method was performed in agreement with the CLSI recommendations (document M07-A10, 2015, "Methods for Dilution Antimicrobial Susceptibility Tests for Bacteria That Grow Aerobically").

A lower MIC of the combination, when compared to that of the antibiotic alone, indicates a potentiation of the antibiotic activity by the tested compound. Such tests were performed using either isogenic laboratory strains producing a defined MBL as described above or clinical isolates showing multi-drug resistance phenotypes and in which the production of a metallo-β-lactamase was confirmed by molecular methods.

MIC values of meropenem (MEM) were determined in duplicate in Mueller-Hinton broth as recommended by CLSI (document M07-A10, 2015), in absence and presence of a fixed concentration of MBL inhibitor (32 µg/ml). Compounds showing a potentiation fold ≥ 3.log₂ dilutions are indicated in bold in Table 3 below. Compounds **37, 39,** and **42** significantly decreased the MIC of Meropenem toward VIM-producing *Klebsiella* clinical isolates. The effect is modest on NDM-1-producing *E. coli.*

***Table 3: MIC values of meropenem (MEM) in absence and presence of a MBL inhibitor.***

| Cpd | MIC (µg/ml) | | |
|---|---|---|---|
| | *K. pneumoniae* SI-001 (*bla*_{VIM-1}+) | *K. pneumoniae* SI-002 (*bla*_{VIM-4}+) | *E. coli* SI-003 (*bla*_{NDM-1}+) |
| - | 128 | 128 | >256 |
| **37** | **16** | **16** | 128 |
| **39** | **16** | **8** | 128 |
| **42** | **8** | **8** | 64 |

### 3- Cell membrane integrity assays on eukaryotic cells to evaluate compound cytotoxicity.

Selected compounds were tested for their ability to induce the lysis of eukaryotic cells by measuring the release of lactate dehydrogenase enzyme (LDH) from HeLa cells, after incubating such cells for 24 hours (37 °C, 5% CO₂) in Dulbecco's modified Eagle medium supplemented with 10% fetal bovine serum, 4.5 mg/ml glucose and 2 mM L-glutamine in the absence and presence of the compound at concentrations ranging from 0.001 to 1.2 mM. LDH activity was determined using commercial kits (such as the CytoTox 96® Non-Radioactive Cytotoxicity Assay, Promega, Madison, Wisconsin, U.S.A.) in the samples, containing HeLa cells incubated with the various concentrations of the assayed compound or with the solvent or buffer used to resuspend such compounds (vehicle control).

Further controls included samples containing the medium only (medium control) or in which cell lysis was induced by the addition of 9% Triton X-100 (maximum LDH release control). The percentage of cytotoxicity was computed as 100 x (Sample LDH release) / (maximum LDH release). The variation of the percentage of cytotoxicity is usually dependent on the compound concentration and allowed to compute an IC₅₀ value, corresponding to the compound concentration inducing 50% cytoxicity.

By using this procedure, compounds 12 (2-{2-[3-(furan-2-yl)-5-sulfanylidene-4,5-dihydro-1H-1,2,4-triazol-4-yl]ethyl}benzoic acid), 13 (2-{2-[3-(thiophen-2-yl)-5-sulfanylidene-4,5-dihydro-1H-1,2,4-triazol-4-yl]ethyl}benzoic acid) and 14 (2-{2-[3-(2-methylphenyl)-5-sulfanylidene-4,5-dihydro-1H-1,2,4-triazol-4-yl]ethyl}benzoic acid) appeared to have a very low cytotoxicity, with an IC₅₀ value estimated to be >1.2 mM.

## Claims

1. A compound of formula (I) or a pharmaceutically acceptable salt thereof, as shown below, 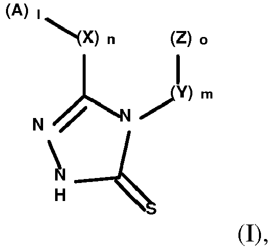 wherein,
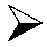 n and m are independently 0 or 1;
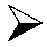 1 is 1 or 2;
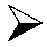 o is 0, 1 or 2;
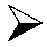 when 1 is 2, n is 1;
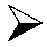 when o is 2, m is 1;
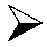 when m is 0, o is 1;
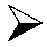 when n is 0,1 is 1;
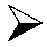 (X) represents a (C₁-C₃)alkyl;
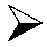 (A) represents a 5-14 membered ring selected in the group consisting of an aryl, a heteroaryl, a cycloalkyl, said 5-14 membered ring is optionally substituted by at least one radical selected in the group consisting of:
▪ a halogen,
▪ OH,
▪ a (C₁-C₆)alkoxy, optionally substituted by a heterocycloalkyl,
▪ a (C₁-C₆)alkyl, optionally substituted by at least one halogen, and
▪ an aryl or aryl(C₁-C₆)alkyloxy;
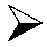 (Y) represents a (C₁-C₁₀)alkyl chain, comprising optionally a heteroatomic group;
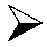 (Z) represents
∘ a 5-14 membered ring selected in the group consisting of an aryl, a cycloalkyl, a heterocycle, said 5-14 membered ring being optionally substituted by at least one radical selected in the group consisting of:
▪ a halogen,
▪ OH, COOH, NO₂, NH₂,
▪ a (C₁-C₆)alkyl, optionally substituted by at least one halogen, or by COOH,
▪ An aryl (preferably a phenyl) optionally substituted by NO₂, or aryl(C₁-C₆)alkyloxy;
∘ A radical selected in the group consisting of:
▪ COOH,
▪ N₃,
▪ a NR₁R₂ group, wherein R₁ and R₂ represent independently H, Boc, (C₁-C₆)alkyl,
▪ SO₂-R₇, wherein R₇ represents a (C₁-C₆)alkyl, or a 5-14 membered ring, preferably an aryl, optionally substituted by an (C₁-C₆)alkyl,
▪ a benzoyl, and
▪ N-phenylurea; or
∘ a (C₁-C₆)alkyl, optionally substituted by at least one halogen or by COOH.

2. Compound according to claim 1, wherein n=1 and X is a methyl or an ethyl.

3. Compound according to claim 1, wherein n=0 and wherein A is an aryl selected in the group consisting of phenyl optionally substituted by a phenyl or a benzyloxy, and a naphthalenyl.

4. Compound according to claim 1, wherein n=0 and wherein A is a phenyl substituted by at least one radical selected in the group consisting of methyl, methoxy, CF₃, OH, halogen.

5. Compound according to claim 1, wherein n=0 and wherein A is a 5-membered ring selected in the group consisting of furanyl, thiophenyl, optionally substituted by a halogen, pyrrolyl, optionally substituted by a methyl.

6. Compound according to claim 1, wherein n=0 and wherein A is quinolinyl.

7. Compound according to claims 1 to 6, wherein m=1 and Y is selected in the group consisting of (C₁-C₇)alkyl chain, optionally comprising a heteroatomic group.

8. Compound according to claims 1 to 7, wherein o=0, m=1 and Y is (C₁-C₇)alkyl chain.

9. Compound according to claims 1 to 7, wherein m=1 and Y is (C₁-C₇)alkyl chain, and wherein o=1 and Z is COOH.

10. Compound according to claims 1 to 7, wherein m=1 and Y is (C₁-C₇)alkyl chain, optionally comprising a heteroatomic group, and wherein o=2 and Z is selected in the group consisting of
- a (C₁-C₃)alkyl, preferably a methyl, optionally substituted by COOH,
- a phenyl optionally substituted by Cl,
- NH-Boc, COOH and N-phenylurea.

11. Compound according to claims 1 to 7, wherein o is 1 and Z is a cyclohexyl substituted by COOH or by a (C₁-C₆)alkyl, preferably a methyl, substituted by COOH.

12. Compound according to claims 1 to 7, wherein m=1 and Y is (C₁-C₇)alkyl chain, comprising optionally a heteroatomic group, and wherein o=1 and Z is a phenyl substituted by a COOH, and optionally a OH or a NH₂.

13. Compound according to claims 1 to 7, wherein o=1 and Z is a phenyl, substituted by at least one OH, preferably two.

14. Compound according to claims 1 to 7, wherein o=1 and Z is a phenyl, substituted by a benzyloxyphenyl.

15. Compound according to claims 1 to 7, wherein m=1 and Y is a (C₁-C₅)alkyl chain, comprising a heteroatomic group, preferably S.

16. Compound according to claims 1 to 7, wherein l and o are 1, A is a phenyl optionally substituted by at least one radical selected from methyl, OMe, OH, halogen and CF₃, and Z is a phenyl optionally substituted by at least one radical selected from COOH, OH.

17. Compound according to claims 16, wherein A is a phenyl optionally substituted by at least one radical selected from OMe and OH, Z is a phenyl optionally substituted by a COOH or at least one OH, and m is 1 and, Y is an ethyl or a methyl.

18. Compound according to claims 1 to 7, wherein l and o are 1, A is a phenyl substituted by a phenyl, and Z is a COOH or a phenyl optionally substituted by at least one radical selected from COOH, OH.

19. Compound according to any one of the preceding claims or a pharmaceutically acceptable salt thereof, selected from :
- Compound 1. 2-[2-(3-phenyl-5-sulfanylidene-4,5-dihydro-1H-1,2,4-triazol-4-yl)ethyl]benzoic acid;
- Compound 2. 4-(3-phenyl-5-sulfanylidene-4,5-dihydro-1H-1,2,4-triazol-4-yl)butanoic acid;
- Compound 3. 2-{2-[3-(naphthalen-2-yl)-5-sulfanylidene-4,5-dihydro-1H-1,2,4-triazol-4-yl]ethyl}benzoic acid;
- Compound 4. 2-{2-[3-(2-hydroxy-5-methoxyphenyl)-5-sulfanylidene-4,5-dihydro-1H-1,2,4-triazol-4-yl]ethyl}benzoic acid;
- Compound 5. 2-{[(3-phenyl-5-sulfanylidene-4,5-dihydro-1H-1,2,4-triazol-4-yl)amino]methyl}benzoic acid;
- Compound 6. 3-(2-methylphenyl)-4-(2-phenylethyl)-4,5-dihydro-1H-1,2,4-triazole-5-thione;
- Compound 7. 4-benzyl-3-(2-hydroxy-5-methoxyphenyl)-4,5-dihydro-1H-1,2,4-triazole-5-thione;
- Compound 8. 3-phenyl-4-(3-phenylpropyl)-4,5-dihydro-1H-1,2,4-triazole-5-thione;
- Compound 9. 4-benzyl-3-phenyl-4,5-dihydro-1H-1,2,4-triazole-5-thione;
- Compound 10. 3-(2-hydroxy-5-methoxyphenyl)-4-(2-phenylethyl)-4,5-dihydro-1H-1,2,4-triazole-5-thione;
- Compound 11. 3-phenyl-4-(2-phenylethyl)-4,5-dihydro-1H-1,2,4-triazole-5-thione;
- Compound 12. 2-{2-[3-(furan-2-yl)-5-sulfanylidene-4,5-dihydro-1H-1,2,4-triazol-4-yl]ethyl}benzoic acid;
- Compound 13. 2-{2-[3-(thiophen-2-yl)-5-sulfanylidene-4,5-dihydro-1H-1,2,4-triazol-4-yl]ethyl}benzoic acid;
- Compound 14. 2-{2-[3-(2-methylphenyl)-5-sulfanylidene-4,5-dihydro-1H-1,2,4-triazol-4-yl]ethyl}benzoic acid;
- Compound 15. 5-(3-phenyl-5-sulfanylidene-4,5-dihydro-1H-1,2,4-triazol-4-yl)pentanoic acid;
- Compound 16. 2-({[3-(naphthalen-2-yl)-5-sulfanylidene-4,5-dihydro-1H-1,2,4-triazol-4-yl]amino}methyl)benzoic acid;
- Compound 17. 2-{1-[(3-phenyl-5-sulfanylidene-4,5-dihydro-1H-1,2,4-triazol-4-yl)methyl]cyclohexyl}acetic acid;
- Compound 18. 4-[3-(2-hydroxy-5-methoxyphenyl)-5-sulfanylidene-4,5-dihydro-1H-1,2,4-triazol-4-yl]butanoic acid;
- Compound 19. 4-[3-(naphthalen-2-yl)-5-sulfanylidene-4,5-dihydro-1H-1,2,4-triazol-4-yl]butanoic acid;
- Compound 20. 3-phenyl-4-[(1R,2S)-2-phenylcyclopropyl]-4,5-dihydro-1H-1,2,4-triazole-5-thione;
- Compound 21. 3-(4-chlorophenyl)-4-(3-phenyl-5-sulfanylidene-4,5-dihydro-1H-1,2,4-triazol-4-yl)butanoic acid;
- Compound 22. 2-{2-[3-(3-methoxyphenyl)-5-sulfanylidene-4,5-dihydro-1H-1,2,4-triazol-4-yl]ethyl}benzoic acid;
- Compound 23. 2-{2-[3-(4-methyl-1,2,3-thiadiazol-5-yl)-5-sulfanylidene-4,5-dihydro-1H-1,2,4-triazol-4-yl]ethyl}benzoic acid;
- Compound 24. 4-[3-(3-methoxyphenyl)-5-sulfanylidene-4,5-dihydro-1H-1,2,4-triazol-4-yl]butanoic acid;
- Compound 25. 2-{[(tert-butoxy)carbonyl]amino}-5-(3-phenyl-5-sulfanylidene-4,5-dihydro-1H-1,2,4-triazol-4-yl)pentanoic acid;
- Compound 26. 4-hexyl-3-phenyl-4,5-dihydro-1H-1,2,4-triazole-5-thione;
- Compound 27. 2-{2-[3-(5-chlorothiophen-2-yl)-5-sulfanylidene-4,5-dihydro-1H-1,2,4-triazol-4-yl]ethyl}benzoic acid;
- Compound 28. 3-phenyl-4-(2-{[2-(trifluoromethyl)quinolin-4-yl]sulfanyl}ethyl)-4,5-dihydro-1H-1,2,4-triazole-5-thione;
- Compound 29. 5-[3-(2-hydroxy-5-methoxyphenyl)-5-sulfanylidene-4,5-dihydro-1H-1,2,4-triazol-4-yl]pentanoic acid;
- Compound 30. 5-[3-(naphthalen-2-yl)-5-sulfanylidene-4,5-dihydro-1H-1,2,4-triazol-4-yl]pentanoic acid;
- Compound 31. 4-[2-(4-hydroxyphenyl)ethyl]-3-phenyl-4,5-dihydro-1H-1,2,4-triazole-5-thione;
- Compound 32. 4-butyl-3-phenyl-4,5-dihydro-1H-1,2,4-triazole-5-thione;
- Compound 33. 4-[2-(benzylsulfanyl)ethyl]-3-phenyl-4,5-dihydro-1H-1,2,4-triazole-5-thione;
- Compound 34. 4-[(3-phenyl-5-sulfanylidene-4,5-dihydro-1H-1,2,4-triazol-4-yl)methyl]cyclohexane-1-carboxylic acid;
- Compound 35. 2-{2-[3-(adamantan-1-yl)-5-sulfanylidene-4,5-dihydro-1H-1,2,4-triazol-4-yl]ethyl}benzoic acid;
- Compound 36. 4-[3-(quinolin-2-yl)-5-sulfanylidene-4,5-dihydro-1H-1,2,4-triazol-4-yl]butanoic acid;
- Compound 37. 5-Phenyl-4-[(3-phthalideyl)methyl]-2,4-dihydro-1,2,4-triazole-3-thione;
- Compound 38. 5-(3-Biphenyl)-4-[3-carboxypropanyl]-1,2,4-triazole-3-thione;
- Compound 39. 5-(3-Biphenyl)-4-[2-(2-carboxyphenyl)ethyl]-1,2,4-triazole-3-thione;
- Compound 40. 5-(4-Benzyloxyphenyl)-4-[2-(2-carboxyphenyl)ethyl]-1,2,4-triazole-3-thione;
- Compound 41. 4-[2-(2,4-Dihydroxyphenyl)ethyl]-5-phenyl-1,2,4-triazole-3-thione;
- Compound 42. 5-(3-Biphenyl)-4-[2-(2,4-dihydroxyphenyl)ethyl]-1,2,4-triazole-3-thione;
- Compound 43. 5-(2-Biphenyl)-4-[2-(2,4-dihydroxyphenyl)ethyl]-1,2,4-triazole-3-thione;
- Compound 44. *o*-{2-[3-Methyl-*1H*-pyrrol-2-yl)-5-thioxo-1,4-dihydro-1,2,4-triazol-4-yl]ethyl}benzoic acid;
- Compound 45. 6-(3-Phenyl-5-sulfanylidene-4,5-dihydro-1H-1,2,4-triazol-4-yl)hexanoic acid;
- Compound 46. 3-Phenyl-4-(3-phenyl-5-thioxo-1,4-dihydro-1,2,4-triazol-4-yl)butyric acid;
- Compound 47. 3-(3-Phenyl-5-sulfanylidene-4,5-dihydro-1H-1,2,4-triazol-4-yl)cyclohexane-1-carboxylic acid;
- Compound 48. 4-[3-(1-Methyl-1*H*-pyrrol-2-yl)-5-thioxo-1,4-dihydro-1,2,4-triazol-4-yl]butyric acid;
- Compound 49. 4-(2-Azidoethyl)-3-phenyl-4,5-dihydro-1H-1,2,4-triazole-5-thione;
- Compound 50. 4-[3-(Quinolin-2-yl)-5-sulfanylidene-4,5-dihydro-1H-1,2,4-triazol-4-yl]butanoic acid;
- Compound 51. *o*-{[3-(3-Biphenylyl)-5-thioxo-1,4-dihydro-1,2,4-triazol-4-ylamino]methyl} benzoic acid;
- Compound 52. *m*-[(3-Phenyl-5-thioxo-1,4-dihydro-1,2,4-triazol-4-yl)methyl]benzoic acid;
- Compound 53. 5-Hydroxy-2-[2-(3-phenyl-5-thioxo-1,4-dihydro-1,2,4-triazol-4-yl)ethyl]benzoic acid;
- Compound 54. 4-[4-(Benzyloxy)phenylethyl]-5-phenyl-1,2,4-triazole-3-thione;
- Compound 55. *p*-[(3-Phenyl-5-thioxo-1,4-dihydro-1,2,4-triazol-4-yl)methyl]benzoic acid;
- Compound 56. *o*-{2-[3-(6-Quinolyl)-5-thioxo-1,4-dihydro-1,2,4-triazol-4-yl]ethyl}benzoic acid;
- Compound 57. *o*-{2-[3-(1*H*-Pyrrol-2-yl)-5-thioxo-1,4-dihydro-1,2,4-triazol-4-yl]ethyl}benzoic acid;
- Compound 58. *o*-{2-[3-(3-Thienyl)-5-thioxo-1,4-dihydro-1,2,4-triazol-4-yl]ethyl}benzoic acid;
- Compound 59. 8-(3-Phenyl-5-thioxo-1,4-dihydro-1,2,4-triazol-4-yl)octanoic acid;
- Compound 60. Phenyl[2-(3-phenyl-5-thioxo-1,4-dihydro-1,2,4-triazol-4-yl)ethylthio]acetic acid; and
- Compound 61. 5-Amino-2-[2-(3-phenyl-5-thioxo-1,4-dihydro-1,2,4-triazol-4-yl)ethyl]benzoic acid.

20. Compound according to any one of the preceding claims or a pharmaceutically acceptable salt thereof, for use in the treatment of a bacterial infection in combination with a beta-lactam antibiotic.

21. A pharmaceutical composition comprising a compound according to claims 1 to 20, or a pharmaceutically acceptable salt thereof, in admixture with a pharmaceutically acceptable diluent or carrier.

22. Pharmaceutical composition according to claim 21, for use in the treatment of a bacterial infection, in combination with a beta-lactam antibiotic.
